(19) Europäisches Patentamt / European Patent Office / Office européen des brevets

(11) EP 4 692 042 A1

(12) **EUROPEAN PATENT APPLICATION**
published in accordance with Art. 153(4) EPC

(43) Date of publication:
**11.02.2026 Bulletin 2026/07**

(21) Application number: **24780648.2**

(22) Date of filing: **28.03.2024**

(51) International Patent Classification (IPC):
***C07C 69/54*** *(2006.01)* ***C07C 271/48*** *(2006.01)*
***C07D 307/885*** *(2006.01)* ***C07D 333/76*** *(2006.01)*
***C08F 2/46*** *(2006.01)* ***C08F 20/30*** *(2006.01)*
***G03H 1/02*** *(2006.01)*

(52) Cooperative Patent Classification (CPC):
**C07C 69/54; C07C 271/48; C07D 307/885; C07D 327/04; C07D 333/76; C07D 409/14; C07D 411/14; C08F 2/46; C08F 20/30; G03H 1/02**

(86) International application number:
**PCT/JP2024/012674**

(87) International publication number:
**WO 2024/204548 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023053749**

(71) Applicant: **Mitsubishi Chemical Corporation**
**Tokyo 100-8251 (JP)**

(72) Inventors:
- **NAKURA, Ryo**
  **Tokyo 100-8251 (JP)**
- **YAMASHITA, Shuji**
  **Tokyo 100-8251 (JP)**
- **YABE, Akiko**
  **Tokyo 100-8251 (JP)**
- **INOUE, Kazuma**
  **Tokyo 100-8251 (JP)**
- **SATO, Ken**
  **Tokyo 100-8251 (JP)**

(74) Representative: **Hoffmann Eitle**
**Patent- und Rechtsanwälte PartmbB**
**Arabellastraße 30**
**81925 München (DE)**

# (54) COMPOUND, POLYMERIZABLE COMPOSITION, HOLOGRAM RECORDING MEDIUM, POLYMER, OPTICAL MATERIAL, AND OPTICAL COMPONENT

(57) A compound represented by Formula (1) described below: [in Formula (1), $A^1$ and $A^2$ are a polymerizable group; n1 and n2 are an integer of 1 to 3; $L^1$ is a single bond or an optionally branched (n1 + 1)-valent linking group; $L^2$ is a single bond or an optionally branched (n2 + 1)-valent linking group; X is a single bond, a divalent organic group having 1 to 20 carbons, a sulfonyl group, or a divalent oxygen atom or sulfur atom; $R^1$ and $R^2$ are a fused aromatic ring group optionally having a substituent; and m1 and m2 are an integer of 2 to 4].

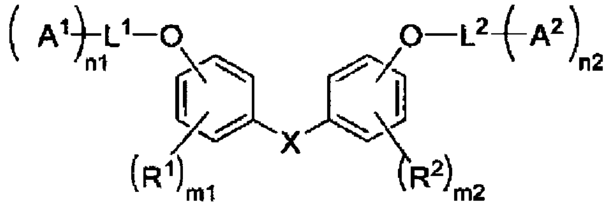

(1)



Processed by Luminess, 75001 PARIS (FR)

(Cont. next page)

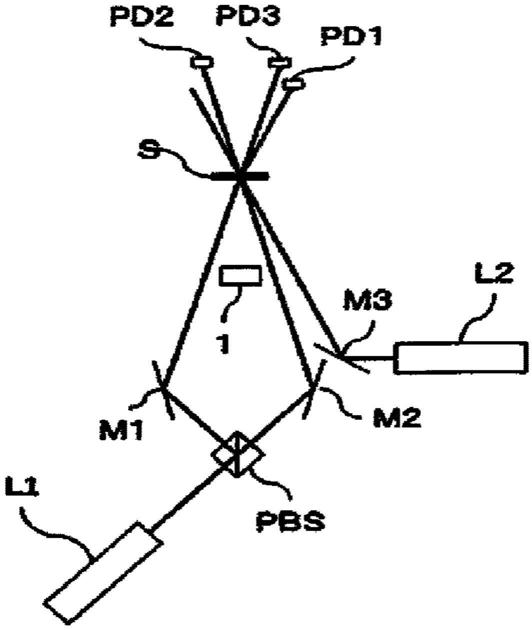


FIG. 1

## Description

Technical Field

The present invention relates to a compound having a high refractive index, high transparency, easily polymerizable properties, and excellent chemical stability, and also having excellent solubility in various solvents. The present invention also relates to a polymerizable composition containing the compound, and a hologram recording medium, an optical material, and an optical component, each of which includes a polymer of the polymerizable composition.

Background Art

Glass has often been used as an optical material. For example, in the case of an optical lens, among lenses having the same focal length, a lens produced using a material having a high refractive index is advantageous in that the lens can be thinned, the weight is reduced, and the degree of freedom in designing the optical path is improved. An optical lens having a high refractive index is also effective for miniaturization, high resolution, and wideangle conversion of optical imaging devices.

In recent years, plastics having high transparency have attracted attention as optical materials replacing glass.

The plastic material has advantages such as easy weight reduction, easy improvement of mechanical strength, and easy processing and molding as compared with glass. With the development of peripheral technologies, there is also an increasing demand for performance improvement for plastic optical materials. For example, resin raw materials for optical lenses are required to be easily polymerized (easily polymerizable properties), have good curability and solubility in various solvents, and have a high refractive index of a polymerized material.

More recently, it has become common to produce a resin material using a plurality of resin raw materials to satisfy many physical properties. For example, in the case of a composite material in which a plurality of these resin raw materials whose molecular structures and chemical properties are greatly different from each other, such as polyurethane resin raw materials, epoxy resin raw materials, acrylic resin raw materials, and methacrylic resin raw materials, are used in combination, excellent solubility and compatibility with other resin raw materials are important for expressing the performance of the composite material exceeding the performance of a single resin.

Until now, many resins have been developed to increase the refractive index.

To increase the refractive index, it is effective to introduce an aromatic ring. For example, 9,9-bis[4-(2-acryloyloxyethoxy)phenyl]fluorene is frequently used as a polyfunctional high refractive index acrylate. However, this compound has a refractive index of about 1.62, which cannot be said sufficiently high (Patent Literature 1).

Patent Literature 2 and Patent Literature 3 describe that a hologram recording medium having high diffraction efficiency and high light transmittance can be obtained by using a high refractive index aromatic acrylate compound in which a large number of aromatic rings are substituted. However, in general, there is a trade-off relationship between an increase in molecular weight through introduction of an aromatic substituent and solubility in various media and resin raw materials, and it is known that the solubility is significantly reduced when the number of aromatic substituents is increased. Among the polymerizable compounds described in Examples of Patent Literature 2 and Patent Literature 3, the polymerizable compounds exhibiting hologram recording performance that withstand practical use do not have sufficient solubility in the resin raw materials. In addition, a monofunctional compound having one polymerizable group may be inferior in heat resistance to a polyfunctional compound that forms a crosslinked structure at the time of polymerization.

Patent Literature 4 describes that solubility is improved by introducing an alkylene linker into a phenol substituted with a large number of aromatic rings. However, examples thereof are limited to disubstituted phenols because of a trade-off concern between an increase in molecular weight through introduction of an aromatic ring and a decrease in solubility as described above.

Patent Literature 5 describes a fused aromatic substituted bisphenol having a fluorene skeleton which is a polyfunctional compound as a high refractive resin raw material. However, the aromatic ring substituent and the number of aromatic ring substituents in examples are limited to a monocyclic phenyl group, the phenyl group being monosubstituted for one phenoxy skeleton.

Similarly, also in Patent Literature 6 related to an acrylate monomer for holograms having a polyaromatic substituted phenol and aniline, the total number of carbons of aromatic ring substituents exhibiting a high refractive index is limited to 24 or less in total.

In addition to introducing an aromatic ring, incorporating a sulfur atom into a molecule is also effective for increasing the refractive index of a compound. For example, Patent Literature 7 describes an acrylate compound having a glycerin skeleton having two benzothiazole rings in one molecule, but the refractive index thereof is 1.63.

Patent Literature 8 describes a diacrylate monomer having a pentaerythritol skeleton having 1 to 2 naphthylthio groups in one molecule. However, this compound also has a refractive index of 1.62 to 1.65.

These materials cannot be said to have a sufficient refractive index for applications requiring an ultrahigh

refractive index more than 1.65.

Patent Literature 9 and Patent Literature 10 describe monomers having a dibenzothiophenethio group and a refractive index of more than 1.65. Patent Literature 11 and Patent Literature 12 describe ultrahigh refractive index acrylate compounds having a dibenzocarbazole group and a refractive index of more than 1.7. However, these compounds have an alkylthio group and an alkylcarbazole group which are easily oxidized by oxygen. Thus, discoloration or color change may occur through heating under air, long-term storage, light irradiation, or the like, and it cannot be said that these materials have high chemical stability.

## Citation List

### Patent Literature

Patent Literature 1: JP H06-220131 A
Patent Literature 2: WO 2009/151061 A
Patent Literature 3: JP 5664707 B
Patent Literature 4: JP 6089867 B
Patent Literature 5: JP 2009-256342 A
Patent Literature 6: EP 2354845 B
Patent Literature 7: JP 2005-133071 A
Patent Literature 8: JP 2008-527413 A
Patent Literature 9: JP 6458645 B
Patent Literature 10: WO 2021/100654 A
Patent Literature 11: WO 2021/006011 A
Patent Literature 12: WO 2021/006012 A


## Summary of Invention

### Technical Problem

An object of the present invention is to provide a polyfunctional compound useful as an optical material or an optical component, the compound having a high refractive index, high transparency, easily polymerizable properties, chemical stability, and high solubility or compatibility with various solvents and resin raw materials.

### Solution to Problem

The present inventors have found that a trade-off relationship between an increase in molecular weight through introduction of a large number of fused aromatic ring substituents and a decrease in solubility in various media and resin raw materials can be overcome by bonding a polymerizable group via a linking group to a high refractive index structure in which a total of four or more fused aromatic ring substituents are introduced into two phenoxy rings of a polyfunctional compound so as to purposely cause an increase in molecular weight. This allows a compound that achieves the object of the present invention to be obtained. In addition, improvement of solubility in a solvent and a resin raw material has been achieved while having a high refractive index, high transparency, easily polymerizable properties, and chemical stability of the polymerizable composition and the polymer. Specifically, the present inventors have found that a high-performance hologram recording medium having high diffraction efficiency can be obtained by using compound (1) described below.

That is, the gist of the present invention is as follows.

[1] A compound represented by Formula (1) described below.

[Chem. 1]

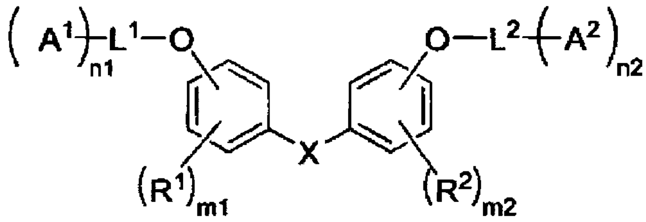

(1)

[In Formula (1), $A^1$ and $A^2$ each independently represent a polymerizable group;
n1 and n2 each independently represent an integer of 1 to 3; when n1 and n2 are 2 or 3, a plurality of $A^1$s or $A^2$s may be the same or different;
$L^1$ represents a single bond or an optionally branched (n1 + 1)-valent linking group, and $L^2$ represents a single bond or an optionally branched (n2 + 1)-valent linking group;
X represents a single bond, a divalent organic group having 1 to 20 carbons, a sulfonyl group, or a divalent oxygen atom or sulfur atom; when n1 and n2 are 2 or 3, a plurality of $A^1$s or $A^2$s may be the same or different;
$R^1$ and $R^2$ each independently represent a fused aromatic ring group optionally having a substituent;
m1 and m2 are each independently an integer of 2 to 4, and a plurality of $R^1$s or $R^2$s may be the same or different; and two benzene rings having $R^1$ and $R^2$ in the formula each independently optionally have an additional substituent other than $R^1$ or $R^2$.]

[2] The compound according to [1], wherein m1 and m2 are 2.
[3] The compound according to [1] or [2], wherein X is a single bond or a divalent group selected from the group consisting of structures represented by Formulae (1a) to (1h) described below.

[Chem. 2]

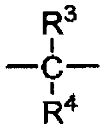

(1a)

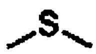

(1b)

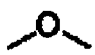

(1c)

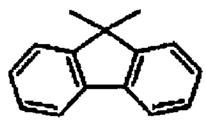

(1d)

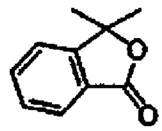

(1e)

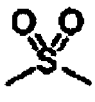

(1f)

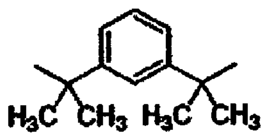

(1g)

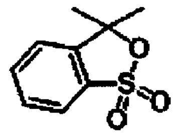

(1h)

[In Formula (1a), $R^3$ and $R^4$ each independently represent a hydrogen atom or an alkyl group having 1 to 4 carbons.]
[4] The compound according to any of [1] to [3], wherein $R^1$ and $R^2$ are each independently a phenanthryl group optionally having a substituent or a fused aromatic heterocyclic group optionally having a substituent.
[5] A polymerizable composition containing the compound described in any of [1] to [4] and a polymerization initiator.
[6] A hologram recording medium containing the polymerizable composition described in [5].
[7] A polymer produced by polymerizing the polymerizable composition described in [5].
[8] An optical material containing the polymer described in [7].
[9] An optical component containing the polymer described in [7].
[10] A large-capacity memory containing the hologram recording medium described in [6].
[11] An optical element produced by performing hologram recording on the hologram recording medium described in [6].
[12] An AR light guide plate (waveguide) containing the optical element described in [11].

[13] An AR glass containing the optical element described in [11].

Advantageous Effects of Invention

The present invention provides a polyfunctional compound useful as an optical material or an optical component, the compound having a high refractive index, high transparency, easily polymerizable properties, chemical stability, and high solubility or compatibility with various solvents and resin raw materials.

The compound of the present invention is particularly useful as a reactive compound to be used for a hard coat layer of an optical lens or an optical member, or a hologram recording medium. The use of the compound of the present invention can realize an optical material and an optical component having high diffraction efficiency, high light transmittance, high chemical stability, and excellent processability.

Brief Description of Drawings

FIG. 1 is a schematic diagram illustrating an overview of a configuration of a device used for hologram recording.

Description of Embodiments

Hereinafter, embodiments of the present invention will be specifically described. The present invention is not limited to the following embodiments, and various modifications can be made within the scope of the gist of the present invention.

In the present invention, "(meth)acrylate" is a generic term for acrylate and methacrylate. "(Meth)acryloyl group" is a generic term for an acryloyl group and a methacryloyl group. The same applies to "(meth)acryloyloxy group" and "(meth)acryl".

In the present invention, "aromatic ring" is a generic term for "aromatic hydrocarbon ring" and "aromatic heterocyclic ring". Similarly, "aromatic ring group" is a generic term for "aromatic hydrocarbon group" and "aromatic heterocyclic group".

In the present invention, the phrase "optionally having a substituent" means that it may have one or more substituents.

1. Compound of Present Invention

The compound of the present invention is represented by Formula (1) described below. Hereinafter, the compound represented by Formula (1) may be referred to as "compound (1)".

[Chem. 3]

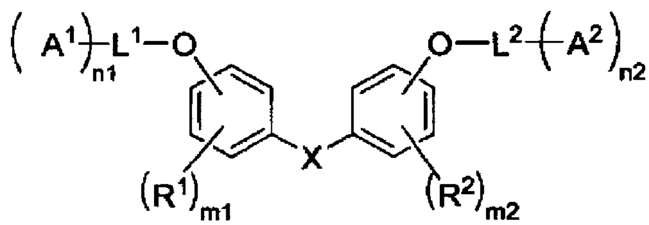

(1)

[In Formula (1), $A^1$ and $A^2$ each independently represent a polymerizable group;
n1 and n2 each independently represent an integer of 1 to 3; when n1 and n2 are 2 or 3, a plurality of $A^1$s or $A^2$s may be the same or different;
$L^1$ represents a single bond or an optionally branched (n1 + 1)-valent linking group, and $L^2$ represents a single bond or an optionally branched (n2 + 1)-valent linking group;
X represents a single bond, a divalent organic group having 1 to 20 carbons, a sulfonyl group, or a divalent oxygen atom or sulfur atom; when n1 and n2 are 2 or 3, a plurality of $A^1$s or $A^2$s may be the same or different;
$R^1$ and $R^2$ each independently represent a fused aromatic ring group optionally having a substituent;
m1 and m2 are each independently an integer of 2 to 4, and a plurality of $R^1$s or $R^2$s may be the same or different; and two benzene rings having $R^1$ and $R^2$ in the formula each independently optionally have an additional substituent other than $R^1$ or $R^2$.]

1-1. Structure of Compound (1)

Compound (1) has a structure including four or more fused aromatic ring groups (fused aromatic ring substituents) in total directly substituted in a skeleton in which a benzene ring (hereinafter, it may be referred to as "phenoxy ring" or "phenoxy skeleton") in which polymerizable groups $A^1$ and $A^2$ are substituted with an oxygen atom via linking groups $L^1$ and $L^2$ is dimerized, in which two or more of the fused aromatic ring groups are directly substituted in each phenoxy skeleton.

In an optical material, an organic compound having an aromatic ring group may be used for improving the refractive index. However, an organic compound having an aromatic ring group with high planarity usually has poor solubility in various solvents and resin raw materials. Thus, it is difficult to use the organic compound in the form of a high concentration solution that realizes a high refractive index.

Even when a high concentration solution can be prepared, there is a problem that the organic compound is likely to be precipitated from the storage solution over time because the organic compound has high crystallinity. In particular, an aromatic organic compound to be used for an optical material having a refractive index of more than 1.65 has a plurality of aromatic rings as substituents, and as the molecular weight thereof increases, the solubility in various solvents tends to decrease.

When a high refractive index material is mixed with a polyurethane resin raw material, an epoxy resin raw material, an acrylic resin raw material, a methacrylic resin raw material, or the like, the refractive index and chemical characteristics of the aromatic organic compound and the resin raw material are greatly different from each other, and thus the solubility or the compatibility is low. Therefore, there are many problems when the mixture is used as a composite material capable of being stored for a long period of time.

In compound (1), a plurality of fused aromatic ring groups are densely included in each of the two phenoxy skeletons, which generates molecular twist between substituents and can reduce the crystallinity of compound (1). In particular, when a larger number of fused aromatic ring substituents having a high refractive index are introduced, the molecular weight increases as the refractive index of compound (1) increases, but the symmetry and planarity of the entire molecule tend to decrease, and high solubility in a solvent and a resin raw material can be realized. For example, when the polymerizable groups $A^1$ and $A^2$ are (meth)acryloyl groups in compound (1) having a large molecular weight, the density of (meth)acrylic groups in a monomer or a polymer relatively decreases. Thus, compound (1) is also very useful as an optical material having small shrinkage at the time of curing.

In addition, by bonding the polymerizable groups $A^1$ and $A^2$ via the linking groups $L^1$ and $L^2$ to appropriate positions away from a high refractive index site where steric hindrance is large, high reactivity as a polymerizable monomer can be realized while greatly improving the flexibility of compound (1). Thus, a high refractive index of the polymer and an improvement in chemical stability can be achieved.

1-2. $L^1$ and $L^2$ in Formula (1)

$L^1$ represents a single bond or an optionally branched (n1 + 1)-valent linking group. $L^2$ represents a single bond or an optionally branched (n2 + 1)-valent linking group. $L^1$ and $L^2$ may have an oxygen atom, a sulfur atom, or a nitrogen atom optionally having a substituent.

The linking group constituting $L^1$ and $L^2$ are each independently preferably an aliphatic hydrocarbon group optionally having a substituent from the viewpoint of ease of synthesis and easy availability. The number of carbons of the aliphatic hydrocarbon group (the number of carbons of the substituent is not included) is preferably 1 to 8. When the number of carbons in the aliphatic hydrocarbon group is 8 or less, the refractive index of compound (1) is unlikely to decrease, and the molecular weight is small. Thus, the viscosity tends to decrease, leading to an improvement in processability. The aliphatic hydrocarbon group constituting $L^1$ and $L^2$ may be any of a cyclic aliphatic hydrocarbon group and a chain aliphatic hydrocarbon group, and these structures may be combined. From the viewpoint of alleviating steric hindrance around the polymerizable groups $A^1$ and $A^2$, a chain aliphatic hydrocarbon group is preferable.

Examples of the chain aliphatic hydrocarbon group constituting $L^1$ and $L^2$ include an alkyl group having 1 to 8 carbons when n1 and n2 are 1. When n1 and n2 are 2 or 3, each of $L^1$s and $L^2$s may be a combination of two or more alkyl groups having 1 to 8 carbons.

Preferably, $L^1$ and $L^2$ are each independently a linking group having an oxygen atom, a sulfur atom, or a nitrogen atom optionally having a substituent, from the viewpoint of providing compound (1) with high solubility to various media and resin raw materials. These linking groups optionally have a substituent, and the number of carbons (the number of carbons of the substituent is not included) is preferably 1 to 8. When the number of carbons in the linking group is 8 or less, the refractive index of compound (1) is unlikely to decrease, and the molecular weight is small. Thus, the viscosity tends to decrease, leading to an improvement in processability. The linking groups constituting $L^1$ and $L^2$ may each independently be any of a cyclic linking group and a chain linking group, and these structures may be combined. From the viewpoint of alleviating steric hindrance around the polymerizable groups $A^1$ and $A^2$, a chain linking group is preferable.

Examples of the chain linking group constituting $L^1$ and $L^2$ and having an oxygen atom, a sulfur atom, or a nitrogen atom optionally having a substituent include, when n1 and n2 are 1, $-CH_2CH_2OCH_2CH_2-$, $-CH_2CH_2OCH_2CH_2OCH_2CH_2-$, $-CH_2CH_2SCH_2CH_2-$, $-CH_2CH_2(CO)-$, $-CH_2CH_2CH_2(CO)-$, $-CH_2CH_2CH_2CH_2(CO)-$, $-CH_2CH_2CH_2CH_2CH_2(CO)-$, $-CH_2CH_2OCH_2CH_2(CO)-$, $-CH_2CH_2NH(CO)-$, $-CH_2CH_2CH_2NH(CO)-$, $-CH_2CH_2CH_2CH_2NH(CO)-$, $-CH_2CH_2OCH_2CH_2NH(CO)-$, $-CH_2CH_2NH(CO)OCH_2CH_2-$, $-CH_2CH_2NH(CO)OCH_2CH_2CH_2-$, $-CH_2CH_2OCH_2CH_2NH(CO)OCH_2CH_2-$, $-OCH_2CH_2-$, $-OCH_2CH_2CH_2-$, $-OCH_2CH_2OCH_2CH_2-$, $-OCH_2CH_2OCH_2CH_2OCH_2CH_2-$, $-OCH_2CH_2SCH_2CH_2-$, $-OCH_2CH_2(CO)-$, $-OCH_2CH_2CH_2(CO)-$, $-OCH_2CH_2CH_2CH_2(CO)-$, $-OCH_2CH_2CH_2CH_2CH_2(CO)-$, $-OCH_2CH_2OCH_2CH_2(CO)-$, $-OCH_2CH_2NH(CO)-$, $-OCH_2CH_2CH_2NH(CO)-$, $-OCH_2CH_2CH_2CH_2NH(CO)-$, $-OCH_2CH_2OCH_2CH_2NH(CO)-$, $-OCH_2CH_2NH(CO)OCH_2CH_2-$, $-OCH_2CH_2NH(CO)OCH_2CH_2CH_2-$, and $-OCH_2CH_2OCH_2CH_2NH(CO)OCH_2CH_2-$. $L^1$ and $L^2$ may be a combination of two or more of these groups.

Examples of $L^1$ and $L^2$ include, when n1 and n2 are 2 or 3, $-(CH_2)_2C(CH_3)-$, $-(CH_2)_2C(CH_3)(CO)-$, $-(CH_2)_2C(CH_2CH_3)(CO)-$, $-(CH_2)_3C(CO)-$, $-(CH_2)_2C(CH_3)NH(CO)-$, $-(CH_2)_2C(CH_3)NH(CO)OCH_2CH_2-$, $-(CH_2)_2C(CH_3)NH(CO)OCH_2CH_2CH_2-$, $-(OCH_2)_2C(CH_3)-$, $-(OCH_2)_2C(CH_3)(CO)-$, $-(OCH_2)_2C(CH_2CH_3)(CO)-$, $-(OCH_2)_3C(CO)-$, $-(OCH_2)_2C(CH_3)NH(CO)-$, $-(OCH_2)_2C(CH_3)NH(CO)OCH_2CH_2-$, $-(OCH_2)_2C(CH_3)NH(CO)OCH_2CH_2CH_2-$, and a linking group in which any hydrogen atom in the chain linking group is substituted with a bond to a polymerizable group. In this case, it may be bonded to the polymerizable group through a branched structure.

$L^1$ and $L^2$ preferably contain a cyclic group from the viewpoint of a high refractive index, and the ring contained in the cyclic group constituting $L^1$ and $L^2$ may have a monocyclic structure or a fused ring structure. The number of rings included in $L^1$ and $L^2$ is preferably 1 to 4, more preferably 1 to 3, and still more preferably 1 to 2. Aromaticity is not required for the rings included in $L^1$ and $L^2$, but an aromatic hydrocarbon ring is preferable to maintain a high refractive index while maintaining a small size in the entire molecule. Examples of the aromatic hydrocarbon ring constituting $L^1$ and $L^2$ include a benzene ring, an indene ring, a naphthalene ring, an azulene ring, a fluorene ring, an acenaphthylene ring, an anthracene ring, a phenanthrene ring, and a pyrene ring.

The linking groups $L^1$ and $L^2$ optionally have a substituent. Examples of the substituent that may be possessed by $L^1$ and $L^2$ include a halogen atom (chlorine atom, bromine atom, or iodine atom), a hydroxyl group, a mercapto group, an alkyl group having 1 to 8 carbons, an alkenyl group having 2 to 8 carbons, an alkoxy group having 1 to 8 carbons, a phenyl group, a mesityl group, a tolyl group, a naphthyl group, a cyano group, an acetyloxy group, an alkylcarbonyloxy group having 2 to 9 carbons, an alkoxycarbonyl group having 2 to 9 carbons, a sulfamoyl group, an alkylsulfamoyl group having 2 to 9 carbons, an alkylcarbonyl group having 2 to 9 carbons, a phenethyl group, a hydroxyethyl group, an acetylamide group, a dialkylaminoethyl group to which an alkyl group having 1 to 4 carbons is bonded, a trifluoromethyl group, an alkylthio group having 1 to 8 carbons, an aromatic thio group having 6 to 10 carbons, and a nitro group.

1-3. $R^1$ and $R^2$ in Formula (1)

$R^1$ and $R^2$ represent a fused aromatic ring group optionally having a substituent. The fused aromatic ring of the fused aromatic ring group is roughly divided into a fused aromatic hydrocarbon ring and a fused aromatic heterocyclic ring.

Examples of the fused aromatic hydrocarbon ring include a naphthalene ring, a phenanthrene ring, a perylene ring, a tetracene ring, a pyrene ring, a benzpyrene ring, a chrysene ring, a triphenylene ring, an acenaphthene ring, a fluoranthene ring, a fluorene ring, and an anthracene ring.

The fused aromatic hydrocarbon ring constituting $R^1$ and $R^2$ is preferably a naphthalene ring, a phenanthrene ring, a pyrene ring, or a fluorene ring from the viewpoint of ease of synthesis and easy availability. From the viewpoint of suppressing fluorescence of compound (1), the fused aromatic hydrocarbon ring constituting $R^1$ and $R^2$ is more preferably a naphthalene ring, a phenanthrene ring, or a fluorene ring, and still more preferably a phenanthrene ring.

The fused aromatic heterocyclic ring constituting $R^1$ and $R^2$ is preferably a fused sulfur-containing aromatic heterocyclic ring because the refractive index of compound (1) tends to be increased. The fused sulfur-containing aromatic heterocyclic ring has at least a sulfur atom as a heteroatom constituting the fused aromatic heterocyclic ring. As the heteroatom, in addition to a sulfur atom, an oxygen atom may be included, a nitrogen atom may be included, or an oxygen atom and a nitrogen atom may be included. From the viewpoint of avoiding coloring and securing solubility, the number of heteroatoms constituting the fused sulfur-containing aromatic heterocyclic ring is preferably 1 to 3, and more preferably 1 to 2.

Examples of the fused sulfur-containing aromatic heterocyclic ring include a fused aromatic heterocyclic ring containing one sulfur atom, such as a benzothiophene ring, a dibenzothiophene ring, a benzonaphthothiophene ring, a dinaphthothiophene ring, a naphthothiophene ring, a dinaphthothiophene ring, or a dibenzothiopyran ring; a fused aromatic heterocyclic ring containing two or more sulfur atoms, such as a thianthrene ring; and a fused aromatic heterocyclic ring containing two or more types of heteroatoms, such as a benzothiazole ring, a naphthothiazole ring, a phenothiazine ring, a thiazoloimidazole ring, a thiazolopyridine ring, a thiazolopyridazine ring, a thiazolopyrimidine ring, a dioxazolopyrazine ring, a thiazolopyrazine ring, a thiazoloxazole ring, a dibenzobenzothiophene ring, a thienoxazole

ring, a thienothiadiazole ring, or a thiazolothiadiazole ring.

The number of rings constituting the fused sulfur-containing aromatic heterocyclic ring is preferably 2 to 8, more preferably 2 to 6, and particularly preferably 2 to 5, from the viewpoint of easy availability of raw material and ease of synthesis.

In particular, from the viewpoint of a high refractive index and low colorability, the fused sulfur-containing aromatic heterocyclic ring is preferably a benzothiazole ring, a dibenzothiophene ring, a benzothiophene ring, a benzonaphthothiophene ring, a dinaphthothiophene ring, or a thianthrene ring.

The fused aromatic heterocyclic ring constituting $R^1$ and $R^2$ may be a nitrogen-containing aromatic heterocyclic ring from the viewpoint of ease of synthesis. The fused nitrogen-containing aromatic heterocyclic ring has at least a nitrogen atom as a heteroatom constituting the fused aromatic heterocyclic ring. As the heteroatom, in addition to a nitrogen atom, an oxygen atom may be included, a sulfur atom may be included, or an oxygen atom and a sulfur atom may be included. From the viewpoint of avoiding coloring, the number of heteroatoms constituting the fused nitrogen-containing aromatic heterocyclic ring is preferably 1 to 3, more preferably 1 to 2.

Examples of the fused nitrogen-containing aromatic heterocyclic ring include a fused aromatic heterocyclic ring containing one nitrogen atom, such as an indole ring, a carbazole ring, a benzocarbazole ring, a dibenzocarbazole ring, a quinoline ring, an isoquinoline ring, a benzoxazole ring, a naphthoxazole ring, a benzothiazole ring, a naphthothiazole ring, a phenoxazine ring, a phenothiazine ring, a thienoxazole ring, a thiazoloxazole ring, an oxazoloxazole ring, a furothiazole ring, a thienothiazole ring, or a thiazolothiazole ring; and a fused aromatic heterocyclic ring containing two or more nitrogen atoms, such as a benzimidazole ring, an oxazoloimidazole ring, an oxazolopyridine ring, an oxazolopyridazine ring, an oxazolopyrimidine ring, an oxazolopyrazine ring, a quinolinoxazole ring, a dioxazolopyrazine ring, a thiazoloimidazole ring, a thienothiadiazole ring, a thiazolothiadiazole ring, a thiazolopyridine ring, a thiazolopyridazine ring, a thiazolopyrimidine ring, a thiazolopyrazine ring, or a quinolinothiazole ring.

The number of rings constituting the fused nitrogen-containing aromatic heterocyclic ring is preferably 2 to 8, more preferably 2 to 6, and particularly preferably 2 to 5, from the viewpoint of easy availability of raw material and ease of synthesis.

In particular, from the viewpoint of a high refractive index and low colorability, the fused nitrogen-containing aromatic heterocyclic ring is preferably a carbazole ring, a benzocarbazole ring, a dibenzocarbazole ring, a quinoline ring, an isoquinoline ring, a benzoxazole ring, a benzothiazole ring, a benzimidazole ring, or a thiadiazole ring, and more preferably a carbazole ring, a benzocarbazole ring, a dibenzocarbazole ring, a benzoxazole ring, a benzothiazole ring, a benzimidazole ring, or a thiadiazole ring.

The fused aromatic heterocyclic ring constituting $R^1$ and $R^2$ may be a fused oxygen-containing aromatic heterocyclic ring. With the fused oxygen-containing aromatic heterocyclic ring, the heat resistance and weather resistance of the polymer containing compound (1) as a raw material tend to improve. The fused oxygen-containing aromatic heterocyclic ring has at least an oxygen atom as a heteroatom constituting the fused aromatic heterocyclic ring. As the heteroatom, in addition to an oxygen atom, a nitrogen atom may be included, a sulfur atom may be included, or a nitrogen atom and a sulfur atom may be included. From the viewpoint of securing heat resistance, the number of oxygen atoms constituting the fused oxygen-containing aromatic heterocyclic ring is preferably 1 to 3, and more preferably 1 to 2.

Examples of the fused oxygen-containing aromatic heterocyclic ring include a fused aromatic heterocyclic ring containing one oxygen atom, such as a benzofuran ring, a dibenzofuran ring, a naphthofuran ring, a benzonaphthofuran ring, a dinaphthofuran ring, a phenoxazine ring, an oxazole ring, an isoxazole ring, a benzoxazole ring, a benzisoxazole ring, a naphthoxazole ring, a thienoxazole ring, a thiazoloxazole ring, an oxazoloimidazole ring, or a furothiazole ring; and a fused aromatic heterocyclic ring containing two or more oxygen atoms, such as a dibenzodioxine ring, an oxazoloxazole ring, or a dioxazolopyrazine ring.

The number of rings constituting the fused oxygen-containing aromatic heterocyclic ring is preferably 2 to 8, more preferably 2 to 6, and particularly preferably 2 to 5, from the viewpoint of easy availability of raw material and ease of synthesis.

In particular, from the viewpoint of a high refractive index and low colorability, the fused oxygen-containing aromatic heterocyclic ring is preferably a dibenzofuran ring, a benzonaphthofuran ring, a dinaphthofuran ring, a benzoxazole ring, a benzisoxazole ring, or a naphthoxazole ring, and more preferably a dibenzofuran ring, a benzonaphthofuran ring, a dinaphthofuran ring, or a benzoxazole ring.

The fused aromatic rings constituting $R^1$ and $R^2$ optionally contain a substituent. Examples of the substituent that may be contained include a halogen atom such as chlorine, bromine, or iodine, an alkyl group having 1 to 8 carbons, an alkenyl group having 2 to 8 carbons, an alkynyl group having 2 to 8 carbons, an alkoxyl group, a cyano group, an acetyloxy group, an alkylcarbonyloxy group having 2 to 9 carbons, an alkoxycarbonyl group having 2 to 9 carbons, a sulfamoyl group, an alkylsulfamoyl group having 2 to 9 carbons, an alkylcarbonyl group having 2 to 9 carbons, a phenethyl group, a hydroxyethyl group, an acetylamide group, a dialkylaminoethyl group to which an alkyl group having 1 to 4 carbons is bonded, a trifluoromethyl group, an alkylthio group having 1 to 8 carbons, an aromatic thio group having 6 to 10 carbons, and a nitro group. Among these, preferable examples include an alkyl group having 1 to 8 carbons, an alkoxyl group having

1 to 8 carbons, an alkylthio group having 1 to 8 carbons, an aromatic thio group having 6 to 10 carbons, a cyano group, an acetyloxy group, an alkylcarboxyl group having 2 to 8 carbons, a sulfamoyl group, an alkylsulfamoyl group having 2 to 9 carbons, and a nitro group.

From the viewpoint of increasing the refractive index of compound (1), preferably, the fused aromatic ring constituting $R^1$ and $R^2$ further has a group containing an aromatic ring as a substituent. Examples of the aromatic ring contained in the substituent include a benzene ring in addition to the fused aromatic ring constituting $R^1$ and $R^2$. The aromatic ring contained in such a substituent may be directly bonded to the fused aromatic ring constituting $R^1$ and $R^2$ at any position, may be bonded via an oxygen atom, a sulfur atom, or a nitrogen atom optionally having a substituent, or may be bonded via any linking group. The substituent is more preferably directly bonded to the fused aromatic ring constituting $R^1$ and $R^2$.

When the aromatic ring contained in the substituent is a sulfur-containing aromatic heterocyclic ring, the refractive index of compound (1) tends to be further increased. The definition of the sulfur-containing aromatic heterocyclic ring is synonymous with that in $R^1$. The sulfur-containing aromatic heterocyclic ring is more preferably a fused sulfur-containing aromatic heterocyclic ring, and particularly preferably a benzothiazole ring, a dibenzothiophene ring, a benzothiophene ring, a benzonaphthothiophene ring, a dinaphthothiophene ring, or a thianthrene ring.

The number of aromatic rings that $R^1$ and $R^2$ have as substituents is not particularly limited, but is preferably 1 to 4, and more preferably 1 to 2, from the viewpoint of ease of synthesis and solubility.

The fused aromatic ring optionally having a substituent and constituting $R^1$ and $R^2$ is preferably a naphthalene ring, a fluorene ring, a thianthrene ring, a phenanthrene ring, or a dibenzothiophene ring, and more preferably a phenanthrene ring, from the viewpoint of achieving both a high refractive index and high solubility in various media and resin raw materials.

The fused aromatic ring constituting $R^1$ and $R^2$ may have two or more selected from the above-described fused aromatic hydrocarbon rings, fused sulfur-containing aromatic heterocyclic rings, fused nitrogen-containing aromatic heterocyclic rings, and fused oxygen-containing aromatic heterocyclic rings.

A plurality of $R^1$s or $R^2$s are present when m is 2 to 4 as described below. A plurality of $R^1$s or $R^2$s may be the same or different.

1-4. m1 and m2 in Formula (1)

m1 and m2 each independently represent an integer of 2 to 4. These m1 and m2 can be appropriately selected. For example, m1 and m2 are preferably 2 or 3, and m1 and m2 are more preferably 2 from the viewpoint of ease of synthesis and easy availability of raw materials of compound (1).

Meanwhile, m1 and m2 are preferably 3 or 4, and m1 and m2 are more preferably 4, because there is a tendency that both an ultrahigh refractive index and high solubility can be achieved.

When m1 and m2 are 2, the substitution position of $R^1$ and $R^2$ in Formula (1) is not particularly limited, but from the viewpoint of ease of synthesis and easy availability of raw materials, the substitution position is preferably a meta position with respect to X of the phenoxy ring in Formula (1), and the substitution position is preferably an ortho position with respect to $(A^1)_{n1}$-$L^1$-O- and $(A^2)_{n2}$-$L^2$-O-.

1-5. X in Formula (1)

X represents a single bond, a divalent organic group having 1 to 20 carbons, a sulfonyl group, or a divalent oxygen atom or sulfur atom.

X is preferably a single bond, or a divalent oxygen atom or sulfur atom from the viewpoint of increasing the refractive index of compound (1). X is more preferably a single bond from the viewpoint of the chemical stability of the intermediate compound.

X is more preferably a single bond or a divalent group selected from the group consisting of structures represented by Formulae (1a) to (1h) described below, and is particularly preferably a dimethylmethylene group, i.e., a group in which $R^3$ and $R^4$ are methyl groups in Formula (1a), a phthalidenylene group (Formula (1e)), or a sulfonyl group (Formula (1f)), from the viewpoint of suppression of coloring, ease of synthesis, and easy availability of raw materials of compound (1).

[Chem. 4]

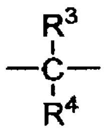
(1a)

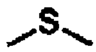
(1b)

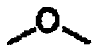
(1c)

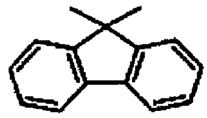
(1d)

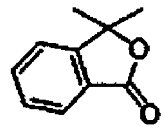
(1e)

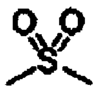
(1f)

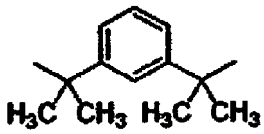
(1g)

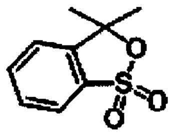
(1h)

[In Formula (1a), $R^3$ and $R^4$ each independently represent a hydrogen atom or an alkyl group having 1 to 4 carbons.]

1-6. $A^1$ and $A^2$ in Formula (1)

$A^1$ and $A^2$ are each independently a polymerizable group. The polymerizable group is a group having polymerizability with a polymerization initiator. Specific examples thereof include an oxiranyl group, an oxetanyl group, a glycidyl group, an oxetanylmethyl group, a vinyl group, an allyl group, and a (meth)acryloyl group. Among these, a (meth) acryloyl group is particularly preferable because of high reactivity.

1-7. n1 and n2 in Formula (1)

n1 and n2 each independently represent an integer of 1 to 3. These n1 and n2 can be appropriately selected. For example, from the viewpoint of causing compound (1) to be easily polymerized, n1 and n2 can be 2 or 3.

In compound (1), the number of polymerizable groups is preferably smaller, and the number of polymerizable groups is more preferably 1 per phenoxy ring, because there is a tendency that a high refractive index can be achieved. From the viewpoint of increasing the refractive index, n1 and n2 are preferably 1 or 2, and more preferably 1.

When n1 and n2 are 2 or 3, a plurality of polymerizable groups $A^1$ or $A^2$ may be the same or different.

1-8. Substituent Other Than $R^1$ and $R^2$ of Phenoxy Ring in Formula (1)

The two phenoxy rings in Formula (1) may have a substituent other than $R^1$ and $R^2$. Examples of the substituent that the phenoxy rings may have include a halogen atom such as fluorine, chlorine, bromine, or iodine, an alkyl group having 1 to 8 carbons, an alkenyl group having 2 to 8 carbons, an alkynyl group having 2 to 8 carbons, an alkoxyl group, a cyano group, an acetyloxy group, an alkylcarbonyloxy group having 2 to 9 carbons, an alkoxycarbonyl group having 2 to 9 carbons, a sulfamoyl group, an alkylsulfamoyl group having 2 to 9 carbons, an alkylcarbonyl group having 2 to 9 carbons, a phenethyl group, a hydroxyethyl group, an acetylamide group, a dialkylaminoethyl group to which an alkyl group having 1 to 4 carbons is bonded, a trifluoromethyl group, an alkylthio group having 1 to 8 carbons, an aromatic thio group having 6 to 10 carbons, and a nitro group.

From the viewpoint of ease of synthesis, the phenoxy rings preferably have no substituent other than $R^1$ and $R^2$. Meanwhile, from the viewpoint of improving the refractive index and solubility, the phenoxy rings preferably have, as a substituent, an alkyl group having 1 to 8 carbons, an alkoxyl group having 1 to 8 carbons, an alkylthio group having 1 to 8 carbons, an aromatic thio group having 6 to 10 carbons, a cyano group, an acetyloxy group, an alkylcarboxyl group having 2 to 8 carbons, a sulfamoyl group, an alkylsulfamoyl group having 2 to 9 carbons, or a nitro group.

1-9. Molecular Weight

The molecular weight of compound (1) is preferably 2500 or less, more preferably 2200 or less, and still more preferably 2000 or less from the viewpoint of suppressing the viscosity to a low level and maintaining good processability. The molecular weight of compound (1) is preferably 600 or more, more preferably 700 or more, and still more preferably 800 or more from the viewpoint of reducing the shrinkage rate during polymerization.

1-10. Relationship between Molecular Structure and Physical Properties

In compound (1), a molecular twist in which the dihedral angle of the linking site between the phenoxy ring and the fused aromatic ring substituent is 1 degree or more can be appropriately introduced into monomers and polymers by bonding a total of four or more fused aromatic ring groups with high planarity to two phenoxy rings. In particular, when $R^1$ and $R^2$ are a fused aromatic ring group having a substituent or a ring structure at an ortho position of a linking site with a phenoxy ring as represented by a 9-phenanthrenyl group, a 1-thianthrenyl group, a 4-dibenzofuranyl group, or a 4-dibenzothiophenyl group, the dihedral angle tends to be larger. This makes it possible to realize high solubility in various media and resin raw materials while suppressing aggregation of the high refractive index structure, and the compound can be used as a polymerizable monomer having an ultrahigh refractive index.

Further, the molecular twist has an effect of suppressing excessive conjugate extension between the substituents $R^1$ and $R^2$ and the phenoxy ring and suppressing an increase in the absorption wavelength of compound (1). This makes it possible to achieve, for example, an optical material that is colorless and transparent in the visible light region, and to improve the stability of the compound against heating, light irradiation, oxidation, and the like.

In the development of high refractive index materials, it is very important to improve the electron density per volume in monomers and polymers, i.e., to shorten the intermolecular distance for improving the refractive index. In compound (1), the introduction of a linker having an appropriate molecular length into a sterically large polyaromatic substituted high refractive index structure may shorten the intermolecular distance as compared with a derivative having no linker because of molecular interaction such as van der Waals force and hydrogen bonding. Thus, a further increase in refractive index with the same high refractive index structure can also be expected.

1-11. Exemplary Compound

Specific examples of compound (1) are described below. Compound (1) of the present invention is not limited to these examples as long as it does not exceed the gist thereof.

[Chem. 5]

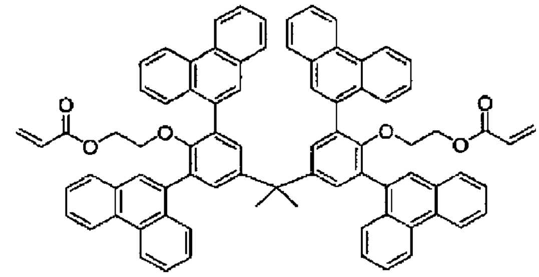

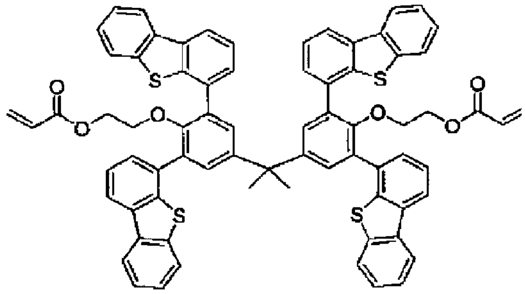

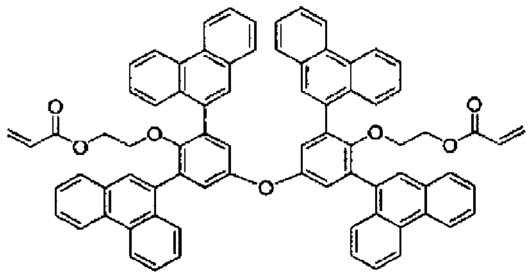

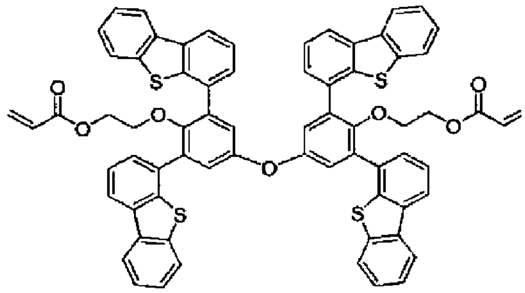

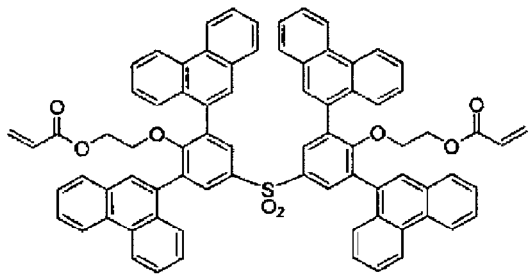

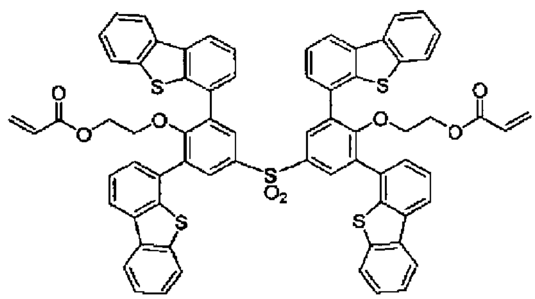

[Chem. 6]

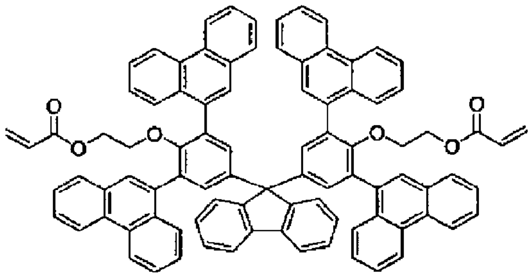
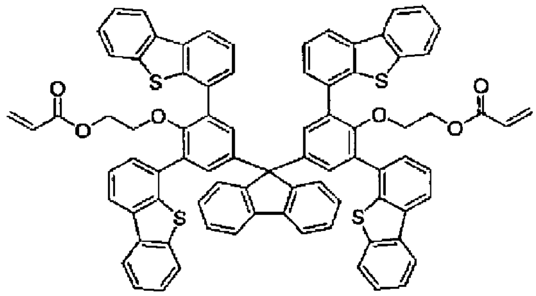

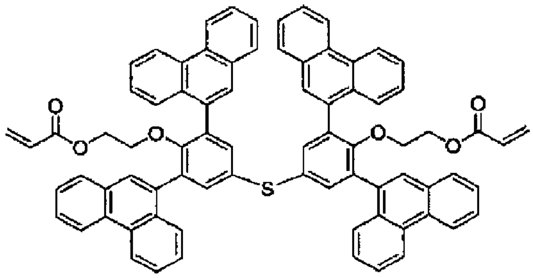
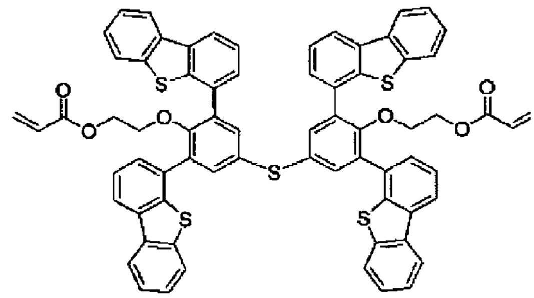

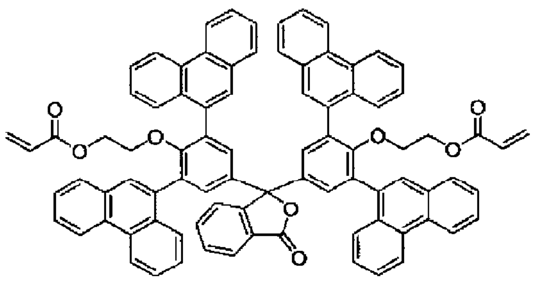
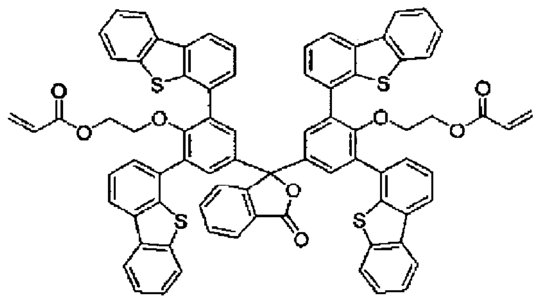

[Chem. 7]

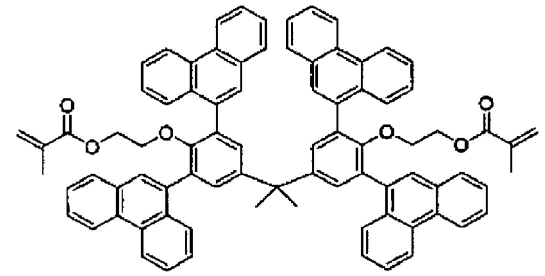

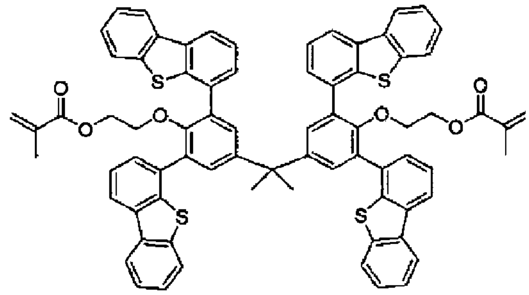

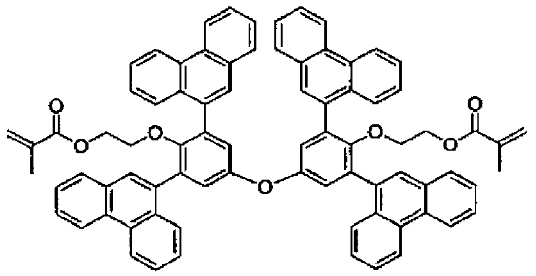

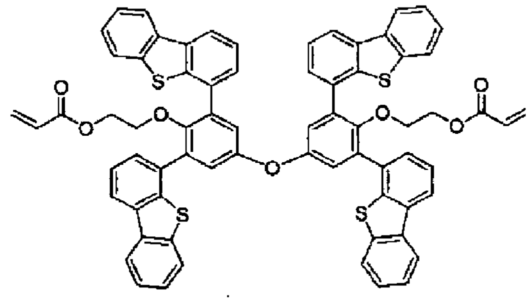

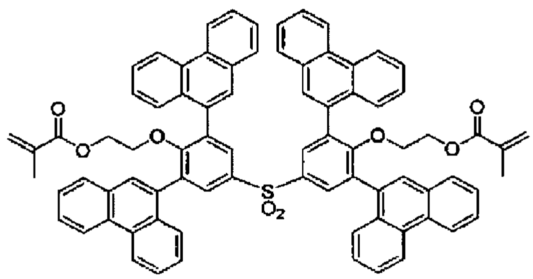

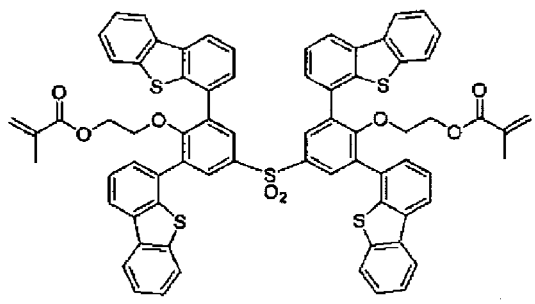

[Chem. 8]

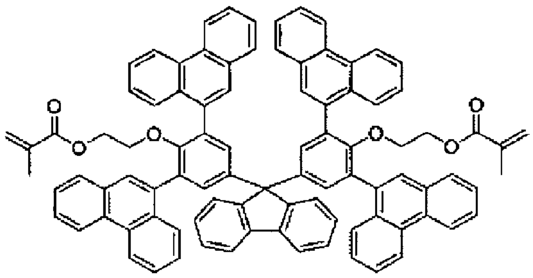

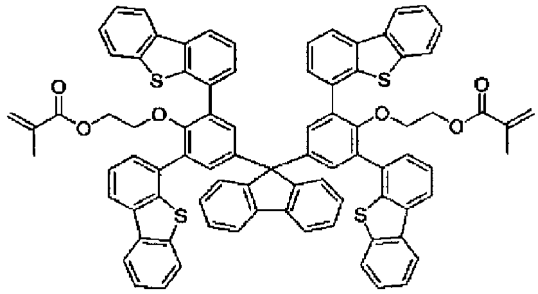

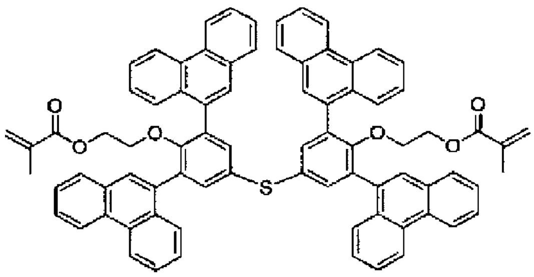

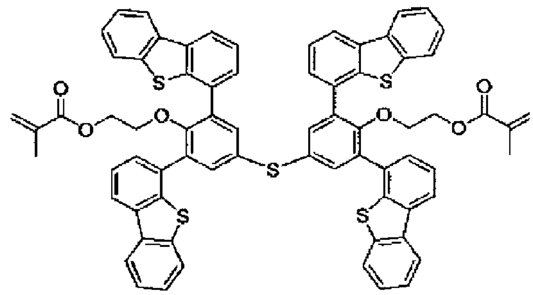

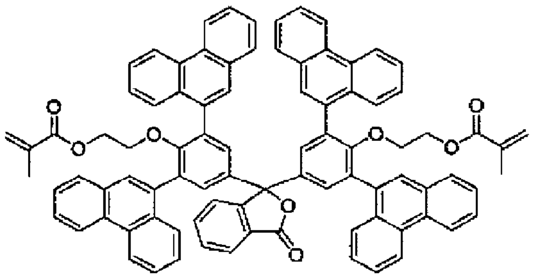

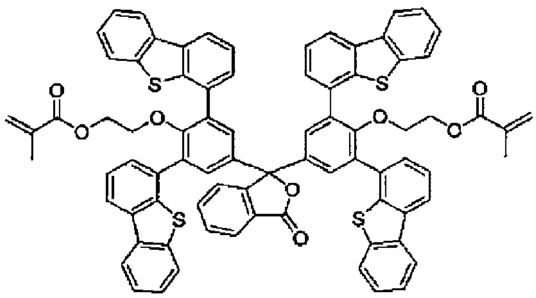

[Chem. 9]

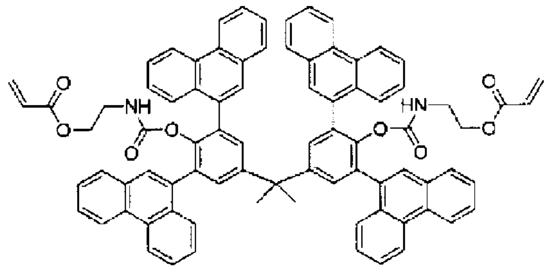

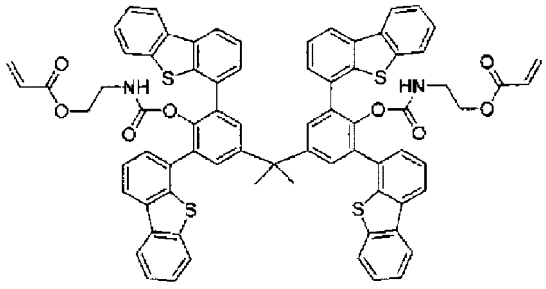

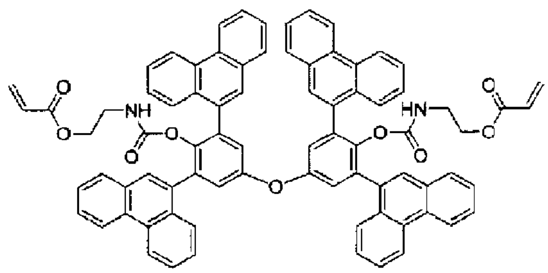

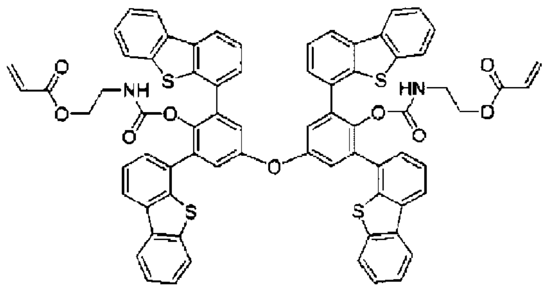

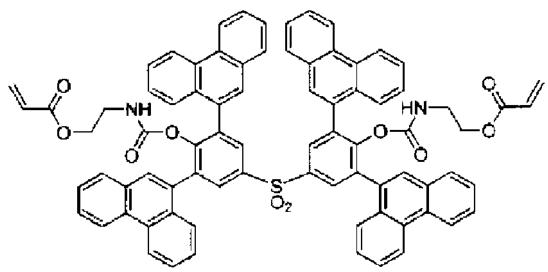

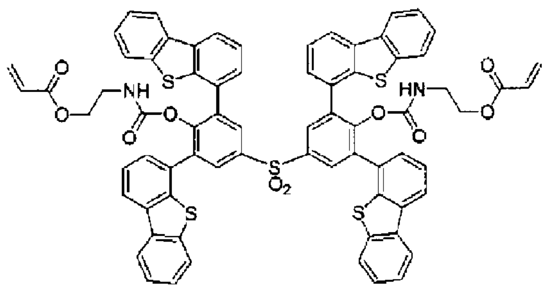

[Chem. 10]

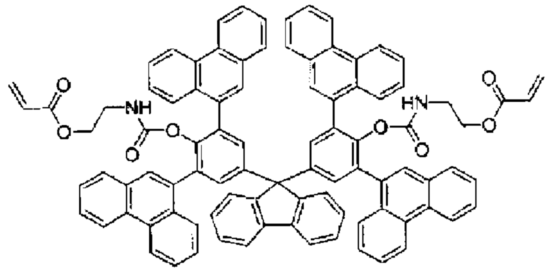

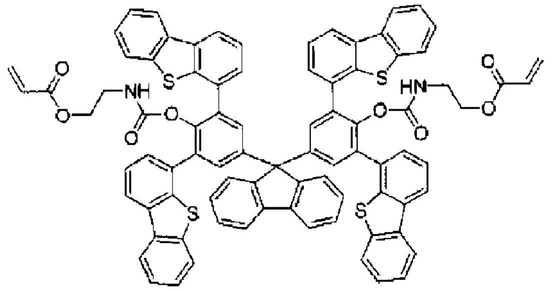

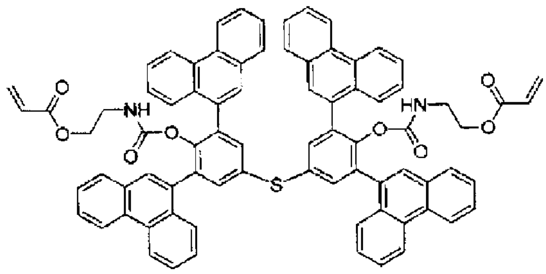

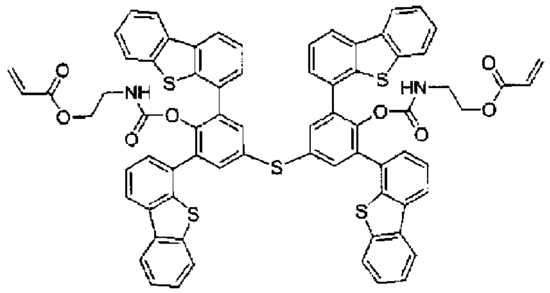

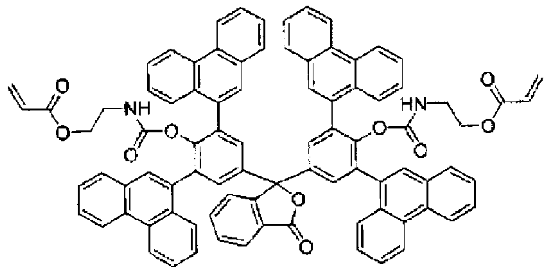

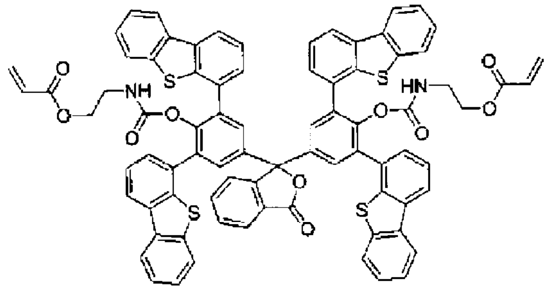

[Chem. 11]

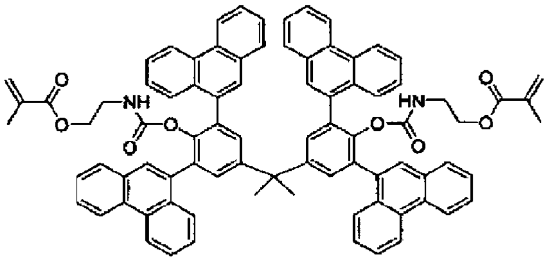

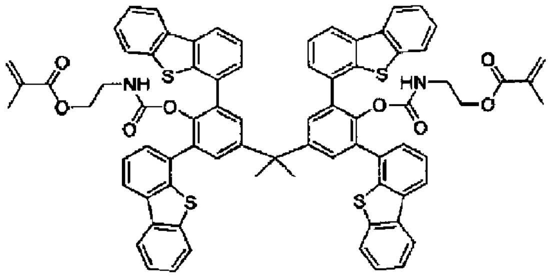

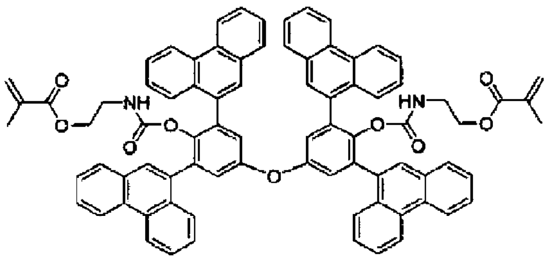

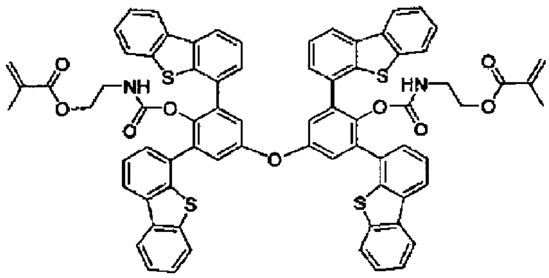

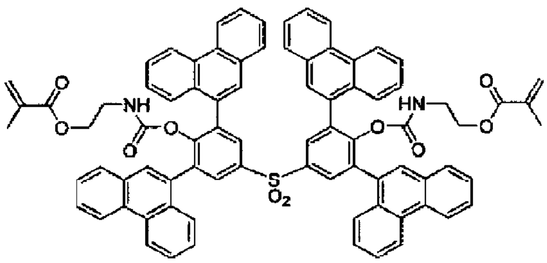

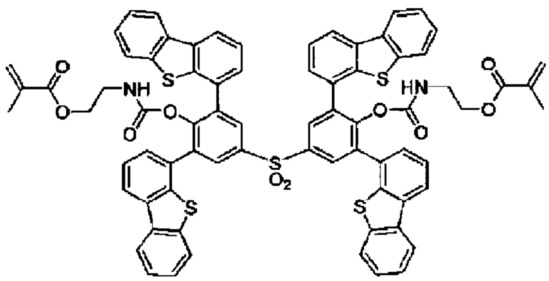

[Chem. 12]

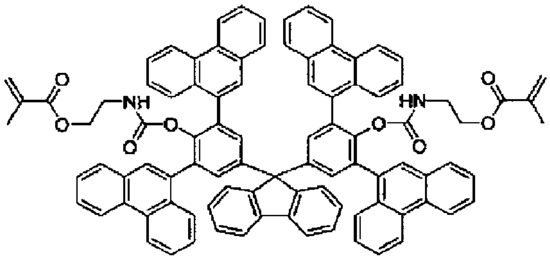

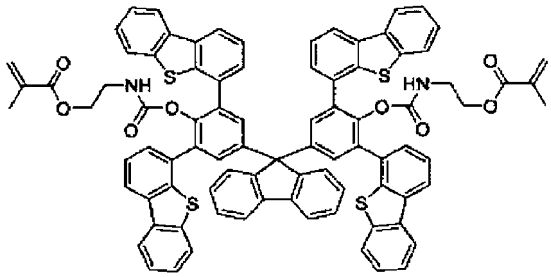

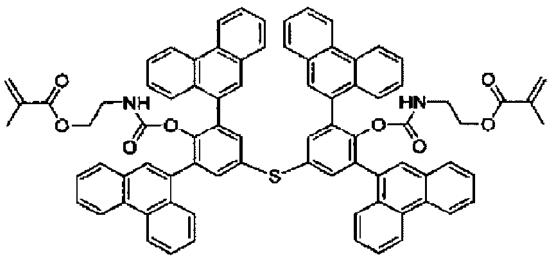

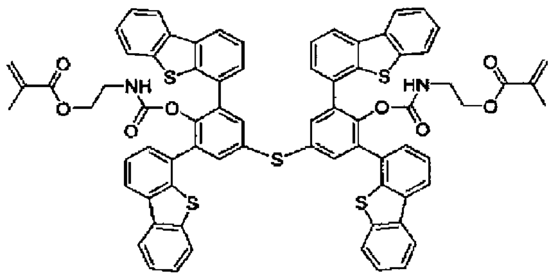

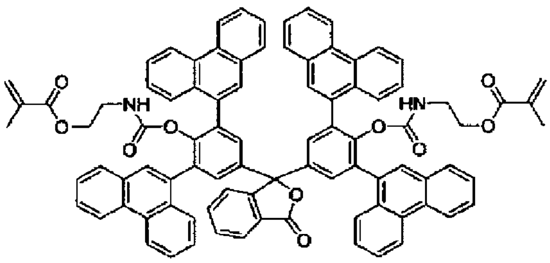

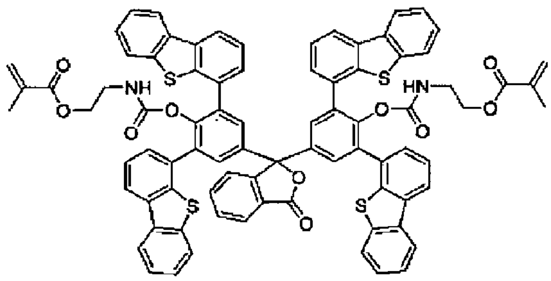

[Chem. 13]

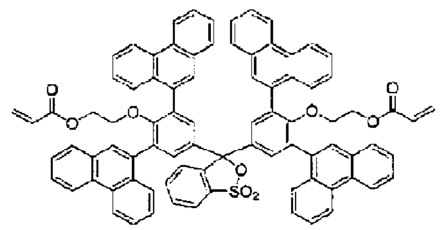

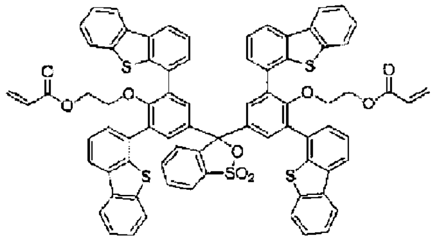

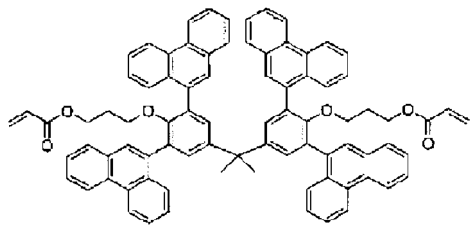

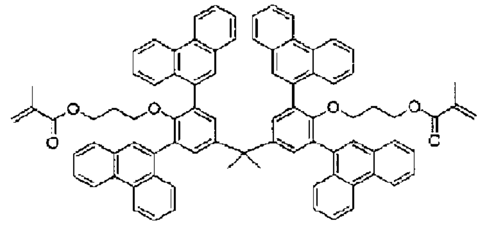

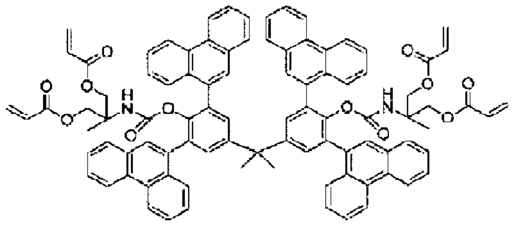

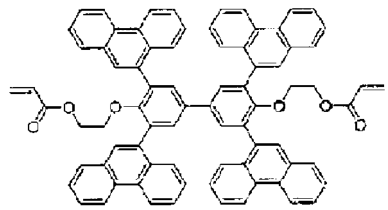

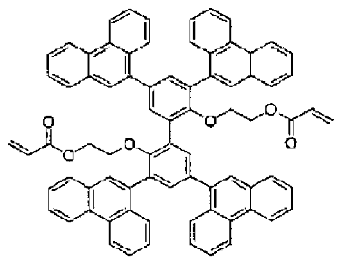

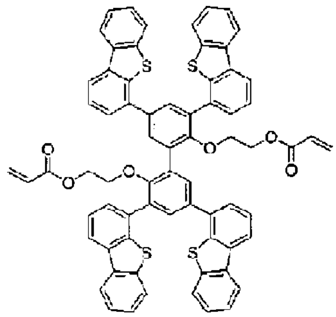

[Chem. 14]

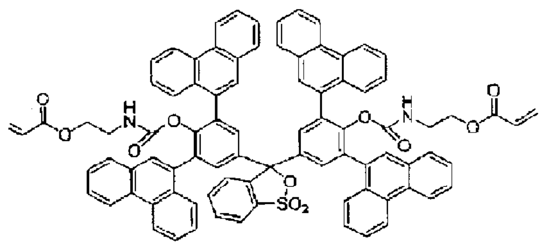

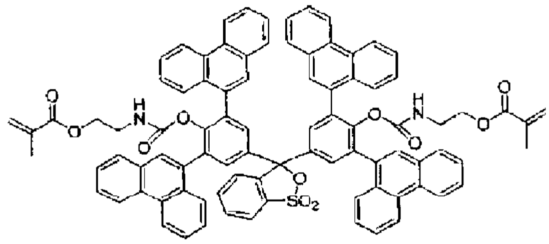

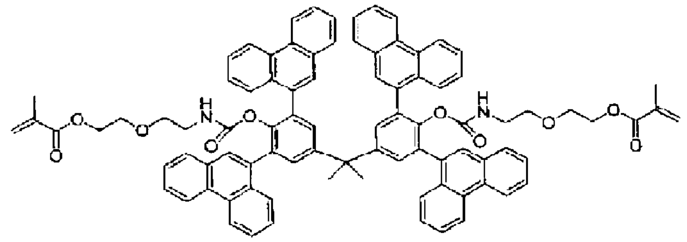

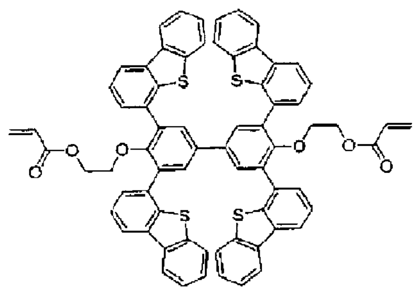

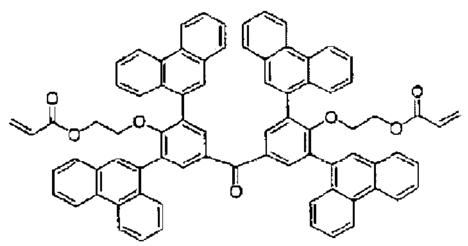

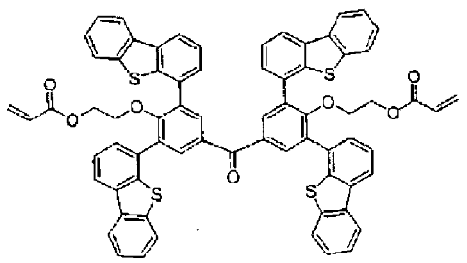

[Chem. 15]

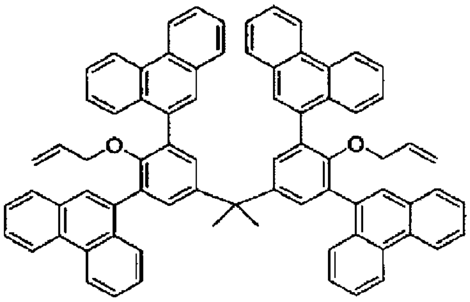

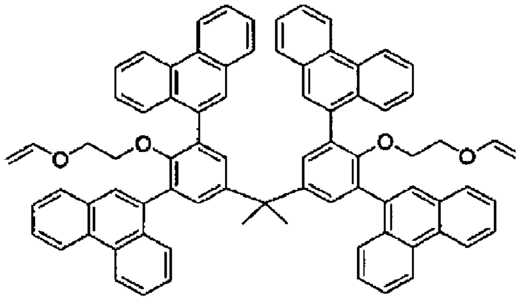

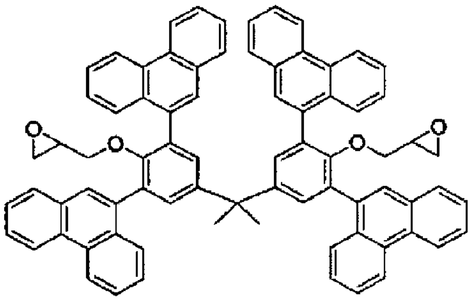

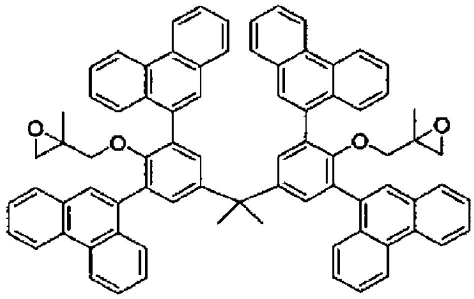

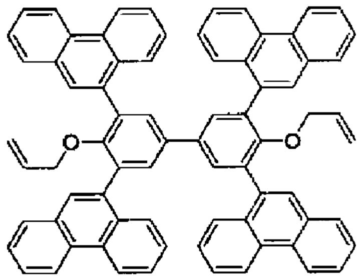

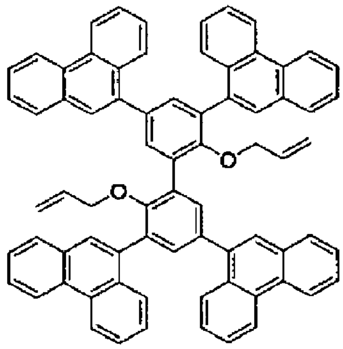

[Chem. 16]

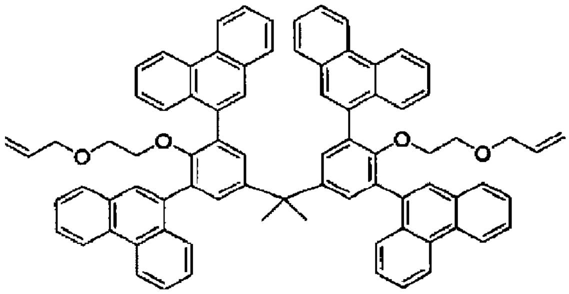

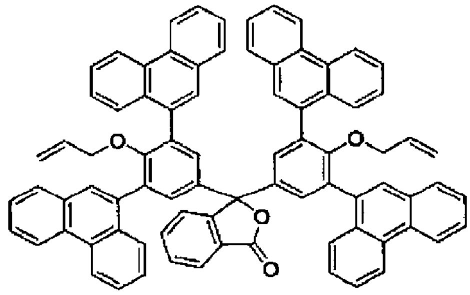

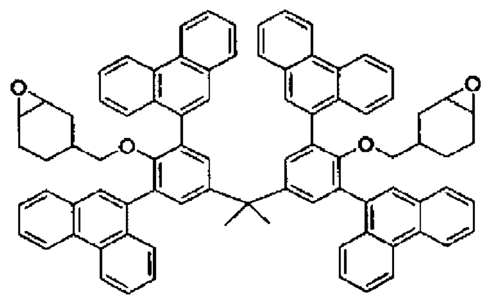

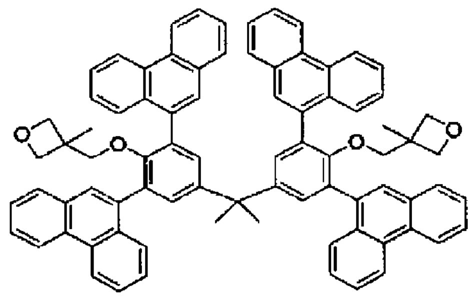

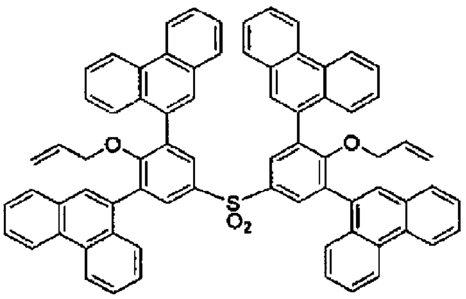

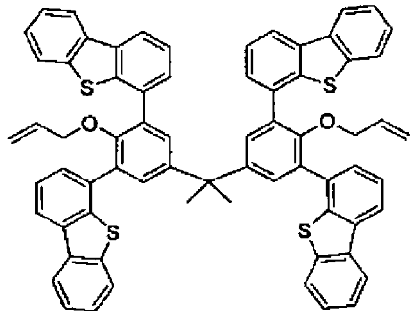

[Chem. 17]

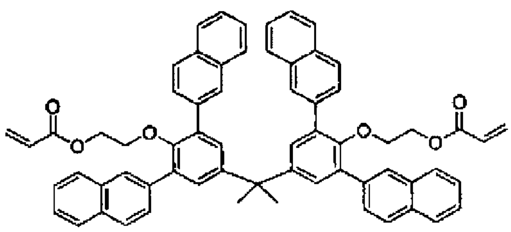

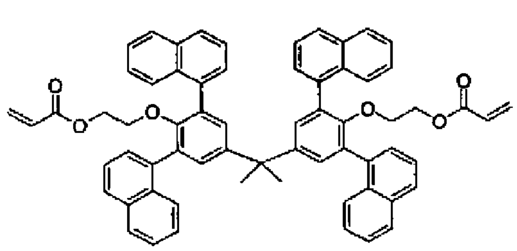

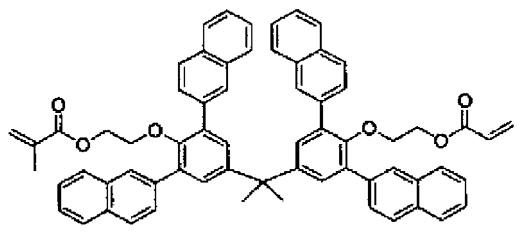

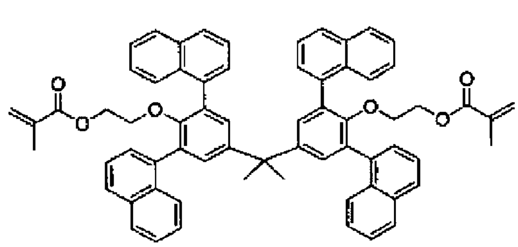

[Chem. 18]

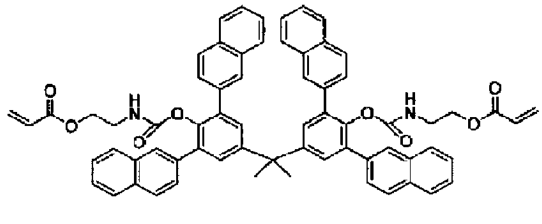

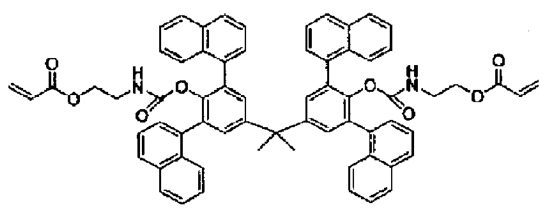

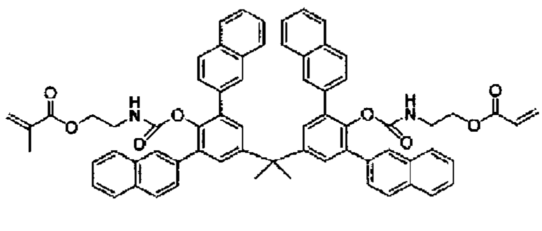

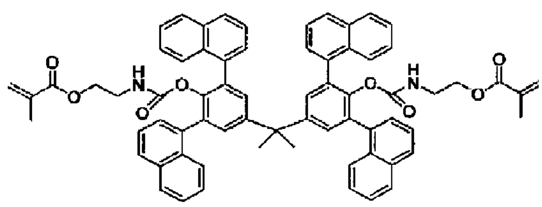

1-12. Synthesis Method

Compound (1) can be synthesized by combining various known methods. For example, the compound can be synthesized through a reaction between a compound represented by Formula (2) described below (hereinafter may be referred to as "compound (2)") and a compound having a group capable of reacting with a hydroxy group.

[Chem. 19]

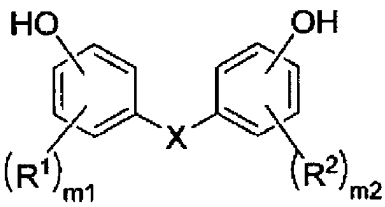

(2)

[In the formula, X, $R^1$, $R^2$, m1, and m2 have the same meanings as in Formula (1).]

An example of synthesis example of compound (1) will be described below.

[Chem. 20]

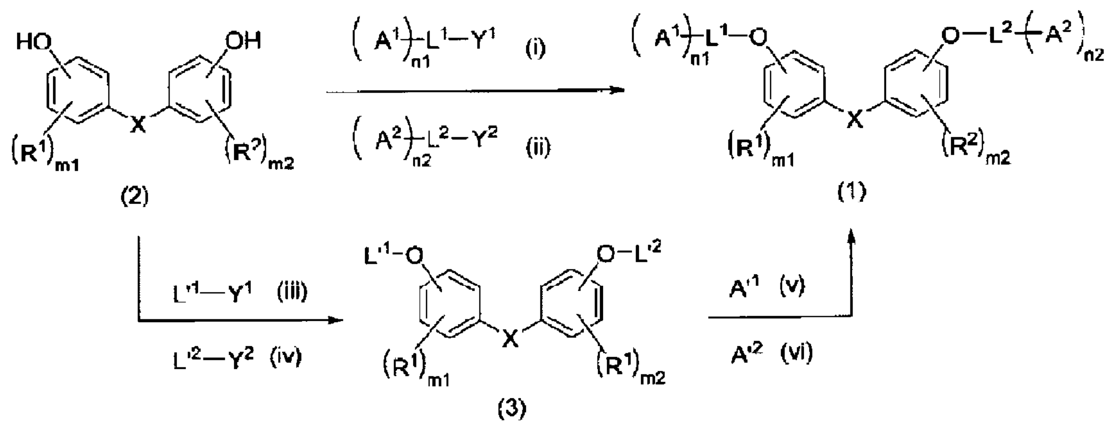


[In the reaction formula, X, $R^1$, $R^2$, $L^1$, $L^2$, $A^1$, $A^2$, m1, m2, n1, and n2 have the same meanings as in Formula (1). $Y^1$ and $Y^2$ represent a leaving group such as a halogen atom, a sulfonate group such as a methanesulfonate group, a tosyl group, or a trifluoromethanesulfonate group, a carboxyl group, a dimethylpyrazolyl group, or a 1-methylpropylideneaminooxy group.

L'$^1$, L'$^2$, A'$^1$, and A'$^2$ represent a precursor structure that undergoes a chemical reaction to become $L^1$, $L^2$, $A^1$, and $A^2$, respectively. In the following reaction formulae, the same symbols represent the same components.]

For example, compound (1) is compound (1A) in which the polymerizable groups $A^1$ and $A^2$ are a (meth) acryloyloxy group in Formula (1). Compound (1A) can be produced by a method of reacting a hydroxyl group of compound (2) with (meth)acrylating reagents having a polymerizable group represented by Formulae (i) and (ii) (hereinafter may be referred to as (meth)acrylating reagents (i) and (ii)).

Examples of the (meth)acrylating reagents (i) and (ii) include alkylating reagents such as 2-methanesulfonylethyl (meth)acrylate, glycidyl (meth)acrylate, and 2,3-dibromopropyl acrylate.

Examples of the (meth)acrylating reagents (i) and (ii) include isocyanates such as 2-acryloyloxyethyl isocyanate, 2-methacryloyloxyethyl isocyanate, 2-(2-methacryloyloxyethyloxy)ethyl isocyanate, and 1,1-(bisacryloyloxymethyl)ethyl isocyanate, and carbonylating reagents such as 2-[(3,5-dimethylpyrazolyl)carbonylamino]ethyl methacrylate and 2-[0-(1'-methylpropylideneamino)carboxyamino]ethyl methacrylate.

Compound (1A) can also be produced through a polymerizable groupforming reaction of a compound represented by Formula (3) (hereinafter may be referred to as "compound (3)") obtained by a reaction between compound (2) and a compound having a linking group represented by Formula (iii) or Formula (iv) with a (meth)acryloylating reagent represented by Formula (v) or Formula (vi).

Known methods can be applied to the reaction of active hydrogen of the hydroxyl group in Formula (2) with the above reagents represented by (i) to (iv) and the reaction of compound (3) with the reagents represented by Formula (v) and Formula (vi). For example, compound (2) is reacted with an isocyanate in the presence of a basic compound to produce compound (1A).

The basic compound may be one or more organic basic compounds (triethylamine, diisopropylethylamine, 1,1,3,3-tetramethylguanidine, diazabicycloundecene, diazabicyclononene, pyridine, imidazole, and the like), one or more inorganic basic compounds (sodium carbonate, potassium carbonate, sodium hydride, potassium hydride, potassium tert-butoxide, and the like), or a combination of one or more organic basic compounds and one or more inorganic basic compounds.

In the reaction between compound (2) and the reagents represented by (i) to (iv) and the reaction between compound (3) and the reagents represented by Formula (v) and Formula (vi), an organic solvent is preferably used. Examples of the organic solvent include dichloromethane, tetrahydrofuran (THF), dimethoxyethane, toluene, and N,N-dimethylformamide (DMF). One type of the organic solvents may be used, or two or more types thereof may be used in combination.

In the production of compound (1A), it is preferable to purify a reaction product (crude product) obtained through the synthesis reaction. Low colorability can be achieved by purifying and removing impurities. As the purification method, a known method can be applied. For example, the reaction product can be purified by an extraction method, a column chromatography method, a recrystallization method, a distillation method, or the like. These purification methods may be performed singly, or may be sequentially performed in combination.

When compound (1A) is solid at normal temperature, it is preferable to use the recrystallization method in view that the colored substance is easily removed to a high degree.

Examples of the recrystallization solvent include aliphatic hydrocarbon-based solvents such as n-pentane, n-hexane, and n-heptane, alicyclic hydrocarbons such as cyclopentane and cyclohexane, aromatic hydrocarbon-based solvents such as toluene, ethylbenzene, xylene, and mesitylene, halogenated hydrocarbon-based solvents such as methylene chloride, chloroform, and 1,2-dichloroethane, ether-based solvents such as diethyl ether, diisopropyl ether, tetrahydrofuran, t-butyl methyl ether, and 1,4-dioxane, ketone-based solvents such as acetone, methyl ethyl ketone, and methyl isobutyl ketone, ester-based solvents such as ethyl acetate, n-butyl acetate, and propylene glycol monomethyl ether acetate, nitrile-based solvents such as acetonitrile and propionitrile, alcohol-based solvents such as methanol, ethanol, n-propanol, isopropanol, n-butanol, 2-butanol, t-butanol, 2-methoxyethanol, 2-butoxyethanol, and propylene glycol monomethyl ether, glycol-based solvents such as ethylene glycol and diethylene glycol, and water. One type of these solvents may be used singly, or two or more types thereof may be used in combination.

Compound (2) and intermediate compound (3), which are raw material compounds used for the production of compound (1A), can be produced through the following reaction.

[Chem. 21]

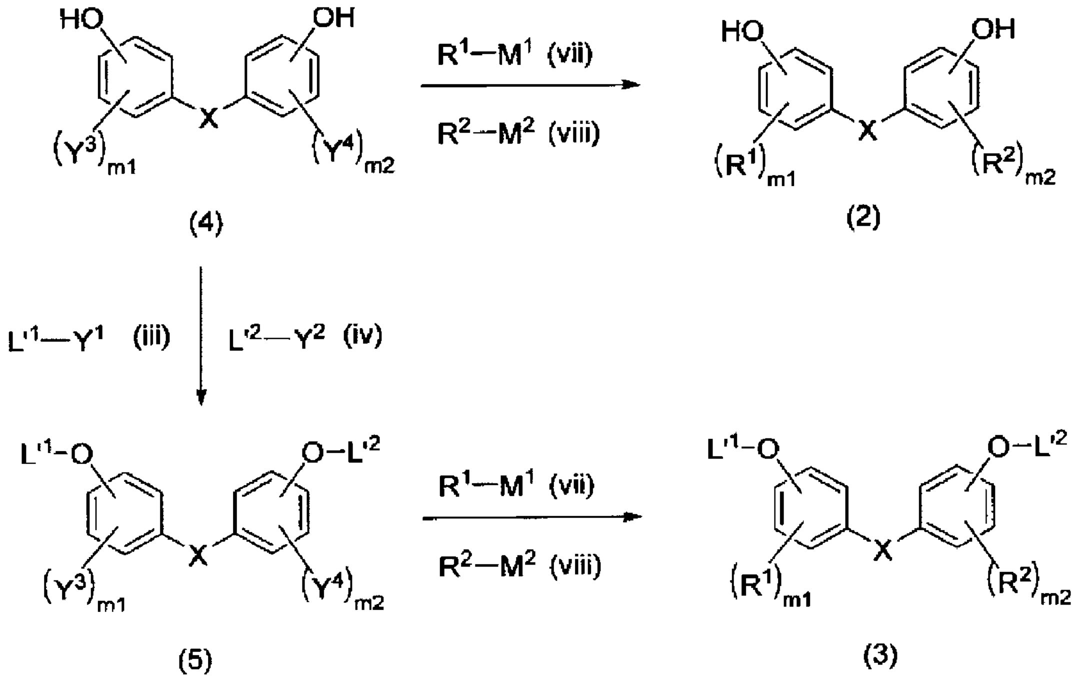


(4) (2)

(5) (3)

[In the reaction formula, X, $R^1$, $R^2$, m1, and m2 have the same meanings as defined in Formula (1). $L'^1$ and $L'^2$ have the same meanings as defined in compound (3) and Formulae (iii) and (iv). $Y^3$ and $Y^4$ represent a leaving group such as a halogen atom or a trifluoromethanesulfonate group. $R^1$-$M^1$ and $R^2$-$M^2$ represent an organometallic reagent capable of reacting with $Y^3$ or $Y^4$ to form a carbon-carbon bond, such as an organolithium reagent, an organomagnesium reagent, an organozinc reagent, an organocerium reagent, an organosilicon reagent, an organoboron reagent, or an organotin reagent, or an aromatic ring compound that directly utilizes a carbon-hydrogen bond to cause a crosscoupling reaction.]

For example, compound (2) can be synthesized by simultaneously or sequentially linking a plurality of aromatic ring groups $R^1$ and $R^2$ through a linking reaction between an aromatic halide represented by Formula (4) (hereinafter may be referred to as "compound (4)") and an organic reagent represented by Formula (vii) or Formula (viii).

Compound (3) can be synthesized by simultaneously or sequentially linking a plurality of aromatic ring groups $R^1$ and $R^2$ to a compound represented by Formula (5) (hereinafter may be referred to as "compound (5)") obtained through a reaction between compound (4) and a compound having a linking group represented by Formula (iii) or Formula (iv) through a linking reaction with an organic reagent represented by Formula (vii) or Formula (viii).

2. Polymerizable Composition of Present Invention

The polymerizable composition of the present invention contains compound (1) and a polymerization initiator.

With the polymerization initiator, the polymerizable groups $A^1$ and $A^2$ of compound (1) cause a polymerization reaction, and the polymer of the present invention can be produced.

2-1. Polymerization Initiator

The type of the polymerization initiator is not particularly limited, and may be appropriately selected from known polymerization initiators depending on the polymerization method. The polymerization method is also not limited, and polymerization can be performed by a known method such as a bulk polymerization method, a solution polymerization method, a suspension polymerization method, an emulsion polymerization method, or a partial polymerization method.

Examples of the polymerization initiator contained in the polymerizable composition of the present invention include a radical polymerization initiator, a redox-based polymerization initiator, an anionic polymerization initiator, and a cationic polymerization initiator. In addition, a photocationic polymerization initiator that generates a cation as an active species through light irradiation can also be used.

Examples of the polymerization initiator described below include those generally referred to as a polymerization catalyst.

2-1-1. Radical Polymerization Initiator

Photopolymerization Initiator

As the photopolymerization initiator that assists the polymerization of the polymerizable composition of the present invention, any known photoradical polymerization initiator can be used. Examples of the photopolymerization initiator include azo compounds, azide compounds, organic peroxides, organic borates, bisimidazole derivatives, titanocene compounds, iodonium salts, organic thiol compounds, halogenated hydrocarbon derivatives, acetophenones, benzophenones, hydroxybenzenes, thioxanthones, anthraquinones, ketals, acylphosphine oxides, sulfone compounds, carbamic acid derivatives, sulfonamides, triarylmethanols, and oxime esters. Among these, benzophenones, acylphosphine oxide compounds, oxime ester compounds, and the like are preferable as the photopolymerization initiator from the viewpoint of compatibility, easy availability, and the like.

Specific examples of the photopolymerization initiator include benzophenone, 2,4,6-trimethylbenzophenone, methylorthobenzoyl benzoate, 4-phenylbenzophenone, t-butylanthraquinone, 2-ethylanthraquinone, diethoxyacetophenone, 2-hydroxy-2-methyl-1-phenylpropan-1-one, oligo{2-hydroxy-2-methyl-1-[4-(1-methylvinyl)phenyl]propanone}, benzyl dimethyl ketal, 1-hydroxycyclohexyl phenyl ketone, benzoin methyl ether, benzoin ethyl ether, benzoin isopropyl ether, benzoin isobutyl ether, 2-methyl-[4-(methylthio)phenyl]-2-morpholino-1-propanone, 2-benzyl-2-dimethylamino-1-(4-morpholinophenyl)-butanone-1, diethylthioxanthone, isopropylthioxanthone, 2,4,6-trimethylbenzoyldiphenylphosphine oxide, bis(2,6-dimethoxybenzoyl)-2,4,4-trimethylpentylphosphine oxide, bis(2,4,6-trimethylbenzoyl)-phenylphosphine oxide, 2-hydroxy-1-{4-[4-(2-hydroxy-2-methylpropionyl)benzyl]phenyl}-2-methylpropan-1-one and methylbenzoylformate, 1-[4-(phenylthio)-2-(O-benzoyloxime)]-1,2-octanedione, and 1-[9-ethyl-6-(2-methylbenzoyl)-9H-carbazol-3-yl]-1-(O-acetyloxime)ethanone.

Any one type of these photopolymerization initiators may be used singly, or two or more types thereof may be used in any combination and proportions.

The content of the photopolymerization initiator in the polymerizable composition of the present invention is usually 0.01 parts by mass or more, preferably 0.02 parts by mass or more, and more preferably 0.05 parts by mass or more, per 100 parts by mass of the total of all radically polymerizable compounds in the polymerizable composition. The upper limit is usually 10 parts by mass or less, preferably 5 parts by mass or less, and more preferably 3 parts by mass or less. When the content of the photopolymerization initiator is too large, polymerization rapidly proceeds, and not only the birefringence of the cured product is increased, but also the hue may be deteriorated. When the content of the polymerization initiator is too small, there is a possibility that the polymerizable composition is not sufficiently polymerized.

Thermal Polymerization Initiator

As the thermal polymerization initiator that assists the polymerization of the polymerizable composition of the present invention, any known thermal radical polymerization initiator can be used. Examples of the thermal polymerization initiator include organic peroxides and azo compounds. Among these, organic peroxides are preferable from the viewpoint that bubbles are hardly generated in the polymer obtained through the polymerization reaction.

Specific examples of the organic peroxide include ketone peroxides such as methyl ethyl ketone peroxide; peroxyketals such as 1,1-di(t-hexylperoxy)-3,3,5-trimethylcyclohexane, 1,1-di(t-hexylperoxy)cyclohexane, and 1,1-di(t-butylperoxy)cyclohexane; hydroperoxides such as 1,1,3,3-tetramethylbutyl hydroperoxide, cumene hydroperoxide, and p-menthane hydroperoxide; dialkyl peroxides such as dicumyl peroxide and di-t-butyl peroxide; diacyl peroxides such as dilauroyl peroxide and dibenzoyl peroxide; peroxydicarbonates such as di(4-t-butylcyclohexyl)peroxydicarbonate and di(2-ethylhexyl)peroxydicarbonate; and peroxyesters such as t-butylperoxy-2-ethylhexanoate, t-hexylperoxyisopropyl monocarbonate, t-butylperoxybenzoate, and 1,1,3,3-tetramethylbutyl-2-ethylhexanoate.

Specific examples of the azo compound include 2,2'-azobisisobutyronitrile, 2,2'-azobis(2-methylbutyronitrile), 2,2'-azobis(2,4-dimethylvaleronitrile), 1,1'-azobis-1-cyclohexanecarbonitrile, dimethyl-2,2'-azobisisobutyrate, 4,4'-azobis-4-cyanovaleric acid, and 2,2'-azobis-(2-amidinopropane)dihydrochloride.

Any one type of these thermal polymerization initiators may be used singly, or two or more types thereof may be used in any combination and proportions.

The content of the thermal polymerization initiator in the polymerizable composition of the present invention is usually 0.1 parts by mass or more, preferably 0.5 parts by mass or more, and more preferably 0.8 parts by mass or more, per 100 parts by mass of the total of all radically polymerizable compounds in the polymerizable composition. The upper limit is usually 10 parts by mass or less, preferably 5 parts by mass or less, and more preferably 2 parts by mass or less. When the content of the thermal polymerization initiator is too large, polymerization rapidly proceeds, and not only the optical uniformity of the polymer to be produced is impaired, but also the hue may be deteriorated. When the content of the thermal polymerization initiator is too small, there is a possibility that thermal polymerization does not sufficiently proceed.

When the photopolymerization initiator and the thermal polymerization initiator are used in combination, the mass ratio thereof is usually "100:1" to "1:100" ("photopolymerization initiator:thermal polymerization initiator", the same applies in this paragraph), and preferably "10:1" to "1:10". When the content of the thermal polymerization initiator is too

small, there is a possibility that the polymerization is insufficient, whereas when the content is too large, there is a possibility of coloration.

2-1-2. Redox-Based Polymerization Initiator

The redox-based polymerization initiator is a radical initiator utilizing a redox reaction with a combination of a peroxide and a reducing agent, can generate radicals even at a low temperature, and is usually used in emulsion polymerization or the like.

Specific examples of the redox-based polymerization initiator include a combination system of dibenzoyl peroxide as a peroxide and an aromatic tertiary amine such as N,N-dimethylaniline, N,N-dimethyl-p-toluidine, or N,N-bis(2-hydroxypropyl)-p-toluidine as a reducing agent; a combination system of hydroperoxide as a peroxide and a metal soap as a reducing agent; and a combination system of hydroperoxide as a peroxide and thiourea as a reducing agent.

In the water-soluble redox polymerization initiator, a peroxide such as persulfate, hydrogen peroxide, or hydroperoxide is used in combination with a water-soluble inorganic reducing agent ($Fe^{2+}$, $NaHSO_3$, or the like) or an organic reducing agent (alcohol, polyamine, or the like).

The preferred range of the content of the redox-based polymerization initiator in the polymerizable composition of the present invention is the same as that of the thermal polymerization initiator.

2-1-3. Anionic Polymerization Initiator

Examples of the anionic polymerization initiator used in the polymerizable composition of the present invention include alkali metal, n-butyllithium, sodium amide, sodium naphthalenide, Grignard reagent, lithium alkoxide, and alkali metal benzophenone ketyl. Any one type of these may be used singly, or two or more types thereof may be used in any combination and proportions.

The amount of the anionic polymerization initiator in the polymerizable composition of the present invention is usually 0.001 parts by mass or more, preferably 0.005 parts by mass or more, and more preferably 0.01 parts by mass or more, per 100 parts by mass of the total of all the anionic polymerizable compounds in the polymerizable composition. The upper limit is usually 5 parts by mass or less, preferably 1 part by mass or less, and more preferably 0.5 parts by mass or less. When the amount of the anionic polymerization initiator is less than 0.001 parts by mass, a sufficient reaction does not occur. When the anionic polymerization initiator is blended in an amount exceeding 5 parts by mass, it is difficult to achieve both the pot life and the polymerization rate.

2-1-4. Cationic Polymerization Initiator

Examples of the cationic polymerization initiator used in the polymerizable composition of the present invention include Bronsted acids such as perchloric acid, sulfuric acid, and trichloroacetic acid; Lewis acids such as boron trifluoride, aluminum trichloride, aluminum tribromide, and tin tetrachloride; iodine, and chlorotriphenylmethane. Any one type of these may be used singly, or two or more types thereof may be used in any combination and proportions.

The amount of the cationic polymerization initiator in the polymerizable composition of the present invention is usually 0.001 parts by mass or more, preferably 0.005 parts by mass or more, and more preferably 0.01 parts by mass or more, per 100 parts by mass of the total of all the cation polymerizable compounds in the polymerizable composition. The upper limit is usually 5 parts by mass or less, preferably 1 part by mass or less, and more preferably 0.5 parts by mass or less. When the amount of the cationic polymerization initiator is less than 0.001 parts by mass, a sufficient reaction does not occur. When the cationic polymerization initiator is blended in an amount exceeding 5 parts by mass, it is difficult to achieve both the pot life and the polymerization rate.

2-1-5. Photocationic Polymerization Initiator

The photocationic polymerization initiator in the present invention is an initiator that generates a cationic species with light. The photocationic polymerization initiator is not particularly limited as long as it is a compound that generates a cationic species through light irradiation. In general, onium salts are well known. Examples of the onium salt include a diazonium salt of a Lewis acid, an iodonium salt of a Lewis acid, and a sulfonium salt of a Lewis acid. Specific examples thereof include a phenyldiazonium salt of boron tetrafluoride, a diphenyliodonium salt of phosphorus hexafluoride, a diphenyliodonium salt of antimony hexafluoride, a tri-4-methylphenylsulfonium salt of arsenic hexafluoride, and a tri-4-methylphenylsulfonium salt of antimony tetrafluoride. An aromatic sulfonium salt is preferably used.

Specific examples of the photocationic polymerization initiator include S,S,S',S'-tetraphenyl-S,S'-(4,4'-thiodiphenyl)disulfonium bishexafluorophosphate, diphenyl-4-phenylthiophenylsulfonium hexafluorophosphate, and diphenyl-4-phenylthiophenylsulfonium hexafluoroantimonate. Examples of commercially available products of the photocationic polymerization initiator include trade name: UVI-6992 available from The Dow Chemical Company, trade name: CPI-100P available from San-Apro Ltd., trade name: CPI-101A available from San-Apro Ltd., trade name: CPI-200K available from San-Apro Ltd., and trade name: Omnicat 270 available from IGM Resins B.V.

Any one type of these photocationic polymerization initiators may be used singly, or two or more types thereof may be used in any combination and proportions.

The amount of the photocationic polymerization initiator in the polymerizable composition of the present invention is preferably 0.02 parts by mass or more and 20 parts by mass or less, and more preferably 0.1 parts by mass or more and 10 parts by mass or less, per 100 parts by mass of the total of all photocationic polymerizable compounds in the polymerizable composition. When the amount of the photocationic polymerization initiator is less than 0.02 parts by mass, a sufficient reaction does not occur. When the photocationic polymerization initiator is blended in an amount exceeding 20 parts by mass, it is difficult to achieve both the pot life and the polymerization rate.

In the use of the photocationic polymerization initiator, the above-described cationic polymerization initiators may be used in combination. In such a case, the cationic polymerization initiator is usually used in an amount of 0.1 to 10 parts by mass, preferably 1 to 5 parts by mass, per 100 parts by mass of the cationic polymerizable compound in the polymerizable composition. When the amount of the cationic polymerization initiator used is too small, the polymerization rate decreases. When the amount of the cationic polymerization initiator used is too large, the physical properties of the resulting polymer may deteriorate.

In the use of the photocationic polymerization initiator, a photocationic polymerization sensitizer can also be used in combination. The photocationic polymerization sensitizer is a prescription for efficiently transmitting energy of irradiation light to the photocationic polymerization initiator by using a photocationic polymerization sensitizer in combination when irradiation light from a light source used for photocationic polymerization does not match well with the absorption wavelength of the photocationic polymerization initiator. Known examples of the photocationic polymerization sensitizer include phenolic compounds such as methoxyphenol (JP H05-230189 A), thioxanthone compounds (JP 2000-204284 A), and dialkoxyanthracene compounds (JP 2000-119306 A).

The photocationic polymerization sensitizer is usually used in an amount of 0.2 to 5 parts by mass, preferably 0.5 to 1 part by mass, per 1 part by mass of the photocationic polymerization initiator. When the amount of the photocationic polymerization sensitizer is too small, it may be difficult to exhibit a sensitizing effect. When the amount of the photocationic polymerization sensitizer is too large, the physical properties of the polymer may deteriorate.

2-2. Polymerizable Compound

As the polymerizable compound contained in the polymerizable composition of the present invention, any one type of compounds (1) may be contained singly, or two or more types thereof may be contained in any combination and proportions.

The polymerizable composition of the present invention may contain an additional polymerizable compound other than compound (1).

The content of compound (1) in the polymerizable composition of the present invention is preferably 1 mass% or more and 99 mass% or less, in particular, preferably 5 mass% or more and 95 mass% or less, in terms of a proportion with respect to the total solid content of the polymerizable composition of the present invention. When the content of compound (1) is less than 1 mass%, the effect of using compound (1) is not sufficiently exhibited. When the content of compound (1) exceeds 99 mass%, curability tends to deteriorate.

Examples of the polymerizable compound other than compound (1) include cationic polymerizable monomers, anionic polymerizable monomers, and radically polymerizable monomers. Any one type of these polymerizable compounds may be used singly, or two or more types thereof may be used in any combination and proportions. A polymerizable compound having two or more polymerizable functional groups in one molecule (the compound may be referred to as polyfunctional monomer) can also be used. When a polyfunctional monomer is used, a crosslinked structure is formed inside the polymer, and thus thermal stability, weather resistance, solvent resistance, and the like can be improved.

When the polymerizable composition of the present invention contains an additional polymerizable compound other than compound (1), the content thereof is preferably 0.1 mass% or more and 10 mass% or less, and particularly preferably 0.3 mass% or more and 5 mass% or less in terms of a proportion with respect to the total solid content of the polymerizable composition of the present invention. When the content of the additional polymerizable compound is less than 0.1 mass%, the effect of imparting characteristics through addition of the polymerizable compound is not sufficiently exhibited. When the content of the additional polymerizable compound exceeds 5 mass%, problems such as impairment of optical characteristics and strength tend to easily occur.

Cationic Polymerizable Monomer

Examples of the cationic polymerizable monomer include compounds having an oxirane ring, styrene and

derivatives thereof, vinylnaphthalene and derivatives thereof, vinyl ethers, N-vinyl compounds, and compounds having an oxetane ring.

Among these, it is preferable to use a compound having at least an oxetane ring, and it is further preferable to use a compound having an oxirane ring in combination with a compound having an oxetane ring.

Examples of the compound having an oxirane ring include a prepolymer containing two or more oxirane rings in one molecule.

Examples of such a prepolymer include alicyclic polyepoxies, polyglycidyl esters of polybasic acids, polyglycidyl ethers of polyhydric alcohols, polyglycidyl ethers of polyoxyalkylene glycols, polyglycidyl ethers of aromatic polyols, hydrogenated compounds of polyglycidyl ethers of aromatic polyols, urethane polyepoxy compounds, and epoxidized polybutadienes.

Examples of styrene and derivatives thereof include styrene, p-methylstyrene, p-methoxystyrene, β-methylstyrene, p-methyl-β-methylstyrene, α-methylstyrene, p-methoxy-β-methylstyrene, and divinylbenzene.

Examples of vinylnaphthalene and derivatives thereof include 1-vinylnaphthalene, α-methyl-1-vinylnaphthalene, β-methyl-1-vinylnaphthalene, 4-methyl-1-vinylnaphthalene, and 4-methoxy-1-vinylnaphthalene.

Examples of the vinyl ethers include isobutyl ether, ethyl vinyl ether, phenyl vinyl ether, p-methylphenyl vinyl ether, and p-methoxyphenyl vinyl ether.

Examples of the N-vinyl compound include N-vinylcarbazole, N-vinylpyrrolidone, N-vinylindole, N-vinylpyrrole, and N-vinylphenothiazine.

Examples of the compound having an oxetane ring include various known oxetane compounds described in JP 2001-220526 A, JP 2001-310937 A, and the like.

Any one type of these cationic polymerizable monomers may be used singly, or two or more types thereof may be used in any combination and proportions.

Anionic Polymerizable Monomer

Examples of the anionic polymerizable monomer include a hydrocarbon monomer and a polar monomer.

Examples of the hydrocarbon monomer include styrene, α-methylstyrene, butadiene, isoprene, vinylpyridine, vinylanthracene, and derivatives thereof.

Examples of the polar monomer include methacrylic acid esters (e.g., methyl methacrylate, ethyl methacrylate, and isopropyl methacrylate); acrylic acid esters (e.g., methyl acrylate and ethyl acrylate); vinyl ketones (e.g., methyl vinyl ketone, isopropyl vinyl ketone, cyclohexyl vinyl ketone, and phenyl vinyl ketone); isopropenyl ketones (e.g., methyl isopropenyl ketone and phenyl isopropenyl ketone); and other polar monomers (e.g., acrylonitrile, acrylamide, nitroethylene, methylenemalonic acid ester, cyanoacrylic acid ester, and vinylidene cyanide).

Any one type of these anionic polymerizable monomers may be used singly, or two or more types thereof may be used in any combination and proportions.

Radically Polymerizable Monomer

The radically polymerizable monomer is a compound having one or more ethylenically unsaturated double bonds in one molecule. Examples thereof include (meth)acrylic acid esters, (meth)acrylamides, vinyl esters, and styrenes.

Examples of (meth)acrylic acid esters include methyl (meth)acrylate, ethyl (meth)acrylate, (n- or i-)propyl (meth) acrylate, (n-, i-, sec-, or t-)butyl (meth)acrylate, amyl (meth)acrylate, adamantyl (meth)acrylate, chloroethyl (meth) acrylate, 2-hydroxyethyl (meth)acrylate, 2-hydroxypropyl (meth)acrylate, 2-hydroxypentyl (meth)acrylate, cyclohexyl (meth)acrylate, allyl (meth)acrylate, trimethylolpropane mono(meth)acrylate, pentaerythritol mono(meth)acrylate, benzyl (meth)acrylate, methoxybenzyl (meth)acrylate, chlorobenzyl (meth)acrylate, hydroxybenzyl (meth)acrylate, hydroxyphenethyl (meth)acrylate, dihydroxyphenethyl (meth)acrylate, furfuryl (meth)acrylate, tetrahydrofurfuryl (meth)acrylate, phenyl (meth)acrylate, hydroxyphenyl (meth)acrylate, chlorophenyl (meth)acrylate, sulfamoylphenyl (meth)acrylate, 2-phenoxyethyl (meth)acrylate, 2-(hydroxyphenylcarbonyloxy)ethyl (meth)acrylate, phenol EO-modified (meth)acrylate, phenylphenol EO-modified (meth)acrylate, paracumylphenol EO-modified (meth)acrylate, nonylphenol EO-modified (meth)acrylate, N-acryloyloxyethylhexahydrophthalimide, bisphenol F EO-modified diacrylate, bisphenol A EO-modified diacrylate, dibromophenyl (meth)acrylate, tribromophenyl (meth)acrylate, dicyclopentenyloxyethyl (meth)acrylate, dicyclopentanyl acrylate, tricyclodecanedimethylol di(meth)acrylate, bisphenoxyethanolfluorene di(meth)acrylate, trimethylolpropane tri(meth)acrylate, pentaerythritol tri(meth)acrylate, pentaerythritol tetra(meth)acrylate, dipentaerythritol penta(meth)acrylate, and dipentaerythritol hexa(meth)acrylate. As used herein, "EO" means "ethylene oxide".

Examples of the (meth)acrylamides include (meth)acrylamide, N-methyl(meth)acrylamide, N-ethyl(meth)acrylamide, N-propyl(meth)acrylamide, N-butyl(meth)acrylamide, N-benzyl(meth)acrylamide, N-hydroxyethyl(meth)acrylamide, N-phenyl(meth)acrylamide, N-tolyl(meth)acrylamide, N-(hydroxyphenyl)(meth)acrylamide, N-(sulfamoylphenyl) (meth)acrylamide, N-(phenylsulfonyl)(meth)acrylamide, N-(tolylsulfonyl)(meth)acrylamide, N,N-dimethyl(meth)acrylamide, N-methyl-N-phenyl(meth)acrylamide, and N-hydroxyethyl-N-methyl(meth)acrylamide.

Examples of the vinyl esters include vinyl acetate, vinyl butyrate, vinyl benzoate, vinyl benzoate, vinyl t-butylbenzoate, vinyl chlorobenzoate, vinyl 4-ethoxybenzoate, vinyl 4-ethylbenzoate, vinyl 4-methylbenzoate, vinyl 3-methylbenzoate, vinyl 2-methylbenzoate, vinyl 4-phenylbenzoate, and vinyl pivalate.

Examples of the styrenes include styrene, p-acetylstyrene, p-benzoylstyrene, 2-butoxymethylstyrene, 4-butylstyrene, 4-sec-butylstyrene, 4-tert-butylstyrene, 2-chlorostyrene, 3-chlorostyrene, 4-chlorostyrene, dichlorostyrene, 2,4-diisopropylstyrene, dimethylstyrene, p-ethoxystyrene, 2-ethylstyrene, 2-methoxystyrene, 4-methoxystyrene, 2-methylstyrene, 3-methylstyrene, 4-methylstyrene, p-methylstyrene, p-phenoxystyrene, p-phenylstyrene, and divinylbenzene.

Any one type of these radically polymerizable monomers may be used singly, or two or more types thereof may be used in any combination and proportions.

Any of the anionic polymerizable monomers and radically polymerizable monomers exemplified above may be used, and two or more types thereof may be used in combination.

For example, for a high refractive index optical lens or a hologram recording medium, a radically polymerizable monomer is preferably used as an additional polymerizable compound used in combination with compound (1) because the reaction for forming the resin matrix is hardly inhibited.

2-3 Additional Additive Component

In the polymerizable composition of the present invention, an additional component can be blended as long as the effects of the present invention are not impaired.

Examples of the additional component include various additives such as a solvent, an antioxidant, a plasticizer, an ultraviolet absorber, a sensitizer, a chain transfer agent, an antifoaming agent, a polymerization inhibitor, any filler composed of an organic substance or an inorganic substance, a diffusing agent, a pigment, and a wavelength conversion material such as a phosphor.

The polymerizable composition of the present invention may contain a solvent for adjusting the viscosity.

Specific examples of the solvent include, depending on the physical properties of the polymerizable composition, alcohols such as ethanol, propanol, isopropanol, ethylene glycol, and propylene glycol; aliphatic hydrocarbons such as hexane, pentane, and heptane; alicyclic hydrocarbons such as cyclopentane and cyclohexane; aromatic hydrocarbons such as toluene and xylene; halogenated hydrocarbons such as methylene chloride and chloroform; chain ethers such as dimethyl ether and diethyl ether; cyclic ethers such as dioxane and tetrahydrofuran; esters such as methyl acetate, ethyl acetate, butyl acetate, ethyl lactate, and ethyl butyrate; ketones such as acetone, ethyl methyl ketone, methyl isobutyl ketone, and cyclohexanone; cellosolves such as methyl cellosolve, ethyl cellosolve, and butyl cellosolve; carbitols such as methyl carbitol, ethyl carbitol, and butyl carbitol; propylene glycol monoalkyl ethers such as propylene glycol monomethyl ether, propylene glycol monoethyl ether, and propylene glycol mono-n-butyl ether; glycol ether esters such as ethylene glycol monomethyl ether acetate and propylene glycol monomethyl ether acetate; amides such as N,N-dimethylformamide and N,N-dimethylacetamide, sulfoxides such as dimethyl sulfoxide; nitriles such as acetonitrile and benzonitrile; and organic solvents such as N-methylpyrrolidone.

These solvents can be used singly or in the form of a mixed solvent. Water can also be used depending on the polymerization method (emulsion polymerization, suspension polymerization, or the like).

In the case of using a solvent (or dispersion medium), the amount thereof is not particularly limited, and the amount of the solvent used may be adjusted so as to provide a polymerizable composition having a suitable viscosity depending on the polymerization method, processing method, and applications.

In the present invention, to improve the heat yellowing resistance and weather resistance of the resulting polymer, it is preferable to blend an antioxidant or a light stabilizer as an additive in the polymerizable composition.

Specific examples of the antioxidant include phenol-based antioxidants such as 2,6-di-t-butylphenol, 2,6-di-t-butyl-p-cresol, n-octadecyl-3-(3',5'-di-t-butyl-4'-hydroxyphenyl)propionate, tetrakis-[methylene-3-(3',5'-di-t-butyl-4'-hydroxyphenyl)propionate]methane, triethylene glycol bis[3-(3-t-butyl-5-methyl-4-hydroxyphenyl)propionate] and 1,6-hexanediol bis[3-(3,5-di-t-butyl-4-hydroxyphenyl)propionate]; and phosphorus-based antioxidants such as triphenyl phosphite, trisisodecyl phosphite, isodecyldiphenyl phosphite, 2-ethylhexyldiphenyl phosphite, tetra(C12 to C15 alkyl)-4,4'-isopropylidenediphenyl diphosphite, tris(nonylphenyl) phosphite, tristridecyl phosphite, 2,4,8,10-tetra-tert-butyl-6-[(2-ethylhexan-1-yl)oxy]-12H-dibenzo[d,g][1,3,2]dioxaphosine, 3,9-bis(2,6-di-tert-butyl-4-methylphenoxy)-2,4,8,10-tetraoxa-3,9-diphosphaspiro[5.5]undecane, 3,9-dioctadecan-1-yl-2,4,8,10-tetraoxa-3,9-diphosphaspiro[5.5]undecane, and tris(2,4-di-t-butylphenyl) phosphite. These can be used singly or in combination of two or more types thereof.

As the antioxidant, a phenol-based antioxidant and a phosphorus-based antioxidant are preferably used in combination. Examples of preferred combinations of a phenol-based antioxidant and a phosphorus-based antioxidant include a combination of at least one selected from tetrakis-[methylene-3-(3',5'-di-t-butyl-4'-hydroxyphenyl)propionate] methane and n-octadecyl-3-(3',5'-di-t-butyl-4'-hydroxyphenyl)propionate as the phenol-based antioxidant and tris(2,4-di-t-butylphenyl) phosphite as the phosphorus-based antioxidant.

The content of the antioxidant in the polymerizable composition of the present invention is preferably from 0.01 to 5 parts by mass, more preferably from 0.05 to 3 parts by mass, and still more preferably from 0.1 to 2 parts by mass, per 100 parts by mass of the total amount of the polymerizable composition, from the viewpoint of improving the heat yellowing resistance of the resulting polymer.

As the light stabilizer, a hindered amine-based light stabilizer (HALS) is suitably used. Specific examples of HALS include 2,2,6,6-tetramethyl-4-piperidinyl stearate, 2,2,6,6-tetramethyl-4-piperidyl methacrylate, 1,2,2,6,6-pentamethyl-4-piperidyl methacrylate, bis(2,2,6,6-tetramethyl-1-undecyloxypiperidin-4-yl) carbonate, bis(2,2,6,6-tetramethyl-4-piperidyl) sebacate, bis(1,2,2,6,6-pentamethyl-4-piperidyl) sebacate, ADK STAB LA-68 (available from ADEKA CORPORATION), ADK STAB LA-63P (available from ADEKA CORPORATION), butane-1,2,3,4-tetracarboxylic acid tetrakis(1,2,2,6,6-pentamethyl-4-piperidinyl), 1,2,3,4-butanetetracarboxylic acid tetrakis(2,2,6,6-tetramethyl-4-piperidinyl), and Tinuvin 111FDL, Tinuvin 123, Tinuvin 144, Tinuvin 152, Tinuvin 249, Tinuvin 292, and Tinuvin 5100 (all are available from BASF). These may be used singly, or two or more types thereof may be used in combination.

The content of the light stabilizer in the polymerizable composition of the present invention is preferably from 0.01 to 5 parts by mass, more preferably from 0.05 to 3 parts by mass, and still more preferably from 0.1 to 2 parts by mass, per 100 parts by mass of the total amount of the polymerizable composition, from the viewpoint of improving the heat yellowing resistance and weather resistance of the resulting polymer.

Each of the antioxidant and the light stabilizer can be used singly or in combination of two or more types thereof.

2-4 Method for Producing Polymerizable Composition

The polymerizable composition of the present invention may be produced by mixing the respective components, or may be produced by mixing components other than the polymerization initiator in advance and adding the polymerization initiator immediately before the polymerization reaction.

3. Method for Polymerizing Polymerizable Composition of Present Invention

The method for polymerizing the polymerizable composition of the present invention is not particularly limited, and examples thereof include a method of polymerizing the polymerizable composition through irradiation with an active energy ray and a method of polymerizing the polymerizable composition through heating.

3-1. Polymerization Initiation Method (Active Energy Ray)

When the polymerizable composition of the present invention is subjected to photoradical polymerization, the photoradical polymerization is performed through irradiation with an active energy ray.

The active energy ray to be used is preferably an electron beam or light in a wavelength range from ultraviolet to infrared. When, for example, the active energy ray is an ultraviolet ray, an ultra-high pressure mercury light source or a metal halide light source can be used. When the active energy ray is visible light, a metal halide light source or a halogen light source can be used. When the active energy ray is an infrared ray, a halogen light source can be used. A light source such as a laser or an LED can also be used.

The irradiation dose of the active energy ray is appropriately set depending on the type of the light source, the thickness of the coating film, and the like, and is preferably, appropriately set so that the reaction rate of the total amount of the polymerizable functional groups of compound (1) and an additional polymerizable compound is 80% or more, and more preferably 90% or more. The reaction rate is calculated from a change in absorption peak intensity of the polymerizable functional group before and after the reaction based on an infrared absorption spectrum.

After the polymerization is performed through irradiation with an active energy ray, a heat treatment or an annealing treatment may be performed as necessary to further advance the polymerization. The heating temperature at this time is preferably in a range of 80 to 200°C. The heating time is preferably in a range from 10 to 60 minutes.

3-2. Polymerization Initiation Method (Heating)

When a heat treatment is performed for polymerization of the polymerizable composition of the present invention, the heating temperature is preferably in a range from 80 to 200°C, more preferably in a range from 100 to 150°C. When the heating temperature is lower than 80°C, it is necessary to lengthen the heating time, and economic efficiency tends to be poor. When the heating temperature is higher than 200°C, energy cost is required, and a temperature rise time and a temperature drop time are required. Thus, economic efficiency tends to be poor.

4. Polymer

The polymer of the present invention produced by polymerizing the polymerizable composition of the present invention will be described below.

4-1. Refractive Index

In general, since the overall density is improved through the polymerization reaction, the refractive index of the polymer tends to be higher than that of a compound before polymerization (referred to as a monomer) which is a precursor of the polymer. When the polymerization reaction is sufficiently advanced using a monomer having a high refractive index, the refractive index of the resulting polymer can be increased. Thus, it is considered to be important to improve the refractive index of the polymer by designing the molecular structure of the monomer.

The refractive index shows a large value when evaluated with irradiation light having a short wavelength, but a sample showing a relatively large refractive index at a short wavelength shows a relatively large refractive index even at a long wavelength, and the relationship is not reversed. Thus, it is possible to compare the magnitude of the essential refractive index of the material by evaluating and comparing the refractive index at a constant wavelength. In the present invention, a value at an irradiation light wavelength of 587 nm is used as a reference.

The refractive indexes of compound (1) and the polymer of the present invention are preferably 1.60 or more, more preferably 1.63 or more, particularly preferably 1.65 or more, and most preferably 1.67 or more. The upper limit of the refractive index of compound (1) and the polymer of the present invention is not particularly limited, but is usually 2.0 or less.

When compound (1) and the polymer of the present invention are used as a recording layer material of a hologram recording medium, the refractive indexes of compound (1) and the polymer of the present invention are usually in a range of 1.65 or more and 1.78 or less, and preferably 1.77 or less. When the refractive index is less than 1.65, the diffraction efficiency is low, and the multiplicity is not sufficient. When the refractive index is larger than 1.78, the difference in refractive index with the matrix resin becomes too large, and scattering becomes large. Thus, the transmittance decreases, and more energy is required for recording and reproduction.

In the case of using compound (1) and the polymer of the present invention as an optical material such as a lens, when the refractive index is smaller than 1.60, the central portion of the optical lens or the like becomes thick, and the lightweight property, which is a feature of plastic, may be impaired, which is not preferable. In the development of a precision optical member such as a lens, it is also important to realize optical characteristics suitable for the member by combining optical materials having a plurality of refractive indexes. From this viewpoint, it can be said that monomers and polymers having a refractive index exceeding 1.65 are particularly useful materials for optical components.

4-2. Glass Transition Temperature

The glass transition temperature of the polymer of the present invention is preferably 90°C or higher, more preferably 100°C or higher, still more preferably 110°C or higher, and particularly preferably 120°C or higher, and is preferably 250°C or lower, more preferably 220°C or lower, and still preferably 200°C or lower. When the glass transition temperature is below the above range, the optical properties may change from the designed value under the use environment, and there is a possibility that the practically necessary heat resistance is not satisfied. When the glass transition temperature exceeds the above range, the processability of the polymer deteriorates, and there is a possibility that a molded product having good appearance and high dimensional accuracy cannot be produced. Further, the polymer becomes brittle and the mechanical strength is lowered, and the handleability of the molded product may deteriorate.

5. Optical Material and Optical Component

The compound, polymerizable composition, and polymer of the present invention have excellent performances such as a high refractive index, easy processability, and high chemical stability, and thus can be applied to various optical materials and optical components.

Examples of the optical material include an optical overcoat, a hard coat agent, an optical component adhesive, an optical fiber resin, and an acrylic resin modifier.

Examples of the optical component include a lens, a filter, a diffraction grating, a prism, a light guide, a cover glass for a display device, a photosensor, a photoswitch, an LED, a light-emitting element, an optical waveguide, an optical divider, an optical fiber adhesive, a substrate for a display element, a substrate for a color filter, a substrate for a touch panel, a polarizing plate, a display backlight, a light guide plate, an antireflection film, a viewing angle increasing film, optical recording, stereolithography, and optical relief printing.

They may be used as layers of these optical components. Examples thereof include a display protective film.

Among these, in particular, compound (1) and the polymer of the present invention can be preferably applied to a plastic lens, because of the high refractive index characteristics of compound (1) and the polymer. Examples of the lens include an imaging lens of a camera (in-vehicle camera, digital camera, PC camera, mobile phone camera, monitoring camera, or the like), an eyeglass lens, a light beam condensing lens, and a light diffusion lens.
In the lens using compound (1) and the polymer of the present invention, physical or chemical treatments such as surface polishing, antistatic treatment, hard coating treatment, non-reflection coating treatment, and dyeing treatment can be performed for improvement in antireflection, provision of high hardness, improvement in abrasion resistance, improvement in chemical resistance, provision of antifogging properties, or provision of fashion properties as necessary.

6. Hologram Recording Medium

The polymerizable composition of the present invention can be suitably used for a recording layer of a hologram recording medium. In such a case, the polymerizable composition of the present invention is preferably a photoreactive composition containing a matrix resin, a photopolymerization initiator, a radical scavenger, and an additional additive besides the compound of the present invention. Hereinafter, details of the use as a material for a hologram recording medium will be described.

6-1. Matrix Resin

The polymerizable composition of the present invention preferably contains a matrix resin. In particular, the matrix resin constituting a recording layer of a hologram recording medium is an organic substance that does not chemically or physically change to a large extent through light irradiation, and is mainly composed of a polymer of an organic compound.
The matrix resin constitutes the polymerizable composition of the present invention together with the polymerizable compound described above, the photopolymerization initiator described below, and the like. Thus, the matrix resin is strongly required to have excellent compatibility with the polymerizable compound, the photopolymerization initiator, and the like. When the compatibility between the matrix resin and the aforementioned other components is low, an interface is formed between the materials, and light is refracted or reflected at the interface, which causes leakage of light to an unnecessary portion. As a result, there is a possibility that the interference fringes are distorted or cut and recorded in an inappropriate portion, thereby causing deterioration of information. As described in, for example, JP 3737306 B, the compatibility between the matrix resin and the aforementioned other components can be evaluated based on scattered light intensity obtained by installing a detector in a direction different from the transmitted light with respect to the sample.
As the matrix resin of the polymerizable composition of the present invention, a resin composed of a plurality of materials soluble in a solvent in the polymerizable composition and three-dimensionally crosslinked after being formed in a use state may be used, and examples thereof include thermoplastic resins, thermosetting resins, and photocurable resins described below.
The three-dimensionally crosslinked resin is solvent-insoluble, and is a reaction cured product of a polymerizable compound that is liquid at normal temperature and a compound reactive with the polymerizable compound. Since the three-dimensionally crosslinked resin becomes a physical obstacle, a volume change at the time of recording is suppressed. That is, in the recording layer after recording, the bright portion expands, the dark portion contracts, and unevenness tends to occur on the surface of the hologram recording medium. To suppress such a volume change, it is more preferable to use a polymerizable composition containing a three-dimensionally crosslinked resin matrix for the recording layer.
In particular, a thermosetting resin is preferable as the matrix resin from the viewpoint of adhesion to the support. Hereinafter, a resin material that can be used as the matrix resin will be described in detail.

6-1-1. Thermoplastic Resin

Specific examples of the material of the thermoplastic resin include chlorinated polyethylene, polymethyl methacrylate resin (PMMA), a copolymer of methyl methacrylate and another acrylic acid alkyl ester, a copolymer of vinyl chloride and acrylonitrile, polyvinyl acetate resin (PVAC), polyvinyl alcohol, polyvinyl formal, polyvinyl pyrrolidone, cellulose resins such as ethyl cellulose and nitrocellulose, polystyrene resin, and polycarbonate resin. One type of these may be used singly, or two or more types thereof may be used in combination.
The solvent for such a thermoplastic resin is not particularly limited as long as the solvent dissolves the resin. Examples of the solvent include ketones such as acetone and methyl ethyl ketone, esters such as butyl acetate and propylene glycol methyl ether acetate, aromatic hydrocarbons such as toluene and xylene, ethers such as tetrahydrofuran and 1,2-dimethoxyethane, and amides such as N,N-dimethylacetamide and N-methylpyrrolidone. One type of these may be used singly, or two or more types thereof may be used in combination.

6-1-2. Thermosetting Resin

When a thermosetting resin is used as the matrix resin, the curing temperature varies depending on the type of crosslinking agent or catalyst.

Typical examples of combinations of functional groups that cure at room temperature include epoxy and amine, epoxy and thiol, and isocyanate and amine. Typical examples in a case where a catalyst is used include epoxy and phenol, epoxy and acid anhydride, and isocyanate and polyol.

The former is convenient because it reacts immediately after mixing. However, when molding of a hologram recording medium is involved, adjustment is difficult because there is no time. In contrast, the latter is suitable for curing involving molding of a hologram recording medium because the curing temperature and the curing time can be freely selected by appropriately selecting the type and amount of the catalyst used. For these, various types of resin raw materials are commercially available from low-molecular-weight to high-molecular-weight resins. Thus, the resin can be selected while maintaining compatibility with a polymerizable reactive compound or a photoinitiator, adhesion to a substrate, and the like.

Raw materials will be described below. One type of the raw materials may be used singly, or two or more types thereof may be used in combination.

Epoxy

Examples of the epoxy include polyglycidyl ether compounds of polyols such as (poly)ethylene glycol, (poly) propylene glycol, (poly)tetramethylene glycol, trimethylolpropane, and glycerin, alicyclic epoxy compounds having a 4 to 7-membered cyclic aliphatic group, such as 3,4-epoxycyclohexylmethyl-3,4-epoxycyclohexane carboxylate and 3,4-epoxy-1-methylcyclohexyl-3,4-epoxy-1-methylhexane carboxylate, bisphenol A type epoxy compounds, hydrogenated bisphenol A type epoxy compounds, bisphenol F type epoxy compounds, and phenol or cresol novolac type epoxy compounds.

The epoxy preferably has two or more epoxy groups in one molecule, but the type thereof is not particularly limited. When the number of epoxy groups is small, the hardness required as a matrix is not achieved in some cases. The upper limit of the number of epoxy groups in one molecule is not particularly limited, but is usually 8 or less, and particularly preferably 4 or less. When the number of epoxy groups is too large, it may take a lot of time to consume the epoxy groups, and the matrix resin formation may take too long.

Amine

As the amine, an amine containing a primary amino group or a secondary amino group can be used. Examples of such amines include aliphatic polyamines such as ethylenediamine, diethylenetriamine, and derivatives thereof, alicyclic polyamines such as isophoronediamine, menthanediamine, N-aminoethylpiperazine, and derivatives thereof, aromatic polyamines such as m-xylylenediamine, diaminodiphenylmethane, and derivatives thereof, polyamides such as condensates of dicarboxylic acids such as dimer acids and the above-described polyamines, imidazole compounds such as 2-methylimidazole and derivatives thereof, dicyandiamide, and adipic acid dihydrazide.

Thiol

Examples of the thiol include dithiols such as 1,3-butanedithiol, 1,4-butanedithiol, 2,3-butanedithiol, 1,2-benzenedithiol, 1,3-benzenedithiol, 1,4-benzenedithiol, 1,10-decanedithiol, 1,2-ethanedithiol, 1,6-hexanedithiol, and 1,9-nonanedithiol, and thiol compounds such as polythiols, including Thiokol (available from Toray Fine Chemicals Co., Ltd.) and jER CURE QX40 (available from Mitsubishi Chemical Corporation). Among these, commercially available fast-curing polythiols such as jER CURE QX40 are suitably used.

Phenol

Examples of the phenol include bisphenol A, a novolac type phenol resin, and a resol type phenol resin.

Acid Anhydride

Examples of the acid anhydride include monofunctional acid anhydrides such as phthalic anhydride, tetrahydrophthalic anhydride, and derivatives thereof, and difunctional acid anhydrides such as pyromellitic anhydride, benzophenone tetracarboxylic anhydride, and derivatives thereof.

Amount of Amine, Thiol, Phenol, or Acid Anhydride Used

The amount of the amine, thiol, phenol, or acid anhydride used is usually 0.1 equivalents or more, particularly preferably 0.7 equivalents or more, and usually 2.0 equivalents or less, particularly preferably 1.5 equivalents or less, in terms of a proportion relative to the number of moles of the epoxy group. When the amount of the amine, thiol, phenol, or acid anhydride used is too small or too large, the number of unreacted functional groups becomes large, and storage stability may be impaired.

Polymerization Initiator for Thermosetting Resin

As a catalyst for curing the thermosetting resin, an anionic polymerization initiator and a cationic polymerization initiator can be used depending on the curing temperature and the curing time.

The anionic polymerization initiator generates an anion with heat or irradiation with an active energy ray, and examples thereof include amines. Examples of amines include amino group-containing compounds such as dimethylbenzylamine, dimethylaminomethylphenol, and 1,8-diazabicyclo[5.4.0]undecene-7, and derivatives thereof; and imidazole compounds such as imidazole, 2-methylimidazole, and 2-ethyl-4-methylimidazole, and derivatives thereof. One type or a plurality of types of these can be used depending on the curing temperature and the curing time.

The cationic polymerization initiator generates a cation with heat or irradiation with an active energy ray, and examples thereof include aromatic onium salts. Specific examples include compounds composed of anion components such as $SbF_6$-, $BF_4$-, $AsF_6$-, $PF_6$-, $CF_3SO_3$-, and $B(C_6F_5)_4$- and aromatic cation components containing atoms such as iodine, sulfur, nitrogen, and phosphorus. Among these, a diaryliodonium salt, a triarylsulfonium salt, and the like are preferable. One type or a plurality of types of these can be used depending on the curing temperature and the curing time.

The amount of the polymerization initiator for a thermosetting resin to be used is usually 0.001 mass% or more, particularly 0.01 mass% or more, and usually 50 mass% or less, particularly preferably 10 mass% or less relative to the matrix resin. When the amount of the polymerization initiator for a thermosetting resin to be used is excessively small, the concentration of the polymerization initiator for a thermosetting resin is too low, and thus the polymerization reaction may take too long. When the amount of the polymerization initiator for a thermosetting resin to be used is excessively large, a continuous ring-opening reaction may not occur as a polymerization reaction in some cases.

Isocyanate

An isocyanate having two or more isocyanate groups in one molecule is preferable, but the type thereof is not particularly limited. When the number of isocyanate groups in one molecule is small, the hardness required as a matrix resin is not achieved in some cases. The upper limit of the number of isocyanate groups in one molecule is not particularly limited, but is usually 8 or less, and particularly preferably 4 or less. When the number of isocyanate groups in one molecule is too large, it may take a lot of time to consume the isocyanate groups, and the matrix resin formation may take too long. The upper limit of the number of isocyanate groups in one molecule is not particularly limited, but is usually about 20 or less.

Examples of the isocyanate include aliphatic isocyanates such as hexamethylene diisocyanate, lysine methyl ester diisocyanate, and 2,4,4-trimethylhexamethylene diisocyanate; alicyclic isocyanates such as isophorone diisocyanate and 4,4'-methylenebis(cyclohexyl isocyanate); aromatic isocyanates such as tolylene diisocyanate, 4,4'-diphenylmethane diisocyanate, xylylene diisocyanate, and naphthalene-1,5'-diisocyanate; and multimers thereof. Among these, trimers to heptamers are preferable.

Examples of the isocyanate include, in addition to the above, reaction products of water, or polyhydric alcohols such as trimethylolethane and trimethylolpropane with the above isocyanates, multimers of hexamethylene diisocyanate, or derivatives thereof.

The molecular weight of the isocyanate is preferably 100 or more and 50000 or less, more preferably 150 or more and 10000 or less, and still more preferably 150 or more and 5000 or less, in terms of number average molecular weight. When the number average molecular weight is excessively small, the hardness of the matrix resin may be excessively high because of an increase in crosslinking density, leading to a decrease in recording speed. When the number average molecular weight is excessively large, the compatibility with other components is reduced or the crosslinking density is reduced, and thus the hardness of the matrix resin is excessively reduced and the recorded contents may disappear.

Polyol

Examples of the polyol include polypropylene polyol, polycaprolactone polyol, polyester polyol, and polycarbonate polyol.

Polypropylene Polyol

The polypropylene polyol is obtained through reaction of propylene oxide with a diol or a polyhydric alcohol. Examples of the diol or the polyhydric alcohol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol. Examples of commercially available polypropylene polyols include SANNIX GP-400 and GP-1000 (all are available from Sanyo Chemical Industries, Ltd., trade names), and ADEKA POLYETHER G400, G700, and G1500 (all are available from ADEKA CORPORATION, trade names).

Polycaprolactone Polyol

The polycaprolactone polyol is obtained through reaction of a lactone with a diol or a polyhydric alcohol. Examples of the lactone include $\alpha$-caprolactone, $\beta$-caprolactone, $\gamma$-caprolactone, $\varepsilon$-caprolactone, $\alpha$-methyl-$\varepsilon$-caprolactone, and $\beta$-methyl-$\varepsilon$-caprolactone.
Examples of the diol or the polyhydric alcohol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol.
Examples of commercially available polycaprolactone polyols obtained from the reaction of $\varepsilon$-caprolactone include PLACCEL 205, PLACCEL 205H, PLACCEL 205U, PLACCEL 205UT, PLACCEL 210, PLACCEL 210N, PLACCEL 210CP, PLACCEL 220, PLACCEL 230, PLACCEL 230N, PLACCEL 240, PLACCEL 220EB, PLACCEL 220EC, PLACCEL 303, PLACCEL 305, PLACCEL 308, PLACCEL 309, PLACCEL 312, PLACCEL 320, PLACCEL 401, PLACCEL L205AL, PLACCEL L212AL, PLACCEL L220AL, PLACCEL L320AL, PLACCEL T2103, PLACCEL T2205, PLACCEL P3403, and PLACCEL 410 (all are available from Daicel Corporation, trade names).

Polyester Polyol

Examples of the polyester polyol include those obtained through polycondensation of a dicarboxylic acid or an anhydride thereof with a polyol.
Examples of the dicarboxylic acid include succinic acid, adipic acid, sebacic acid, azelaic acid, dimer acid, maleic anhydride, isophthalic acid, terephthalic acid, and trimellitic acid.
Examples of the polyol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol.
Examples of the polyester polyol include polyethylene adipate, polybutylene adipate, and polyhexamethylene adipate. Examples of commercially available polyester polyols include ADEKA NEWACE F series, ADEKA NEWACE Y series, and ADEKA NEWACE NS series (available from ADEKA Corporation, trade names), and KURARAY POLYOL N-2010, P-4011, and P-1020 (all are available from KURARAY CO., LTD., trade names).

Polycarbonate Polyol

Examples of the polycarbonate polyol include those obtained through a dealcoholization condensation reaction between glycols and dialkyl carbonates (e.g., dimethyl carbonate and diethyl carbonate), those obtained through a dephenolization condensation reaction between glycols and diphenyl carbonates, and those obtained through a deglycolization condensation reaction between glycols and carbonates (e.g., ethylene carbonate and diethyl carbonate).
Examples of the glycols include aliphatic diols such as 1,6-hexanediol, diethylene glycol, propylene glycol, 1,4-butanediol, 3-methyl-1,5-pentanediol, and neopentyl glycol, and alicyclic diols such as 1,4-cyclohexanediol and 1,4-cyclohexanedimethanol.
Examples of the polycarbonate polyol include poly(hexamethylene carbonate) polyol obtained through a condensation reaction of 1,6-hexanediol and diethyl carbonate, poly(pentylene carbonate) obtained through a condensation reaction of pentanediol and diethyl carbonate, and poly(butylene carbonate) obtained through a condensation reaction of 1,4-butanediol and diethyl carbonate.
Examples of commercially available polycarbonate polyols include PLACCEL CD CD205, PLACCEL CD CD210, and PLACCEL CD CD220 (all are available from Daicel Corporation, trade names), and DURANOL T5651, DURANOL T5652, and DURANOL T5650J (all are available from Asahi Kasei Corporation, trade names).

Molecular Weight of Polyol

The molecular weight of the polyol described above is preferably 100 or more and 50000 or less, more preferably 150 or more and 10000 or less, and still more preferably 150 or more and 5000 or less in terms of number average molecular weight. When the number average molecular weight is excessively small, the hardness of the matrix resin may be excessively high because of an increase in crosslinking density, leading to a decrease in recording speed. When the number average molecular weight is excessively large, the hardness of the matrix resin may become too low, and the recorded contents may disappear, because of a decrease in compatibility with other components or a decrease in crosslinking density.

Additional Component

The matrix resin in the present embodiment may contain an additional component in addition to the above-described components as long as it does not contradict the gist of the present invention.
Examples of such an additional component include compounds having a hydroxyl group, such as ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, trimethylolpropane, polyethylene glycol, and polytetramethylene glycol, which are used for the purpose of changing the physical properties of the matrix resin.

Urethane Polymerization Catalyst

To promote the reaction of the isocyanate and the polyol, an appropriate urethane polymerization catalyst may be contained.
Examples of the urethane polymerization catalyst include onium salts such as bis(4-t-butylphenyl)iodonium perfluoro-1-butanesulfonate, bis(4-t-butylphenyl)iodonium p-toluenesulfonate, bis(4-t-butylphenyl)iodonium trifluoromethanesulfonate, (4-bromophenyl)diphenylsulfonium triflate, (4-t-butylphenyl)diphenylsulfonium trifluoromethanesulfonate, diphenyliodonium perfluoro-1-butanesulfonate, (4-fluorophenyl)diphenylsulfonium trifluoromethanesulfonate, diphenyl-4-methylphenylsulfonium trifluoromethanesulfonate, triphenylsulfonium trifluoromethanesulfonate, and bis(alkylphenyl)iodonium hexafluorophosphonate, catalysts mainly composed of Lewis acids such as zinc chloride, tin chloride, iron chloride, aluminum chloride, and $BF_3$, protic acids such as hydrochloric acid and phosphoric acid, amines such as trimethylamine, triethylamine, triethylenediamine, dimethylbenzylamine, and diazabicycloundecene, imidazoles such as 2-methylimidazole, 2-ethyl-4-methylimidazole, and 1-cyanoethyl-2-undecylimidazolium trimellitate, bases such as sodium hydroxide, potassium hydroxide, and potassium carbonate, tin catalysts such as dibutyltin laurate, dioctyltin laurate, and dibutyltin octoate, bismuth catalysts such as tris(2-ethylhexanoate)bismuth and tribenzoyloxybismuth, and zirconium catalysts such as tetrakis(ethylacetoacetate)zirconium, 1,1'-isopropylidenezirconocene dichloride, and tetrakis (2,4-pentanedionato)zirconium.
Among these, a bismuth catalyst and a zirconium catalyst are preferable for improving storage stability.
The bismuth-based catalyst is not particularly limited as long as it is a catalyst containing a bismuth element and is a compound that promotes the reaction of isocyanate and polyol.
Examples of the bismuth-based catalyst include tris(2-ethylhexanoate)bismuth, tribenzoyloxybismuth, bismuth triacetate, tris(dimethyldithiocarbamic acid)bismuth, bismuth hydroxide, triphenylbismuth(V) bis(trichloroacetate), tris(4-methylphenyl)oxobismuth(V), and triphenylbis(3-chlorobenzoyloxy)bismuth(V).
Among these, a trivalent bismuth compound is preferable from the viewpoint of catalytic activity, and a bismuth carboxylate represented by the general formula $Bi(OCOR)_3$ (wherein R represents a linear or branched alkyl group, a cycloalkyl group, or a substituted or unsubstituted aromatic group) is more preferable. Any one type of the bismuth-based catalysts may be used singly, or two or more types thereof may be used in any combination and proportions.
The zirconium-based catalyst is not particularly limited as long as it is a catalyst containing a zirconium element and is a compound that promotes the reaction of isocyanate and polyol.
Examples thereof include cyclopentadienylzirconium trichloride, decamethylzirconocene dichloride, 1,1'-dibutylzirconocene dichloride, 1,1'-isopropylidenezirconocene dichloride, tetrakis(2,4-pentanedionato)zirconium, tetrakis(trifluoro-2,4-pentanedionato)zirconium, tetrakis(hexafluoro-2,4-pentanedionato)zirconium, zirconium butoxide, zirconium-t-butoxide, zirconium propoxide, zirconium isopropoxide, zirconium ethoxide, bis(ethylacetoacetate)dibutoxyzirconium, tetrakis(ethylacetoacetate)zirconium, zirconium oxide, barium zirconium oxide, calcium zirconium oxide, zirconium bromide, zirconium chloride, zirconium fluoride, (indenyl)zirconium dichloride, and zirconium carbonate.
Among these, a compound having an organic ligand is preferable from the viewpoint of compatibility with other components, and a compound having an alkoxide or an acetylacetonate (2,4-pentanedionato) structure is more preferable.

Any one type of the zirconium compounds may be used singly, or two or more types thereof may be used in any combination and proportions.

The bismuth-based catalyst and the zirconium-based catalyst may each be used singly or in combination.

The amount of the urethane polymerization catalyst used is usually 0.0001 mass% or more, particularly 0.001 mass% or more, and usually 10 mass% or less, particularly preferably 5 mass% or less in terms of a proportion relative to the matrix resin. When the amount of the urethane polymerization catalyst used is excessively small, curing may take too long. Meanwhile, when the amount of use is excessively large, it may be difficult to control the curing reaction.

Curing can be performed at room temperature by using a urethane polymerization catalyst, but it may be cured by increasing the temperature. The temperature at this time is preferably between 40°C and 90°C.

6-1-3. Photocurable Resin

When a photocurable resin is used as the matrix resin, it is necessary to cure the matrix resin using a photoinitiator for the matrix resin in accordance with the wavelength to be used. It is desirable that the curing reaction is stable around room temperature, which is a temperature at which the work is mainly performed, because the curing occurs during the light irradiation, and problems are caused in molding and adhesion. From this point of view, it can be said that catalytic curing with a photoinitiator for a matrix resin is a desirable selection.

In general, an active substrate of either a cation or an anion is generated from the photoinitiator for a matrix resin through light irradiation. Thus, it is considered to be preferable to select one that causes curing with such an active substrate and cure the selected one to form a matrix resin.

Examples of the functional group that reacts with a cation such as a proton include an epoxy group and an oxetanyl group. Examples of the compound having these include, as ones having an epoxy group, polyglycidyl ether compounds of polyols such as (poly)ethylene glycol, (poly)propylene glycol, (poly)tetramethylene glycol, trimethylolpropane, and glycerin, alicyclic epoxy compounds having a 4 to 7-membered cyclic aliphatic group such as 3,4-epoxycyclohexylmethyl-3,4-epoxycyclohexanecarboxylate and 3,4-epoxy-1-methylcyclohexyl-3,4-epoxy-1-methylhexanecarboxylate, bisphenol A type epoxy compounds, hydrogenated bisphenol A type epoxy compounds, bisphenol F type epoxy compounds, and phenol or cresol novolac type epoxy compounds. Examples of those having an oxetanyl group include 2-ethyl-2-oxetanyl ether of bisphenol A and 1,6-bis(2-ethyl-2-oxetanyloxy)hexane. (Here, the term "(poly)ethylene glycol" or the like refers to both "ethylene glycol" and its polymer "polyethylene glycol".)

Examples of the functional group that reacts with an anion include an epoxy group and an episulfide group. Specific examples of the compound having an episulfide group include phenyl episulfide and diepisulfide methyl ether of bisphenol A.

The amount of the photoinitiator for a matrix resin used in the case of photocuring the matrix resin described above is usually 0.01 mass% or more, particularly 0.1 mass% or more, and usually 1 mass% or less, particularly preferably 0.5 mass% or less in terms of a proportion relative to the polymerizable compound. When the amount of the photoinitiator for a matrix resin used is excessively small, curing may take too long. When the amount of the photoinitiator for a matrix resin used is excessively large, it may be difficult to control the curing reaction.

Particularly when the matrix resin is used as a hologram recording material, it is important that the wavelength at the time of curing and the wavelength at the time of recording are different because light is irradiated also at the time of recording. The difference in wavelength is at least 10 nm, preferably 30 nm. The selection of the photoinitiator for a matrix resin can be generally predicted from the absorption wavelength of the initiator.

6-2. Photopolymerization Initiator

As the photopolymerization initiator that assists the polymerization of the compound of the present invention, any known photoradical polymerization initiator can be used. Examples include azo compounds, azide compounds, organic peroxides, organic borates, onium salts, bisimidazole derivatives, titanocene compounds, iodonium salts, organic thiol compounds, halogenated hydrocarbon derivatives, acetophenones, benzophenones, hydroxybenzenes, thioxanthones, anthraquinones, ketals, acylphosphine oxides, sulfone compounds, carbamic acid derivatives, sulfonamides, triarylmethanols, and oxime esters. Among these, as the photopolymerization initiator, a titanocene compound, an acylphosphine oxide compound, an oxime ester compound, or the like are preferable because a polymerization reaction occurs with light in a visible region.

6-2-1. Titanocene Compound

When a titanocene compound is used as the photopolymerization initiator, the type thereof is not particularly limited. For example, the titanocene compound can be appropriately selected and used from various titanocene compounds described in JP S59-152396 A, JP S61-151197 A, and the like.

Specific examples of the titanocene compound include di-cyclopentadienyl-Ti-di-chloride, di-cyclopentadienyl-Ti-bis-phenyl, di-cyclopentadienyl-Ti-bis-2,3,4,5,6-pentafluorophen-1-yl, dicyclopentadienyl-Ti-bis-2,3,5,6-tetrafluorophen-1-yl, di-cyclopentadienyl-Ti-bis-2,4,6-trifluorophen-1-yl, di-cyclopentadienyl-Ti-bis-2,6-di-fluorophen-1-yl, di-cyclopentadienyl-Ti-bis-2,4-di-fluorophen-1-yl, di-methylcyclopentadienyl-Ti-bis-2,3,4,5,6-pentafluorophen-1-yl, di-methylcyclopentadienyl-Ti-bis-2,3,5,6-tetrafluorophen-1-yl, di-methylcyclopentadienyl-Ti-bis-2,6-difluorophen-1-yl, and di-cyclopentadienyl-Ti-bis-2,6-difluoro-3-(pyr-1-yl)-phen-1-yl.

6-2-2. Acylphosphine Oxide Compound

Specific examples of the acylphosphine oxide compound include a monofunctional initiator having only one cleavage point with light in one molecule, and a bifunctional initiator having two cleavage points with light in one molecule.
Examples of the monofunctional initiator include triphenylphosphine oxide, diphenyl(2,4,6-trimethylbenzoyl) phosphine oxide, and 2,6-dichlorobenzoyldiphenylphosphine oxide.
Examples of the bifunctional initiator include bis(2,4,6-trimethylbenzoyl)-phenylphosphine oxide, bis(2,6-dimethoxybenzoyl)-2,4,4-trimethylpentylphosphine oxide, bis(2,6-dichlorobenzoyl)-4-propylphenylphosphine oxide, and bis(2,6-dichlorobenzoyl)-2,5-dimethylphenylphosphine oxide.

6-2-3. Oxime Ester-Based Compound

Specific examples of the oxime ester-based compound include 1-[4-(phenylthio)-2-(O-benzoyloxime)]-1,2-octanedione, 1-[9-ethyl-6-(2-methylbenzoyl)-9H-carbazol-3-yl]-1-(O-acetyloxime)ethanone, methyl 4-(acetoxyimino)-5-[9-ethyl-6-(2-methylbenzoyl)-9H-carbazol-3-yl]-5-oxopentanoate, methyl 1-(9-ethyl-6-cyclohexanoyl-9H-carbazol-3-yl)-1-(O-acetyloxime)glutarate, methyl 1-(9-ethyl-9H-carbazol-3-yl)-1-(O-acetyloxime)glutarate, and 1-(9-ethyl-9H-carbazol-3-yl)-1-(O-acetyloxime)-3-methyl-butanoic acid.

6-2-4. Amount of Photopolymerization Initiator Used

Any one type of the above-described various photopolymerization initiators may be used singly, or two or more types thereof may be used in any combination and proportions.
The content of the photopolymerization initiator in the polymerizable composition of the present invention is preferably 0.5 μmol/g or more, more preferably 1 μmol/g or more and 100 μmol/g or less, more preferably 50 μmol/g or less in terms of a molar amount per unit weight of the polymerizable composition.
When the content of the photopolymerization initiator is too small, the amount of radicals to be generated is reduced. Thus, the rate of photopolymerization is reduced, and the recording sensitivity in the hologram recording medium may be lowered. When the content of the photopolymerization initiator is too large, radicals generated through light irradiation are recombined, or disproportionation occurs. Thus, contribution to photopolymerization decreases, and recording sensitivity in the hologram recording medium may also decrease. When two or more photopolymerization initiators are used in combination, it is preferable that the total amount thereof satisfies the above range.

6-3. Radical Scavenger

In the hologram recording, a radical scavenger may be added to accurately fix the interference light intensity pattern as a polymer distribution in the hologram recording medium. The radical scavenger preferably has both a functional group that traps radicals and a reactive group that is fixed to the matrix resin with a covalent bond. Examples of the functional group that traps a radical include a stable nitroxyl radical group.

6-3-1. Type of Radical Scavenger

Examples of the reactive group fixed to the matrix resin with a covalent bond include a hydroxyl group, an amino group, an isocyanate group, and a thiol group. Examples of such a radical scavenger include 4-hydroxy-2,2,6,6-tetramethylpiperidine-1-oxyl free radical (TEMPOL), 3-hydroxy-9-azabicyclo[3.3.1]nonane N-oxyl, 3-hydroxy-8-azabicyclo[3.2.1]octane N-oxyl, and 5-HO-AZADO:5-hydroxy-2-azatricyclo[3.3.1.1$^{3,7}$]decane N-oxyl.

6-3-2. Content of Radical Scavenger

Any one type of the various radical scavengers may be used singly, or two or more types thereof may be used in any combination and proportions.
The content of the radical scavenger in the polymerizable composition of the present invention is preferably 0.5

μmol/g or more, more preferably 1 μmol/g or more and 100 μmol/g or less, more preferably 50 μmol/g or less in terms of a molar amount per unit weight of the polymerizable composition.

When the content of the radical scavenger is too small, the efficiency of trapping radicals is reduced, and there is a tendency that a polymer having a low polymerization degree is diffused to increase the number of components that do not contribute to a signal. When the content of the radical scavenger is too large, the polymerization efficiency of the polymer decreases, and signal recording tends to be impossible. When two or more radical scavengers are used in combination, the total amount thereof preferably satisfies the above range.

6-4. Additional Component

The polymerizable composition of the present invention may contain an additional component besides the above components as long as it does not contradict the gist of the present invention.

Examples of the additional component include a solvent, a plasticizer, a dispersant, a leveling agent, an antifoaming agent, and an adhesion promoter for preparing the polymerizable composition, and particularly in the case where the polymerizable composition is used for a hologram recording medium, include a chain transfer agent, a polymerization terminator, a compatibilizer, a reaction auxiliary agent, and a sensitizer for controlling the reaction of recording. Examples of other additives that may be required for improving characteristics include an antiseptic, a stabilizer, an antioxidant, an ultraviolet absorber, and a light stabilizer. Any one type of these components may be used singly, or two or more types of these components may be used in any combination and proportions.

Sensitizer

A compound that controls excitation of the photopolymerization initiator can be added to the polymerizable composition of the present invention. Examples of such a compound include a sensitizer and a sensitization aid.

As the sensitizer, any sensitizer can be selected from various known sensitizers and used. In general, as the sensitizer, a colored compound such as a dye is often used to absorb visible and ultraviolet laser light. In the case of using in a hologram recording medium, although depending on the wavelength of laser light used for recording and the type of initiator used, in a system using a green laser, specific examples of preferable sensitizers include compounds described in JP H5-241338 A, JP H2-69 A, JP H2-55446 B, and the like. In a system using a blue laser, examples thereof include compounds described in JP 2000-10277 A, JP 2004-198446 A, and the like. Any one type of these sensitizers may be used singly, or two or more types thereof may be used in any combination and proportions.

When colorless transparency is required for the resulting hologram recording medium, it is preferable to use a cyanine pigment as a sensitizer. Since a cyanine pigment is easily decomposed by light in general, post-exposure is performed. That is, when the hologram recording medium is left under indoor light or sunlight for several hours to several days, the cyanine pigment in the hologram recording medium is decomposed and does not have absorption in the visible range, and thus, a colorless and transparent hologram recording medium can be obtained.

The amount of the sensitizer needs to be increased or decreased depending on the thickness of the recording layer to be formed, and is preferably in a range of usually 0.01 mass% or more, particularly 0.1 mass% or more, and usually 10 mass% or less, particularly 5 mass% or less in terms of a proportion relative to the photopolymerization initiator described above in 6-2. When the amount of the sensitizer used is too small, the starting efficiency decreases, and recording may take a lot of time. When the amount of the sensitizer used is too large, absorption of light to be used for recording and reproduction increases, and it may be difficult for light to reach in the depth direction. When two or more sensitizers are used in combination, the total amount thereof is set to satisfy the above range.

Plasticizer

To improve the reaction efficiency and adjust the physical properties of the recording layer of the hologram recording medium, the polymerizable composition of the present invention may contain a plasticizer.

Examples of the plasticizer include phthalic acid esters such as dioctyl phthalate, diisononyl phthalate, diisodecyl phthalate, and diundecyl phthalate; adipic acid esters such as bis(2-ethylhexyl) adipate, diisononyl adipate, and din-butyl adipate; sebacic acid esters such as dioctyl sebacate and dibutyl sebacate; phosphoric acid esters such as tricresyl phosphate; citric acid esters such as tributyl acetylcitrate; trimellitic acid esters such as trioctyl trimellitate; alkoxylated (poly)alkylene glycol esters such as epoxidized soybean oil, chlorinated paraffin, and acetoxymethoxypropane; and terminal alkoxylated polyalkylene glycols such as dimethoxypolyethylene glycol.

A plasticizer containing a fluorine element as exemplified in JP 6069294 B can also be used. Examples of the plasticizer containing a fluorine element include 2,2,2-trifluoroethylbutyl carbamate, bis(2,2,2-trifluoroethyl)-(2,2,4-trimethylhexane-1,6-diyl) biscarbamate, bis(2,2,2-trifluoroethyl)-[4-({[(2,2,2-trifluoroethoxy)carbonyl]amino}-methyl)octane-1,8-diyl] biscarbamate, 2,2,3,3,4,4,5,5,6,6, 7, 7,8,8,9,9-hexadecafluorononylbutyl carbamate, and 2,2,2-trifluoroethylphenyl carbamate.

Such a plasticizer is usually used in a range of 0.01 mass% or more and 50 mass% or less, and preferably 0.05 mass% or more and 20 mass% or less in terms of a proportion relative to the total solid content of the polymerizable composition. When the content of the plasticizer is smaller than this range, the effect of improving the reaction efficiency and adjusting the physical properties are not exhibited. When the content of the plasticizer is more than this range, the transparency of the recording layer is lowered, or bleeding out of the plasticizer becomes remarkable.

Leveling Agent

In the polymerizable composition of the present invention, a leveling agent can be used. Examples of the leveling agent include polycarboxylic acid sodium salts, polycarboxylic acid ammonium salts, polycarboxylic acid amine salts, silicon leveling agents, acrylic leveling agents, ester compounds, ketone compounds, and fluorine compounds. Any one type of these may be used singly, or two or more types thereof may be used in any combination and proportions.

Chain Transfer Agent

In the polymerizable composition of the present invention, a chain transfer agent can be used. Examples of the chain transfer agent include phosphinates such as sodium phosphite and sodium hypophosphite, mercaptans such as mercaptoacetic acid, mercaptopropionic acid, 2-propanethiol, 2-mercaptoethanol, and thiophenol, aldehydes such as acetaldehyde and propionaldehyde, ketones such as acetone and methyl ethyl ketone, halogenated hydrocarbons such as trichloroethylene and perchloroethylene, terpenes such as terpinolene, $\alpha$-terpinene, $\beta$-terpinene, and $\gamma$-terpinene, non-conjugated dienes such as 1,4-cyclohexadiene, 1,4-cycloheptadiene, 1,4-cyclooctadiene, 1,4-heptadiene, 1,4-hexadiene, 2-methyl-1,4-pentadiene, 3,6-nonanedien-1-ol, and 9,12-octadecadienol, linolenic acids such as linolenic acid, $\gamma$-linolenic acid, methyl linolenate, ethyl linolenate, isopropyl linolenate, and linolenic anhydride, linoleic acids such as linoleic acid, methyl linoleate, ethyl linoleate, isopropyl linoleate, and linoleic anhydride, eicosapentaenoic acids such as eicosapentaenoic acid and ethyl eicosapentaenoate, and docosahexaenoic acids such as docosahexaenoic acid and ethyl docosahexaenoate.

The amount of such an additive used is usually 0.001 mass% or more, particularly 0.01 mass% or more, and usually 30 mass% or less, particularly preferably 10 mass% or less in terms of a proportion relative to the total solid content of the polymerizable composition of the present embodiment. When two or more additives are used in combination, the total amount thereof is set to satisfy the above range.

6-5. Compositional Proportion of Each Component in Polymerizable Composition

The content of each component in the polymerizable composition of the present invention can be freely determined as long as it does not contradict the gist of the present invention. The proportion of each component described below is preferably in the following range based on the molar amount per unit mass of the polymerizable composition.

When the content of the polymerizable compound is equal to or more than the lower limit value described above, sufficient diffraction efficiency is achieved in the hologram recording medium. When the content of the polymerizable compound is equal to or less than the upper limit value described above, compatibility with the resin matrix in the recording layer is maintained, and shrinkage of the recording layer due to recording tends to be maintained low.

When an isocyanate and a polyol are used as the matrix resin in the polymerizable composition of the present invention, the total content thereof is usually 0.1 mass% or more, preferably 10 mass% or more, more preferably 35 mass% or more, and usually 99.9 mass% or less, preferably 99 mass% or less. With the content set to be equal to or more than the lower limit value described above, the recording layer is easily formed.

In this case, the ratio of the number of isocyanate-reactive functional groups of the polyol to the number of isocyanate groups of the isocyanate is preferably 0.1 or more, more preferably 0.5 or more, usually 10.0 or less, and preferably 2.0 or less. When the ratio is within the above range, the amount of unreacted functional groups is reduced, and storage stability is improved.

In this polymerizable composition, the content of the urethane polymerization catalyst is preferably determined in consideration of the reaction rates of isocyanate and polyol. The content of the urethane polymerization catalyst is preferably 5 mass% or less, more preferably 4 mass% or less, more preferably 1 mass% or less, and preferably 0.003 mass% or more.

The total amount of the additional components other than the above components only needs to be 30 mass% or less, and is preferably 15 mass% or less, and more preferably 5 mass%.

6-6. Method for Producing Polymerizable Composition

In the present invention, the method for producing a polymerizable composition containing a polymerizable compound, a matrix resin, and a photopolymerization initiator is not particularly limited, and the order of mixing and the like can also be appropriately adjusted. When the polymerizable composition contains components other than the above components, the components may be mixed in any combination and order.
When an isocyanate and a polyol are used as the matrix resin, the polymerizable composition can be produced by, for example, the following method, but the present invention is not limited to this method.
In addition to the polymerizable compound and the photopolymerization initiator, all components other than the isocyanate and the urethane polymerization catalyst are mixed to prepare a photoreactive composition (liquid A). The isocyanate and the urethane polymerization catalyst are mixed to prepare liquid B.
The photoreactive composition (liquid A) can also be obtained by mixing all components other than the isocyanate with the polymerizable compound and the photopolymerization initiator.
Dehydration and degassing are preferably performed on each liquid. When dehydration and degassing are insufficient, bubbles are generated at the time of preparing the hologram recording medium, and a uniform recording layer may fail to be obtained in some cases. At the time of dehydration and degassing, heating and decompression may be performed as long as each component is not impaired.
The production of the polymerizable composition obtained by mixing liquid A and liquid B is preferably performed immediately before molding of the hologram recording medium. At this time, it is also possible to use a known mixing technique. When liquid A and liquid B are mixed, degassing may be performed as necessary to remove the residual gas. Further, liquid A and liquid B are preferably subjected to a filtration step to remove foreign matters and impurities, respectively, or after mixing. In this case, it is more preferable to separately filter each liquid.
An isocyanate-functional prepolymer formed from the reaction of an isocyanate having excess isocyanate groups with a polyol can also be used as a matrix resin. Further, an isocyanate-reactive prepolymer formed from the reaction of a polyol having excess isocyanate-reactive functional groups with an isocyanate can also be used as a matrix resin.

6-7. Hologram Recording Medium of Present Invention

The hologram recording medium of the present invention using the polymerizable composition of the present invention includes a recording layer and, as necessary, a support and an additional layer. Usually, the hologram recording medium has a support, and the recording layer and an additional layer are laminated on the support to constitute the hologram recording medium. When the recording layer or the additional layer has strength and durability necessary for the medium, the hologram recording medium may not have a support. Examples of the additional layer include a protective layer, a reflection layer, or an antireflection layer (antireflection film).

6-7-1. Recording Layer

The recording layer of the hologram recording medium of the present invention is a layer formed of the polymerizable composition of the present invention, and is a layer on which information is recorded. Information is usually recorded as a hologram. As described below in the section of the recording method, the polymerizable compound (hereinafter will be referred to as polymerizable monomer) contained in the recording layer partially causes a chemical change such as polymerization through hologram recording or the like. Thus, in the hologram recording medium after recording, a portion of the polymerizable monomer is consumed and present as a compound after reaction, such as a polymer.
The thickness of the recording layer is not particularly limited, and it may be appropriately determined in consideration of the recording method and the like. The thickness of the recording layer is preferably 1 μm or more, more preferably 10 μm or more, and preferably 1 cm or less, more preferably 3 mm or less. When the thickness of the recording layer is set to be equal to or more than the lower limit value described above, the selectivity of each hologram is increased at the time of multiple recording in the hologram recording medium, and the degree of multiple recording tends to be increased. When the thickness of the recording layer is set to be equal to or less than the upper limit value described above, the entire recording layer can be uniformly molded, and multiple recording in which the diffraction efficiency of each hologram is uniform and the S/N ratio is high tends to be performed.
The shrinkage rate of the recording layer due to exposure at the time of recording and reproducing information is preferably 0.25% or less from the viewpoint of recording reproducibility.

6-7-2. Support

The details of the support are not particularly limited as long as the support has strength and durability necessary for the hologram recording medium, and any support can be used.

The shape of the support is not limited either, but is usually formed into a flat plate shape or a film shape.

The material constituting the support is not limited, and the material may be transparent or opaque.

Examples of the transparent material of the support include organic materials such as acrylic, polyethylene terephthalate, polyethylene naphthoate, polycarbonate, polyethylene, polypropylene, amorphous polyolefin, polystyrene, polycycloolefin, and cellulose acetate; and inorganic materials such as glass, silicon, and quartz. Among these, polycarbonate, acrylic, polyester, amorphous polyolefin, glass, and the like are preferable, and in particular, polycarbonate, acrylic, amorphous polyolefin, polycycloolefin, and glass are more preferable.

Examples of the opaque material of the support include a metal such as aluminum; and a material in which a metal such as gold, silver, or aluminum, or a dielectric such as magnesium fluoride or zirconium oxide is coated on the transparent support described above.

The thickness of the support is not particularly limited, but is preferably in a range of 0.05 mm or more and 1 mm or less. When the thickness of the support is equal to or more than the lower limit value described above, the mechanical strength of the hologram recording medium can be achieved, and warpage of the substrate can be prevented. When the thickness of the support is equal to or less than the upper limit value described above, advantages such as an increase in the light transmission amount and a reduction in the weight and cost of the hologram recording medium can be obtained.

The surface of the support may be subjected to a surface treatment. This surface treatment is usually performed to improve adhesion between the support and the recording layer. Examples of the surface treatment include application of a corona discharge treatment to the support and formation of an undercoat layer on the support in advance. Examples of the composition of the undercoat layer include halogenated phenol, a partially hydrolyzed vinyl chloride-vinyl acetate copolymer, and a polyurethane resin.

The surface treatment of the support may be performed for the purpose other than improvement of adhesiveness. Examples thereof include a reflection coating treatment for forming a reflection coating layer made of a metal such as gold, silver, or aluminum; and a dielectric coating treatment for forming a dielectric layer of magnesium fluoride, zirconium oxide, or the like. These layers may be formed as a single layer or two or more layers.

Such a surface treatment may be provided for the purpose of controlling the permeability of gas and moisture of the substrate. For example, the reliability of the hologram recording medium can be improved by providing the support sandwiching the recording layer with a function of suppressing the permeability of gas or moisture.

The support may be provided only on either one of the upper side and the lower side of the recording layer of the hologram recording medium of the present invention, or may be provided on both sides. When the supports are provided on both upper and lower sides of the recording layer, at least one of the supports is formed to be transparent so as to transmit active energy rays (excitation light, reference light, reproduction light, and the like).

In the case of a hologram recording medium having a support on one side or both sides of a recording layer, a transmission type or reflection type hologram can be recorded. When a support having reflection characteristics is used on one side of the recording layer, a reflection type hologram can be recorded.

The support may be provided with patterning for data addresses. The patterning method in this case is not limited. For example, unevenness may be formed on the support itself, a pattern may be formed on a reflection layer described below, or unevenness and a pattern may be formed by combining these methods.

6-7-3. Protective Layer

The protective layer is a layer for preventing deterioration or the like of recording and reproducing characteristics of the recording layer. The specific configuration of the protective layer is not limited, and any known configuration can be applied. For example, a layer made of a water-soluble polymer, an organic/inorganic material, or the like can be formed as the protective layer.

The position at which the protective layer is formed is not particularly limited. The protective layer may be formed, for example, on the surface of the recording layer, between the recording layer and the support, or on the outer surface side of the support. The protective layer may be formed between the support and an additional layer.

6-7-4. Reflection Layer

The reflection layer is formed when the hologram recording medium is formed into a reflection type. In the case of a reflection type hologram recording medium, the reflection layer may be formed between the support and the recording layer, or may be formed on the outer surface of the support. Usually, the reflection layer is preferably positioned between the support and the recording layer.

As the reflection layer, any known layer can be applied, and for example, a metal thin film or the like can be used.

6-7-5. Antireflection Film

For both the transmission type hologram recording medium and the reflection type hologram recording medium, an antireflection film may be provided on a side on which information light, reference light, and reproduction light are incident and emitted, or between the recording layer and the support. The antireflection film functions to improve light utilization efficiency and suppress generation of noise.
As the antireflection film, any known film can be used.

6-7-6. Method for Producing Hologram Recording Medium

The method for producing the hologram recording medium of the present invention is not limited. For example, it can be produced by applying the polymerizable composition of the present invention on a support to form a recording layer without a solvent. In this case, any method can be used as the coating method. Specific examples thereof include a spray method, a spin coating method, a wire bar method, a dip method, an air knife coating method, a roll coating method, a blade coating method, and a doctor roll coating method.
In the formation of the recording layer, in particular, in the case of forming a thick recording layer, a method of putting the recording layer in a mold and molding the recording layer, or a method of coating the recording layer on a release film and punching the layer may be used. The recording layer may be produced by mixing the polymerizable composition of the present invention with a solvent or an additive to prepare a coating liquid, applying the coating liquid onto a support, and drying the coating liquid to form a recording layer. In this case as well, any method can be used as the coating method. For example, a method similar to that described above can be adopted.
The solvent used for the coating liquid is not limited, but it is usually preferable to use a solvent that has sufficient solubility with respect to the components to be used, provides good coating film properties, and does not invade the support such as a resin substrate. One type of the solvents may be used singly, or two or more types of the solvents may be used in any combination and proportions.
The amount of the solvent used is not limited. However, it is preferable to prepare a coating liquid having a solid content concentration of about 1 to 100 mass% from the viewpoint of coating efficiency and handleability.
Examples of the solvent include ketone-based solvents such as acetone, methyl ethyl ketone, methyl isobutyl ketone, cyclohexanone, and methyl amyl ketone; aromatic solvents such as toluene and xylene; alcohol-based solvents such as methanol, ethanol, propanol, n-butanol, heptanol, hexanol, diacetone alcohol, and furfuryl alcohol; ketone alcohol-based solvents such as diacetone alcohol and 3-hydroxy-3-methyl-2-butanone; ether-based solvents such as tetrahydrofuran and dioxane; halogen-based solvents such as dichloromethane, dichloroethane, and chloroform; cello solve-based solvents such as methyl cellosolve, ethyl cellosolve, butyl cellosolve, methyl cellosolve acetate, and ethyl cellosolve acetate; propylene glycol-based solvents such as propylene glycol monomethyl ether, propylene glycol monoethyl ether, propylene glycol monobutyl ether, propylene glycol monomethyl ether acetate, propylene glycol monoethyl ether acetate, propylene glycol monobutyl ether acetate, and dipropylene glycol dimethyl ether; ester-based solvents such as ethyl acetate, butyl acetate, amyl acetate, butyl acetate, ethylene glycol diacetate, diethyl oxalate, ethyl pyruvate, ethyl-2-hydroxybutyrate, ethyl acetoacetate, methyl lactate, ethyl lactate, methyl 2-hydroxyisobutyrate, and methyl 3-methoxypropionate; perfluoroalkyl alcohol-based solvents such as tetrafluoropropanol, octafluoropentanol, and hexafluorobutanol; high-polarity solvents such as dimethylformamide, dimethylacetamide, N-methylpyrrolidone, and dimethyl sulfoxide; chain hydrocarbon-based solvents such as n-hexane and n-octane; cyclic hydrocarbon-based solvents such as cyclohexane, methylcyclohexane, ethylcyclohexane, dimethylcyclohexane, n-butylcyclohexane, tert-butylcyclohexane, and cyclooctane; and mixed solvents thereof.
Examples of the method for producing the hologram recording medium include a production method in which a recording layer is formed by applying a polymerizable composition melted with heat to a support and solidifying the composition by cooling, a production method in which a recording layer is formed by applying a liquid polymerizable composition to a support and curing the composition through thermal polymerization, and a production method in which a recording layer is formed by applying a liquid polymerizable composition to a support and curing the composition through photopolymerization.
The hologram recording medium produced in this manner can take the form of a self-standing slab or a disk, and can be used for a three-dimensional image display device, a diffractive optical element, a large-capacity memory, and the like.
In particular, the hologram recording medium of the present invention using the polymerizable composition of the present invention has high refractive index modulation, and is also useful as an AR glass light guide plate (waveguide). As used herein, AR is an abbreviation for augmented reality.

6-7-7. Application of Hologram Recording Medium

Application as Large-Capacity Memory

Writing (recording) and reading (reproducing) of information into and from the hologram recording medium of the present invention are both performed through irradiation with light.

At the time of recording information, light capable of causing a chemical change of a polymerizable monomer, i.e., polymerization and concentration change thereof is used as object light (also referred to as recording light).

For example, when information is recorded as a volume hologram, the recording layer is irradiated with object light together with reference light, and the object light and the reference light are caused to interfere with each other in the recording layer. With this, the interference light causes polymerization and concentration change of the polymerizable monomer in the recording layer, and as a result, the interference fringes cause a refractive index difference in the recording layer, and the interference fringes recorded in the recording layer are recorded as holograms in the recording layer.

In the case of reproducing the volume hologram recorded on the recording layer, the recording layer is irradiated with predetermined reproduction light (typically, reference light). The irradiated reproduction light is diffracted according to the interference fringes. Since the diffracted light includes information similar to that of the recording layer, the information recorded in the recording layer can be reproduced by reading the diffracted light with an appropriate detection means.

Wavelength regions of the object light, the reproduction light, and the reference light can be determined appropriately depending on each application, and may be a visible light region or an ultraviolet region. Among these types of light, preferable examples include solid lasers such as ruby, glass, Nd-YAG, and Nd-$YVO_4$; diode lasers such as GaAs, InGaAs, and GaN; gas lasers such as helium-neon, argon, krypton, excimer, and CO2; and lasers having excellent monochromaticity and directivity, such as a dye laser having a dye.

The irradiation doses of the object light, the reproduction light, and the reference light are not limited, and the irradiation dose can be determined appropriately as long as recording and reproduction can be performed. When the irradiation dose is extremely small, there is a possibility that the chemical change of the polymerizable monomer is too incomplete to sufficiently express the heat resistance and mechanical properties of the recording layer. Meanwhile, when the irradiation dose is extremely large, the components of the recording layer (the components of the polymerizable composition of the present invention) may deteriorate. Thus, irradiation with the object light, the reproduction light, and the reference light is usually performed in a range of 0.1 $J/cm^2$ or more and 20 $J/cm^2$ or less depending on the composition of the polymerizable composition of the present invention used for formation of the recording layer, the type and blending amount of the photopolymerization initiator, and the like.

Examples of the hologram recording method include a polarized collinear hologram recording method and a reference light incident angle multiple hologram recording method. When the hologram recording medium of the present invention is used as a recording medium, good recording quality can be provided by any recording method.

Application as AR Glass Light Guide Plate (Application as AR Glass Light Guide Plate)

On the hologram recording medium of the present invention, a volume hologram is recorded in the same manner as in the above-described application as large-capacity memory.

For the volume hologram recorded on the recording layer, the recording layer is irradiated with predetermined reproduction light. The irradiated reproduction light is diffracted according to the interference fringes. In this case, even though the wavelength of the reproduction light does not match the wavelength of the recording light, diffraction occurs when the interference fringes and the Bragg condition are satisfied. Thus, the reproduction light in a wide wavelength region can be diffracted as long as the corresponding interference fringes are recorded according to the wavelength and the incident angle of the reproduction light to be diffracted. This can expand the display color gamut of the AR glasses.

When the corresponding interference fringes are recorded according to the wavelength and diffraction angle of the reproduction light, the reproduction light incident from the outside of the hologram recording medium can be guided into the hologram recording medium, the reproduction light guided in the hologram recording medium can be reflected, demultiplexed, enlarged, and reduced, and the reproduction light guided in the hologram recording medium can be emitted to the outside of the hologram recording medium. This can widen the viewing angle of the AR glasses.

The wavelength region of the object light and the reproduction light can be determined appropriately according to each application, and may be a visible light region or an ultraviolet region. Among these types of light, the above-described laser or the like is preferable. The reproduction light is not limited to a laser or the like, and display devices such as a liquid crystal display (LCD) and an organic electroluminescence display (OLED) are also preferable.

The irradiation doses of the object light, the reproduction light, and the reference light are not limited, and the irradiation dose can be determined appropriately as long as recording and reproduction can be performed. When the irradiation dose is extremely small, there is a possibility that the chemical change of the polymerizable monomer is too incomplete to sufficiently express the heat resistance and mechanical properties of the recording layer. Meanwhile, when

the irradiation dose is extremely large, the components of the recording layer (the components of the polymerizable composition of the present invention) may deteriorate. Thus, irradiation with the object light, the reproduction light, and the reference light is usually performed in a range of 0.1 J/cm$^2$ or more and 20 J/cm$^2$ or less depending on the composition of the polymerizable composition of the present invention used for formation of the recording layer, the type and blending amount of the photopolymerization initiator, and the like.

6-8. Performance Index of Hologram Recording Medium

The performance of the hologram recording medium is indicated by total Δn calculated using the sum of diffraction efficiency over the entire multiple recording. In the case of a transmission hologram, the diffraction efficiency of the hologram is given with the ratio of the intensity of the diffracted light to the sum of the transmitted light intensity and the diffracted light intensity. From the obtained diffraction efficiency, Δn is calculated by using the following equation according to Coupled Wave Theory (H. Kogelnik, The Bell System Technical Journal (1969), 48, 2909-2947), and the total sum in the entire multiple recording is defined as total Δn.

[Math. 1]

$$\eta = \sin^2\left(\frac{\pi \cdot T \cdot \Delta n}{\lambda \cdot \cos\theta}\right)$$

$$total\Delta n = \sum \Delta n$$

In the equation, η represents diffraction efficiency, T represents the thickness of a medium, λ represents the wavelength of reference light, and θ represents the incident angle of reference light.

In the case of large-capacity memory, a higher total Δn means that a larger amount of information can be recorded per unit volume, which is preferable. In the case of AR glasses, high total Δn is preferred because the projection image of the projector can be delivered brightly to pupils, power consumption can be suppressed, and the viewing angle can be widened.

## Examples

Hereinafter, the present invention will be described in more detail by way of Examples. The present invention is not limited to the following Examples as long as it does not depart from the gist thereof.

### Raw Materials Used

The composition raw materials used in Examples and Comparative Examples are as follows.

Isocyanate

· DURANATE (registered trademark) TSS-100: hexamethylene diisocyanate-based polyisocyanate (NCO 17.6%) (available from Asahi Kasei Corporation)

Polyol

· PLACCEL PCL-205U: polycaprolactone diol (molecular weight: 530) (available from Daicel Corporation)
· PLACCEL PCL-305: polycaprolactone triol (molecular weight: 550) (available from Daicel Corporation)

Photopolymerization Initiator

· HLI02: methyl 1-(9-ethyl-6-cyclohexanoyl-9H-carbazol-3-yl)-1-(O-acetyloxime)glutarate

Radical Scavenger

- TEMPOL: 4-hydroxy-2,2,6,6-tetramethylpiperidine-1-oxyl free radical (available from Tokyo Chemical Industry Co., Ltd.)

Urethane Polymerization Catalyst

- Octylic acid solution of tris(2-ethylhexanoate)bismuth (active ingredient amount: 56 mass%)

Example 1

Production of Compound M-1

Compound M-1 was produced by the following synthesis method.

[Chem. 22]

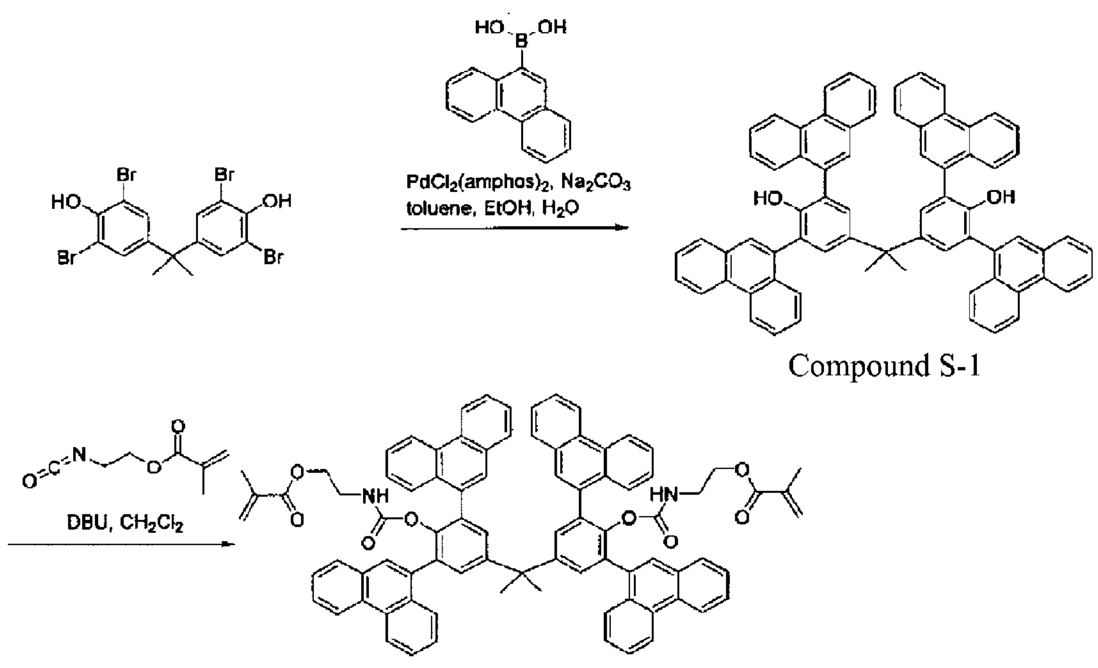


Compound S-1

Compound M-1

Tetrabromobisphenol A (10.0 g), phenanthrene-9-boronic acid (18.1 g), and sodium carbonate (9.9 g) were suspended in 250 mL of toluene, 250 mL of ethanol, and 125 mL of water, and degassed by passing nitrogen gas. To the reaction solution was added 1.4 mg of dichlorobis[di-t-butyl(p-dimethylaminophenyl)phosphino]palladium(II), and nitrogen gas was caused to further pass for 10 minutes. The reaction solution was heated under a nitrogen atmosphere and stirred under reflux for 4 hours. After the solution was cooled to room temperature, 100 mL of water was added, and the mixture was subjected to extraction with toluene. The organic layer was dried over sodium sulfate, and then the resultant organic layer was concentrated. A crude product of compound S-1 obtained through the concentration was produced in an amount of 19.9 g.

NMR measurement data of compound S-1 were as follows.

$^1$H-NMR (400 MHz, $CDCl_3$, $\delta$, ppm) 8.81-8.67 (Ar, 8H), 7.94-7.31 (Ar, 32H), 4.92-4.87 (OH, 2H), 1.91-1.76 ($CH_3$, 6H)

Under a nitrogen atmosphere, a crude product of compound S-1 (15.7 g), 2-isocyanatoethyl methacrylate (5.3 g), and 100 mL of dichloromethane were mixed. Diazabicycloundecene (2.0 mg) was added thereto, and the mixture was

stirred at room temperature. After completion of the reaction, 8 g of neutral silica gel was added to the reaction liquid, and the mixture was stirred at room temperature for 30 minutes. Thereafter, the silica gel and solid components were separated through filtration, and the solid was washed with dichloromethane/ethyl acetate (10:1). The resultant organic layer was concentrated, then dissolved in 30 mL of dichloromethane, and added dropwise to 300 mL of methanol cooled to 0°C. The resultant solid was filtered, washed with a small amount of cold methanol, and then dried with a vacuum dryer to produce 14.5 g of compound M-1.

NMR measurement data of compound M-1 were as follows.

$^1$H-NMR (400 MHz, $CDCl_3$, δ, ppm) 8.82-8.61 (Ar, 8H), 8.01-7.51 (Ar, 28H), 7.44-7.18 (Ar, 4H), 5.62-5.48 ($C=CH_2$, 2H), 5.32-5.19 ($C=CH_2$, 2H), 4.20-3.78 (NH, 2H), 3.33-2.73 ($CH_2$, 4H), 2.73-2.05 ($CH_2$, 4H), 1.98-1.79 ($CH_3$, 6H), 1.65-1.52 ($CH_3$, 6H)

Production of Hologram Recording Medium

In 2.51 g of DURANATE (registered trademark) TSS-100, 0.3821 g of compound M-1 as a polymerizable monomer, 0.0096 g of a photopolymerization initiator HLI02, and 3.29 mg of a radical scavenger TEMPOL were dissolved to prepare liquid A.

Separately, 1.74 g of PLACCEL PCL-205U and 0.75 g of PLACCEL PCL-305 were mixed (PLACCEL PCL-205U:PLACCEL PCL-305 = 70:30 (mass ratio)), and 0.2 mg of an octylic acid solution of tris(2-ethylhexanoate) bismuth was dissolved, to prepare liquid B.

Liquid A and liquid B were each degassed under reduced pressure at room temperature or 45°C for 2 hours, then 3.23 g of liquid A and 2.77 g of liquid B were mixed with stirring, and further degassed under vacuum for several minutes.

Subsequently, the vacuum-degassed mixture was poured onto a slide glass on which a spacer sheet having a thickness of 0.5 mm was placed on two opposing side edges, a slide glass was put thereon, the periphery was fixed with a clip, and the mixture was heated at 80°C for 24 hours, to prepare a hologram recording medium as a sample for evaluation. In this evaluation sample, a recording layer having a thickness of 0.5 mm was formed between the slide glasses as covers.

In this hologram recording medium, the ratio of the number of isocyanate groups in liquid A to the number of isocyanate-reactive groups in liquid B was 1.0, the polymerizable monomer was 56.9 μmol/g, the photopolymerization initiator was 3.54 μmol/g, and the radical scavenger was 3.54 μmol/g.

Hologram Recording and Evaluation

Using the hologram recording medium produced as the sample for evaluation, hologram recording and evaluation of hologram recording performance of the hologram recording medium were performed through the procedure described below.

Two-beam plane-wave Hologram recording was performed using a semiconductor laser having a wavelength of 405 nm and using an exposure device illustrated in FIG. 1 at an exposure power density of 10.2 $mW/cm^2$ per beam. The medium was rotated from -22.5° to 22.5°, and angle-multiplexed recording was performed at the same position. The diffraction efficiency at each multiple recording was measured. $\Delta n$ was calculated from the obtained diffraction efficiency, and the total sum in the entire multiple recording was taken as total $\Delta n$.

Details will be described below.

Hologram Recording

FIG. 1 is a configuration diagram illustrating an overview of a device used for hologram recording.

In FIG. 1, S denotes a sample of the hologram recording medium, and M1 to M3 each denote a mirror. PBS denotes a polarizing beam splitter. L1 denotes a laser light source for recording light that emits light having a wavelength of 405 nm (single mode laser ("L1" in FIG. 1) available from TOPTICA Photonics AG with which light having a wavelength of around 405 nm can be obtained). L2 denotes a laser light source for reproduction light that emits light having a wavelength of 633 nm. PD1, PD2, and PD3 denote photodetectors. Reference numeral 1 denotes an LED unit.

As illustrated in FIG. 1, light having a wavelength of 405 nm was divided by a polarizing beam splitter ("PBS" in the drawing), and two beams were caused to intersect on a recording surface so that an angle formed by the two beams was 59.3°. In this case, irradiation was performed so that the bisector of the angle formed by the two beams was perpendicular to the recording surface, and the vibration surface of the electric field vector of the two beams obtained by the division was perpendicular to the plane including the two intersecting beams.

After hologram recording, a light source that can obtain light having a wavelength of 633 nm with a He-Ne laser (V05-LHP 151 available from Melles Griot: "L2" in the drawing) was used. The hologram recording medium was irradiated with light at an angle of 50.7°, and the diffracted light was detected using a photodiode and a photosensor amplifier (S2281 and C9329 available from Hamamatsu Photonics K.K.: "PD1" in the drawing) to determine whether hologram recording

was correctly performed.

Measurement of Diffraction Efficiency

The angle (angle formed by the bisector of internal angle at point of intersection of two beams, i.e., incident light from mirrors M1 and M2 in FIG. 1 and the normal line from sample) at which the sample was moved with respect to the optical axis was changed from -22.5° to 22.5°, and 151 multiple recording was performed in 0.3° increments.
After the multiple recording, the remaining initiator and monomer were consumed by lighting the LED unit (1 in the drawing, central wavelength: 405 nm) for a certain period of time. This step is referred to as post-exposure. The power of the LED was set to 30 mW/cm$^2$, and irradiation was performed so that the integrated energy was 3.6 J/cm$^2$.
The diffraction efficiency of the hologram is given with the ratio of the intensity of the diffracted light to the sum of the transmitted light intensity and the diffracted light intensity. Irradiation with light (wavelength: 405 nm) from the mirror M1 in FIG. 1 was performed, and the diffraction efficiency from an angle of -23° to 23° was measured. From the obtained diffraction efficiency, $\Delta n$ was calculated by using the following equation according to Coupled Wave Theory (H. Kogelnik, The Bell System Technical Journal (1969), 48, 2909-2947), and the total sum in the entire multiple recording was set as total $\Delta n$.

[Math. 2]

$$\eta = \sin^2\left(\frac{\pi \cdot T \cdot \Delta n}{\lambda \cdot \cos\theta}\right)$$

$$total\Delta n = \sum \Delta n$$

In the equation, $\eta$ represents diffraction efficiency, T represents the thickness of a medium, $\lambda$ represents the wavelength of reference light, and $\theta$ represents the incident angle of reference light (29.65°).
Using a plurality of prepared samples, evaluation was performed a plurality of times while changing irradiation energy conditions such as increase or decrease in irradiation energy at the initial stage of recording and increase or decrease in total irradiation energy, and conditions under which the polymerizable monomer was almost consumed (total $\Delta n$ reached substantially equilibrium in multiple recording) were searched so that total $\Delta n$ had a maximum value. The obtained maximum value was defined as total $\Delta n$ of the medium.
The evaluation results are shown in Table 2 below.

Example 2

Production of Compound M-2

Compound M-2 was produced by the following synthesis method.

[Chem. 23]

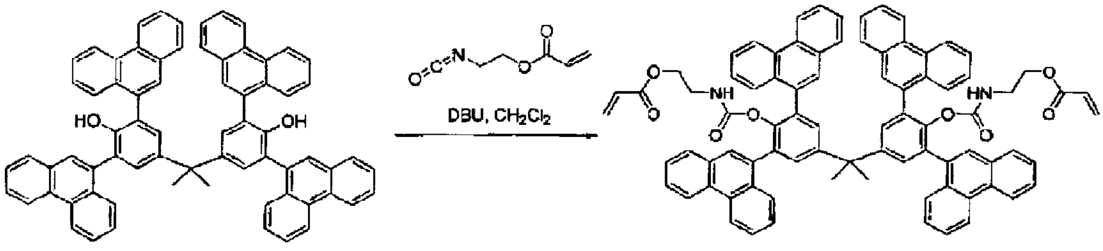


Compound S-1 Compound M-2

Under a nitrogen atmosphere, a crude product (8.0 g) of compound S-1 produced in the same manner as in Example 1, 2-isocyanatoethyl acrylate (2.5 g), and 60 mL of dichloromethane were mixed. Diazabicycloundecene (11.4 mg) was added thereto, and the mixture was stirred for 30 minutes.
After completion of the reaction, the reaction liquid was poured into 50 mL of an aqueous ammonium chloride solution, extracted with 50 mL of dichloromethane, then washed with 50 mL of saturated salt water, dried over anhydrous sodium sulfate, filtered, and concentrated. The resultant crude product was purified with a silica gel column (hexane/ethyl acetate), to thereby produce 7.2 g of compound M-2.

NMR measurement data of compound M-2 were as follows.
$^1$H-NMR (400 MHz, $CDCl_3$, $\delta$, ppm) 8.76-8.66 (Ar, 8H), 7.90-7.51 (Ar, 28H), 7.41-7.21 (Ar, 4H), 6.05-5.91 ($CH=CH_2$, 2H), 5.64-5.48 ($CH=CH_2$, 4H), 4.24-3.72 (NH, 2H), 3.28-2.74 ($CH_2$, 4H), 2.74-2.04 ($CH_2$, 4H), 1.94-1.79 ($CH_3$, 6H)

Example 3

Production of Compound M-3

Compound M-3 was produced by the following synthesis method.

[Chem. 24]

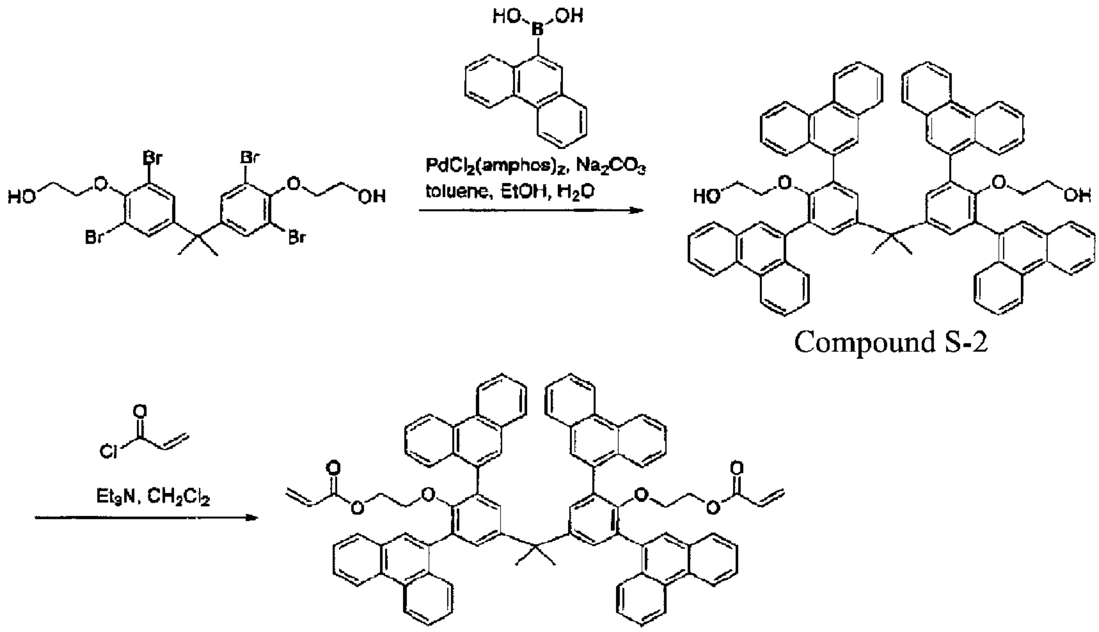


Compound M-3

Tetrabromobisphenol A bis(2-hydroxyethyl) ether (5.0 g), phenanthrene-9-boronic acid (7.4 g), and sodium carbonate (3.8 g) were suspended in 125 mL of toluene, 125 mL of ethanol, and 63 mL of water, and degassed by passing nitrogen gas. To the reaction solution was added 0.6 mg of dichlorobis[di-t-butyl(p-dimethylaminophenyl)phosphino] palladium(II), and nitrogen gas was caused to further pass for 10 minutes. The reaction solution was heated under a nitrogen atmosphere and stirred under reflux for 2 hours. After the solution was cooled to room temperature, 50 mL of water was added, and the mixture was subjected to extraction with 50 mL of toluene. The organic layer was dried over sodium sulfate, and then concentrated. The resultant crude product was purified with a silica gel column (hexane/ethyl acetate), to thereby produce 7.1 g of compound S-2.

NMR measurement data of compound S-2 were as follows.
$^1$H-NMR (400 MHz, $CDCl_3$, $\delta$, ppm) 8.81-8.71 (Ar, 8H), 7.94-7.31 (Ar, 32H), 3.21-3.11 ($CH_2$, 4H), 2.79-2.62 ($CH_2$, 4H) 1.90-1.80 ($CH_3$, 6H), 0.54-0.38 (OH, 2H)

Under a nitrogen atmosphere, compound S-2 (5.0 g) and triethylamine (1.5 g) were added to 30 mL of dichloromethane, and acryloyl chloride (1.2 g) was slowly added dropwise thereto in an ice bath. After completion of the reaction, 20 mL of 5 mass% aqueous sodium hydrogen carbonate solution was added to the reaction liquid, and the mixture was subjected to extraction with 50 mL of dichloromethane. Thereafter, the organic layer was dried over sodium sulfate and then concentrated. The resultant crude product was purified with a silica gel column (hexane/ethyl acetate), to thereby produce 3.5 g of compound M-3.

NMR measurement data of compound M-3 were as follows.
$^1$H-NMR (400 MHz, $CDCl_3$, $\delta$, ppm) 8.78-8.69 (Ar, 8H), 7.92-7.28 (Ar, 32H), 5.51-5.43 ($CH=CH_2$, 2H), 5.13-5.07 ($CH=CH_2$, 2H), 4.95-4.83 ($CH=CH_2$, 2H), 3.42-3.24 ($CH_2$, 8H), 1.87-1.76 ($CH_3$, 6H)

Example 4

Production of Compound M-4

Compound M-4 was produced by the following synthesis method.

[Chem. 25]

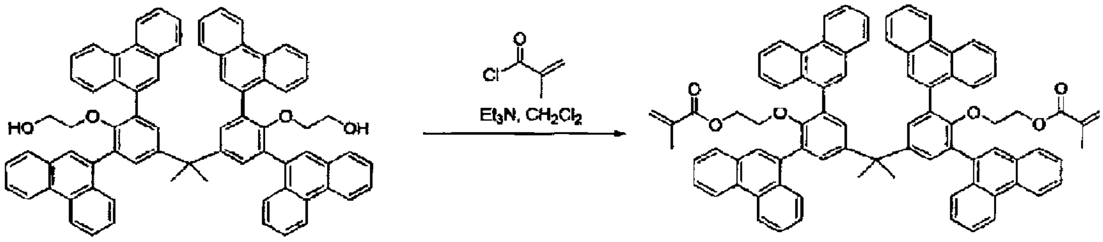


Compound S-2 Compound M-4

Under a nitrogen atmosphere, compound S-2 (8.1 g) produced in the same manner as in Example 3 and triethylamine (2.4 g) were added to 80 mL of dichloromethane, and acryloyl chloride (2.1 g) was slowly added dropwise thereto in an ice bath. After completion of the reaction, 20 mL of 5 mass% aqueous sodium hydrogen carbonate solution was added to the reaction liquid, and the mixture was subjected to extraction with 50 mL of dichloromethane. Thereafter, the organic layer was dried over sodium sulfate and then concentrated. The resultant crude product was purified with a silica gel column (hexane/ethyl acetate), to thereby produce 4.8 g of compound M-4.

NMR measurement data of compound M-4 were as follows.

$^1$H-NMR (400 MHz, $CDCl_3$, $\delta$, ppm) 8.84-8.62 (Ar, 8H), 7.92-7.74 (Ar, 12H), 7.71-7.56 (Ar, 12H), 7.55-7.31 (Ar, 8H), 5.01-4.91 (C=$CH_2$, 2H), 4.82-4.76 (C=$CH_2$, 2H), 3.45-3.15 ($CH_2$, 8H), 1.91-1.73 ($CH_3$, 6H), 1.21-1.14 ($CH_3$, 6H)

Example 5

Production of Compound M-5

Compound M-5 was produced by the following synthesis method.

[Chem. 26]

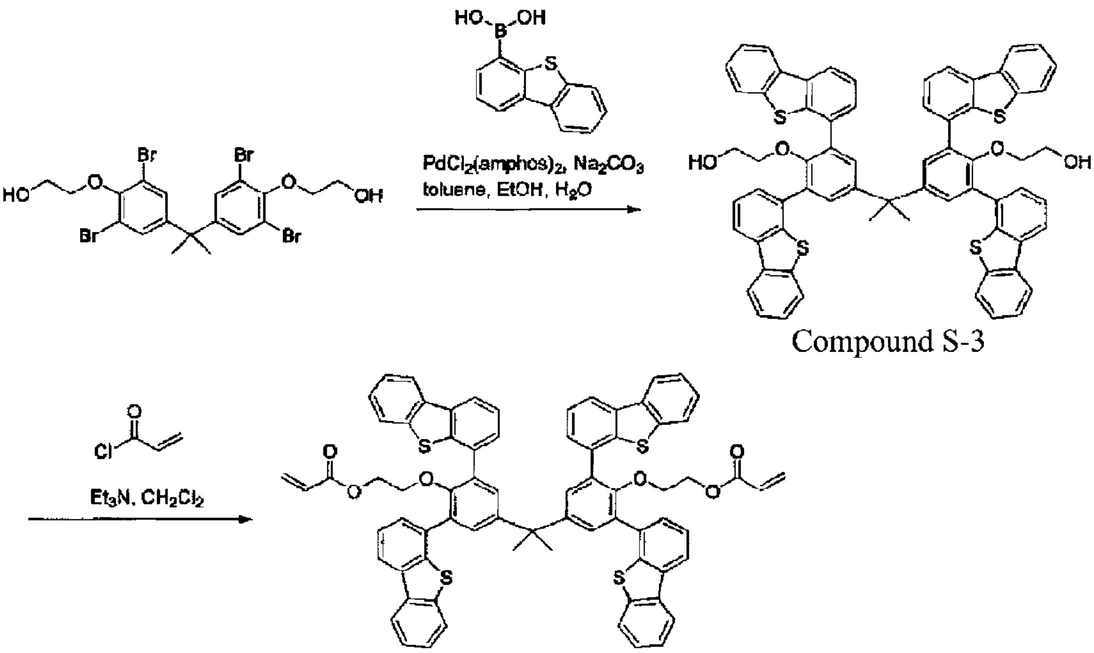


Compound S-3

Compound M-5

Tetrabromobisphenol A bis(2-hydroxyethyl) ether (5.0 g), dibenzothiophene-4-boronic acid (7.6 g), and sodium

carbonate (3.8 g) were suspended in 125 mL of toluene, 125 mL of ethanol, and 63 mL of water, and degassed by passing nitrogen gas. To the reaction solution was added 0.6 mg of dichlorobis[di-t-butyl(p-dimethylaminophenyl)phosphino]palladium(II), and nitrogen gas was caused to further pass for 10 minutes. The reaction solution was heated under a nitrogen atmosphere and stirred under reflux for 2 hours. After the solution was cooled to room temperature, 50 mL of water was added, and the mixture was subjected to extraction with 100 mL of toluene. The organic layer was dried over sodium sulfate and then concentrated, to thereby produce 9.9 g of a crude product of compound S-3.

NMR measurement data of compound S-3 were as follows.

$^1$H-NMR (400 MHz, $CDCl_3$, $\delta$, ppm) 8.20-8.14 (Ar, 8H), 7.73 (s, Ar, 4H), 7.72-7.31 (Ar, 20H), 3.22-3.17 ($CH_2$, 4H), 3.01-2.92 ($CH_2$, 4H), 0.84 (t, OH, 2H)

Under a nitrogen atmosphere, compound S-3 (8.0 g) and triethylamine (2.1 g) were added to 80 mL of dichloromethane, and acryloyl chloride (1.6 g) was slowly added dropwise thereto in an ice bath.

After completion of the reaction, 30 mL of 5 mass% aqueous sodium hydrogen carbonate solution was added to the reaction liquid, and the mixture was subjected to extraction with 80 mL of dichloromethane. Thereafter, the organic layer was dried over sodium sulfate and then concentrated. The resultant crude product was purified with a silica gel column (hexane/ethyl acetate), to thereby produce 4.1 g of compound M-5.

NMR measurement data of compound M-5 were as follows.

$^1$H NMR (400 MHz, $CDCl_3$, $\delta$, ppm) 8.15 (td, Ar, 8H), 7.72 (s, Ar, 4H), 7.67 (dd, Ar, 4H), 7.60 (d, Ar, 4H), 7.53 (t, Ar, 4H), 7.49-7.33 (Ar, 8H), 5.82 (dd, $CH=CH_2$, 2H), 5.41 (dd, $CH=CH_2$, 2H), 5.32 (dd, $CH=CH_2$, 2H), 3.63-3.51 ($CH_2$, 4H), 3.38-3.22 ($CH_2$, 4H), 1.89 (s, $CH_3$, 6H)

## Example 6

Production of Compound M-6

Compound M-6 was produced by the following synthesis method.

[Chem. 27]

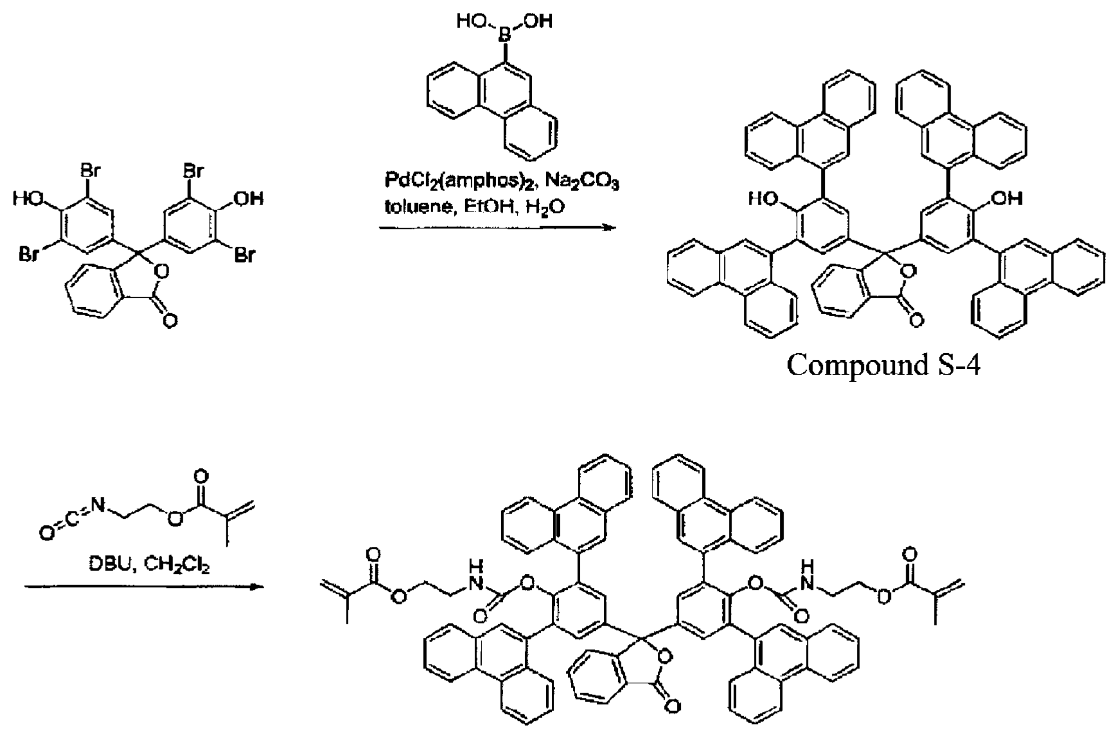


Compound S-4

Compound M-6

3',3",5',5"-Tetrabromophenolphthalein (10.0 g), diphenanthrene-9-boronic acid (15.8 g), and sodium carbonate (3.7 g) were suspended in 100 mL of toluene, 100 mL of ethanol, and 50 mL of water, and degassed by passing nitrogen gas. To the reaction solution was added 0.6 mg of dichlorobis[di-t-butyl(p-dimethylaminophenyl)phosphino]palladium(II),

and nitrogen gas was caused to further pass for 10 minutes. The reaction solution was heated under a nitrogen atmosphere and stirred under reflux for 2 hours. After the solution was cooled to room temperature, 100 mL of water was added and filtered. The resultant solid was washed with a small amount of toluene and ethanol and dried, to thereby produce 12.6 g of a crude product of compound S-4.

NMR measurement data of compound S-4 were as follows.
$^1$H-NMR (400 MHz, $CDCl_3$, $\delta$, ppm) 8.85-8.68 (Ar, 8H), 7.99-7.12 (Ar, 36H), 5.07-5.01 (OH, 2H)

Under a nitrogen atmosphere, a crude product of compound S-4 (12.6 g), 2-isocyanatoethyl methacrylate (3.8 g), and 100 mL of dichloromethane were mixed. Diazabicycloundecene (1.9 mg) was added thereto, and the mixture was stirred at room temperature. After completion of the reaction, 7 g of neutral silica gel was added to the reaction liquid, and the mixture was stirred at room temperature for 30 minutes. Thereafter, the silica gel and solid components were separated through filtration, and the solid was washed with dichloromethane/ethyl acetate (10:1). The resultant organic layer was concentrated, then dissolved in 100 mL of dichloromethane, and added dropwise to 300 mL of methanol cooled to 0°C. The resultant solid was filtered, washed with a small amount of cold methanol, and then dried with a vacuum dryer to produce 13.9 g of compound M-6.

NMR measurement data of compound M-6 were as follows.
$^1$H NMR (400 MHz, $CDCl_3$, $\delta$, ppm) 8.83-8.61 (Ar, 8H), 8.02-7.18 (Ar, 36H), 5.62-5.48 (C=$CH_2$, 2H), 5.32-5.21 (C=$CH_2$, 2H), 4.21-3.73 (NH, 2H), 3.31-1.90 ($CH_2$, 8H), 1.64-1.53 ($CH_3$, 6H)

Example 7

Production of Compound M-7

Compound M-7 was produced by the following synthesis method.

[Chem. 28]

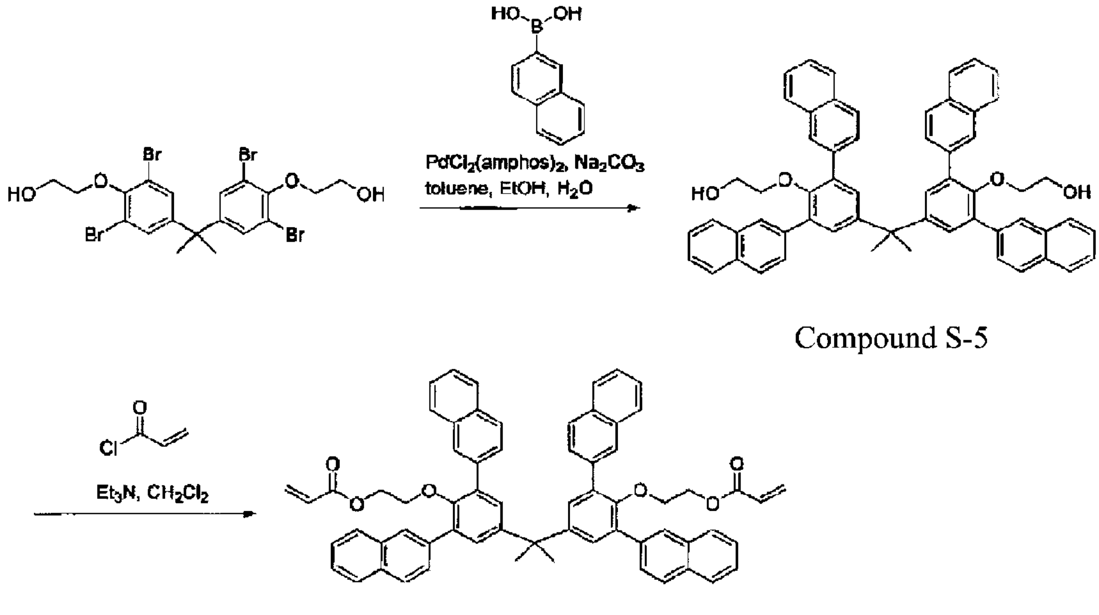


Compound S-5

Compound M-7

Tetrabromobisphenol A bis(2-hydroxyethyl) ether (5.0 g), 2-naphthaleneboronic acid (5.7 g), and sodium carbonate (3.8 g) were suspended in 40 mL of toluene, 40 mL of ethanol, and 20 mL of water, and degassed by passing nitrogen gas. To the reaction solution was added 0.6 mg of dichlorobis[di-t-butyl(p-dimethylaminophenyl)phosphino] palladium(II), and nitrogen gas was caused to further pass for 10 minutes. The reaction solution was heated under a nitrogen atmosphere and stirred under reflux for 2 hours. After the solution was cooled to room temperature, 50 mL of water was added, and the mixture was subjected to extraction with 50 mL of toluene. The organic layer was dried over sodium sulfate, and then concentrated. The resultant crude product was diluted with 25 mL of toluene and added dropwise to 100 mL of hexane, and the resultant solid was dried, to thereby produce 6.3 g of compound S-5.

NMR measurement data of compound S-5 were as follows.
$^1$H-NMR (400 MHz, $CDCl_3$, $\delta$, ppm) 8.03 (s, Ar, 4H), 7.97-7.78 (Ar, 16H), 7.56-7.42 (Ar, 12H), 3.40-3.28 ($CH_2$, 4H)

3.22-3.11 ($CH_2$, 4H), 1.90 (s, $CH_3$, 6H), 1.06 (t, OH, 2H)

Under a nitrogen atmosphere, compound S-5 (6.1 g) and triethylamine (2.3 g) were added to 40 mL of dichloromethane, and acryloyl chloride (1.6 g) was slowly added dropwise thereto in an ice bath. After completion of the reaction, 40 mL of 5 mass% aqueous sodium hydrogen carbonate solution was added to the reaction liquid, and the mixture was stirred for 1 hour and then subjected to extraction with 50 mL of dichloromethane. Subsequently, the organic layer was dried over sodium sulfate, and then concentrated. The resultant crude product was purified with a silica gel column (hexane, ethyl acetate), to thereby produce 3.0 g of compound M-7.

NMR measurement data of compound M-7 were as follows.

$^1$H-NMR (400 MHz, $CD_3OD$, $\delta$, ppm) 8.04 (s, Ar, 4H), 7.92-7.77 (Ar, 16H), 7.56-7.39 (Ar, 12H), 5.90-5.79 (CH=$CH_2$, 2H), 5.44-5.32 (CH=$CH_2$, 4H), 3.79-3.70 ($CH_2$, 4H), 3.49-3.38 ($CH_2$, 4H), 1.89 (s, $CH_3$, 6H)

Example 8

Production of Compound M-8

Compound M-8 was produced by the following synthesis method.

[Chem. 29]

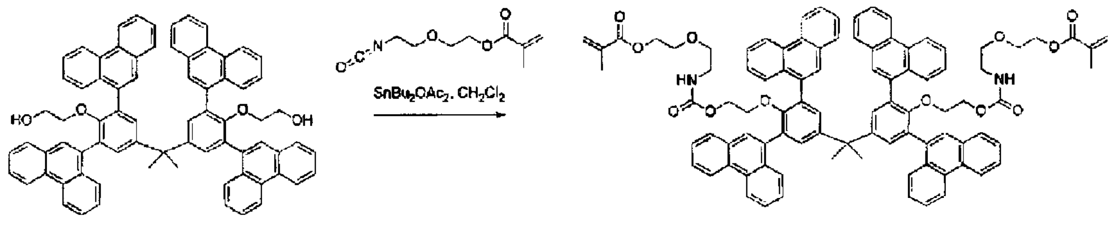


S-2 M-8

Under a nitrogen atmosphere, compound S-2 (8.0 g), 2-(2-isocyanatoethoxy)ethyl methacrylate (3.75 g), and 50 mL of dichloromethane were mixed. Dibutyltin diacetate (82.5 mg) was added thereto, and the mixture was stirred for 6 days. After completion of the reaction, 11 g of diamine silica (available from FUJI SILYSIA CHEMICAL LTD.) was added and stirred for 30 minutes, and then 4 g of neutral silica gel was added and stirred in the same manner for 30 minutes. Thereafter, the silica gel and the solid component were separated through filtration, and the resultant organic layer was concentrated. Then, 40 mL of dichloromethane was added, and the mixture was added dropwise to 240 mL of methanol cooled to -10°C. The resultant solid was filtered, washed with a small amount of cold methanol, and then dried with a vacuum dryer to produce 7.84 g of compound M-8.

NMR measurement data of compound M-8 were as follows.

$^1$H-NMR (400 MHz, $CD_3OD$, $\delta$, ppm) 8.84-8.68 (Ar, 8H), 8.02-7.30 (Ar, 32H), 6.09 (C=$CH_2$, 2H), 5.53 (C=$CH_2$, 2H), 4.36-3.94 ($CH_2$, 4H), 3.65-3.02 (NH, $CH_2$, 18H), 2.83-2.61 ($CH_2$, 4H), 2.10-1.72 (s, $CH_3$, 12H)

Example 9

Production of Compound M-9

Compound M-9 was produced by the following synthesis method.

[Chem. 30]

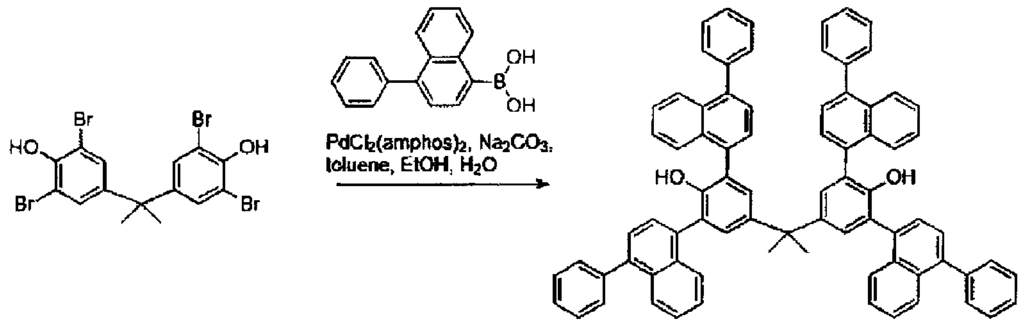


Compound S-6

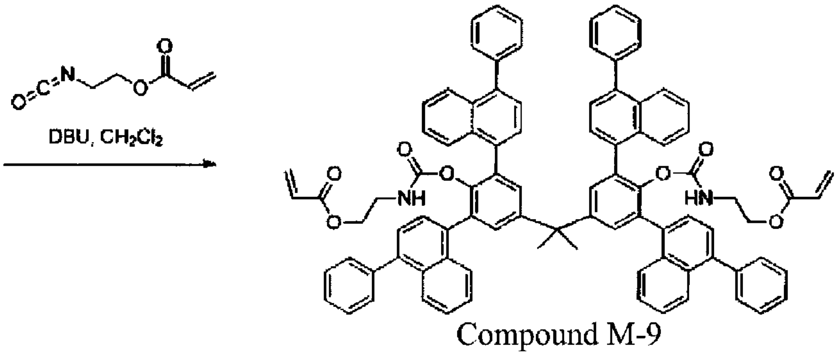


Compound M-9

Tetrabromobisphenol A (5.1 g), 4-phenylnaphthalene-1-boronic acid (9.7 g), and sodium carbonate (2.1 g) were suspended in 25 mL of toluene, 25 mL of ethanol, and 12 mL of water, and degassed by passing nitrogen gas. To the reaction solution was added 0.7 mg of dichlorobis[di-t-butyl(p-dimethylaminophenyl)phosphino]palladium(II) was added, and nitrogen gas was caused to further pass for 10 minutes. The reaction solution was heated under a nitrogen atmosphere and stirred under reflux for 3 hours. After the solution was cooled to room temperature, 50 mL of water was added, and the mixture was subjected to extraction with toluene. The organic layer was dried over sodium sulfate, and then the resultant organic layer was concentrated. The resultant crude product was purified with a silica gel column (hexane, ethyl acetate), to thereby produce 8.7 g of compound S-6.

NMR measurement data of compound S-6 were as follows.

$^1$H-NMR (400 MHz, $CDCl_3$, $\delta$, ppm) 8.07-7.77 (Ar, 8H), 7.73-7.29 (Ar, 40H), 4.97-4.86 (OH, 2H), 1.95-1.73 ($CH_3$, 6H)

In a nitrogen atmosphere, compound S-6 (3.6 g), 2-isocyanatoethyl acrylate (1.1 g), and 45 mL of dichloromethane were mixed. Diazabicycloundecene (3.0 mg) was added thereto, and the mixture was stirred at room temperature. After completion of the reaction, 8 g of neutral silica gel was added to the reaction liquid, and the mixture was stirred at room temperature for 30 minutes. Thereafter, the silica gel and solid components were separated through filtration, and the solid was washed with dichloromethane/ethyl acetate (10:1). The resultant organic layer was concentrated, then dissolved in 20 mL of dichloromethane, and added dropwise to 100 mL of methanol cooled to 0°C. The resultant solid was filtered, washed with a small amount of cold methanol, and then dried with a vacuum dryer to produce 4.2 g of compound M-9.

NMR measurement data of compound M-9 were as follows.

$^1$H-NMR (400 MHz, $CDCl_3$, $\delta$, ppm) 8.01-7.69 (Ar, 8H), 7.68-7.32 (Ar, 40H), 6.22-6.09 ($CH=CH_2$, 2H), 5.88-5.74 ($CH=CH_2$, 2H), 5.71-5.60 ($CH=CH_2$, 2H), 4.40-4.02 (NH, 2H), 3.68-3.34 ($CH_2$, 4H), 3.08-2.14 ($CH_2$, 4H), 1.98-1.71 ($CH_3$, 6H)

A hologram recording medium was produced in the same manner as in Example 1 except for using compound M-9 (0.4054 g) as the polymerizable monomer, and the hologram recording medium was evaluated. The results are shown in Table 2 below.

Example 10

Production of Compound M-10

Compound M-10 was produced by the following synthesis method.

[Chem. 31]

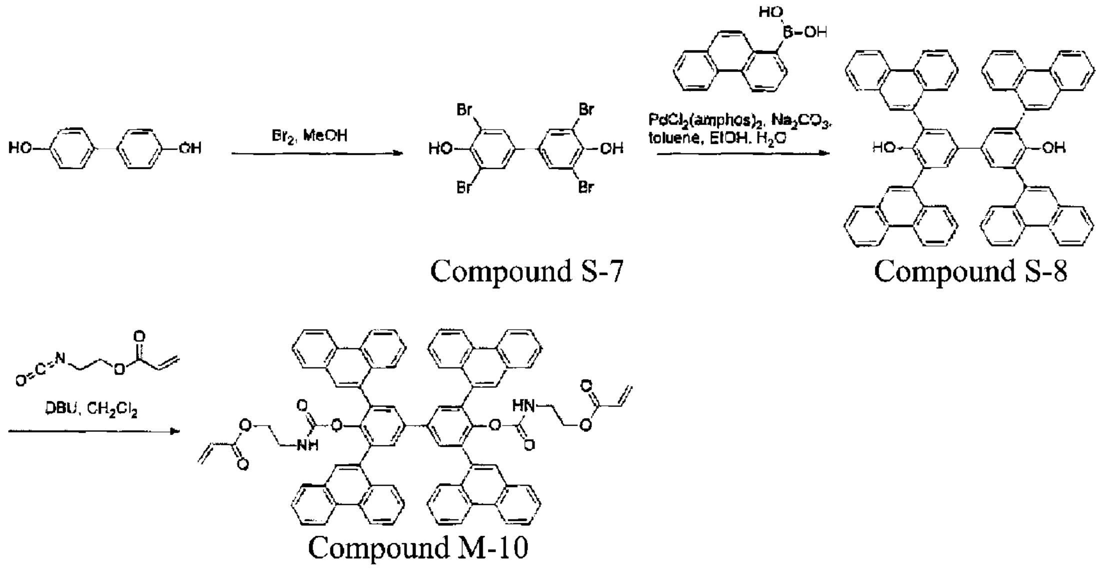


Compound S-7

Compound S-8

Compound M-10

4,4'-dihydroxybiphenyl (14 g) was dissolved in 400 mL of methanol, and cooled to 0°C. Bromine (50 g) was slowly added dropwise, and the mixture was stirred at 0°C for 1 hour after the dropwise addition. The produced pale yellow solid was collected through filtration, and then the solid was washed with cold methanol, to thereby produce 34.6 g of tetrabromo product S-7.

NMR measurement data of compound S-7 were as follows.

$^1$H-NMR (400 MHz, $CDCl_3$, $\delta$, ppm) 7.56 (s, Ar, 4H), 5.90 (s, OH, 2H)

Tetrabromo product S-7 (12.6 g), phenanthrene-9-boronic acid (22.3 g), and sodium carbonate (10.6 g) were suspended in 100 mL of toluene, 100 mL of ethanol, and 50 mL of water, and degassed by passing nitrogen gas. To the reaction solution was added 2.3 mg of dichlorobis[di-t-butyl(p-dimethylaminophenyl)phosphino]palladium(II), and nitrogen gas was caused to further pass for 10 minutes. The reaction solution was heated under a nitrogen atmosphere and stirred under reflux for 3 hours. After the solution was cooled to room temperature, 150 mL of water was added, and the resultant solid was collected through filtration. The solid was washed with ethanol and then dried with a vacuum dryer, to thereby produce 16.0 g of compound S-8.

NMR measurement data of compound S-8 were as follows.

$^1$H-NMR (400 MHz, $CDCl_3$, $\delta$, ppm) 8.78-8.67 (Ar, 8H), 8.01-7.80 (Ar, 16H), 7.71-7.53 (Ar, 16H), 4.99-4.95 (OH, 2H)

In a nitrogen atmosphere, compound S-8 (7.0 g), 2-isocyanatoethyl acrylate (52.4 g), and 70 mL of dichloromethane were mixed. Diazabicycloundecene (1.3 mg) was added thereto, and the mixture was stirred at room temperature. After completion of the reaction, 4 g of neutral silica gel was added to the reaction liquid, and the mixture was stirred at room temperature for 30 minutes. Thereafter, the silica gel and solid components were separated through filtration, and the solid was washed with dichloromethane. The resultant organic layer was concentrated, and then subjected to silica gel column purification with dichloromethane/ethyl acetate. The resultant target product was dissolved in 35 mL of dichloromethane and added dropwise to 300 mL of ice-cooled methanol. The precipitated solid was separated through filtration, washed with a small amount of cold methanol, and then dried under reduced pressure, to thereby produce 7.4 g of compound M-10.

NMR measurement data of compound M-10 were as follows.

$^1$H-NMR (400 MHz, $CDCl_3$, $\delta$, ppm) 8.78-8.59 (Ar, 8H), 8.10-7.81 (Ar, 16H), 7.70-7.49 (Ar, 16H), 6.04-5.91 ($CH=CH_2$, 2H), 5.65-5.43 ($CH=CH_2$, 4H), 4.23-3.85 (NH, 2H), 3.23-2.75 ($CH_2$, 4H), 2.74-2.01 ($CH_2$, 4H)

A hologram recording medium was produced in the same manner as in Example 1 except for using compound M-10 (0.3625 g) as the polymerizable monomer, and the hologram recording medium was evaluated. The results are shown in Table 2 below.

Example 11

Production of Compound M-11

Compound M-11 was produced by the following synthesis method.

[Chem. 32]

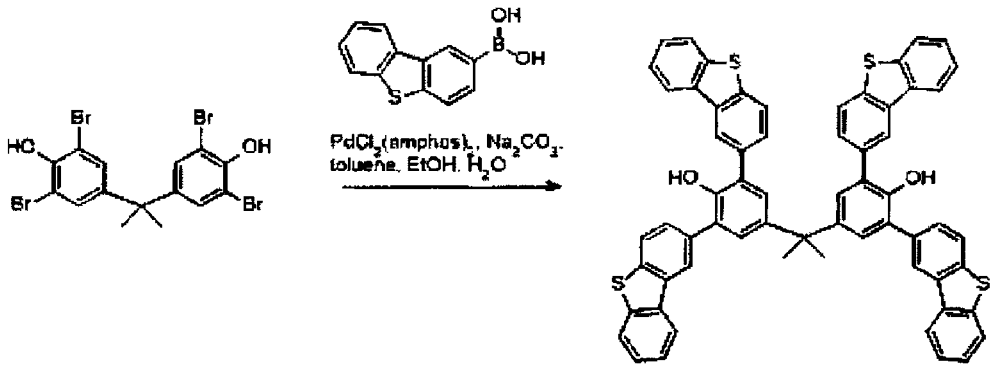


Compound R-1

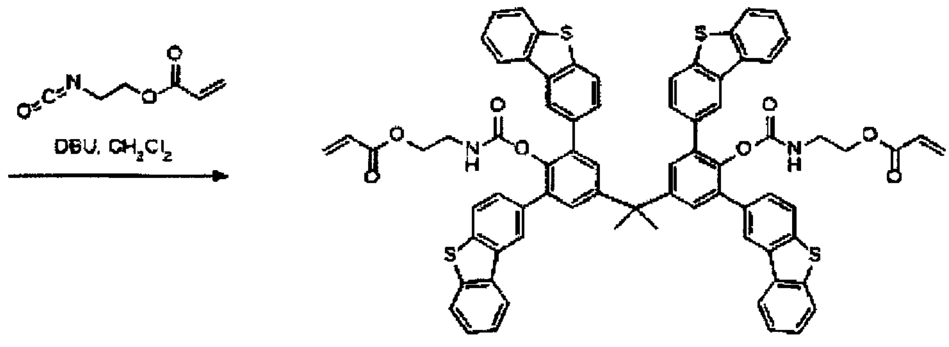


Compound M-11

Tetrabromobisphenol A (2.5 g), dibenzothiophene-2-boronic acid (4.4 g), and sodium carbonate (1.07 g) were suspended in 13 mL of toluene, 13 mL of ethanol, and 7 mL of water, and degassed by passing nitrogen gas. To the reaction solution was added 1.0 mg of dichlorobis[di-t-butyl(p-dimethylaminophenyl)phosphino]palladium(II), and nitrogen gas was caused to further pass for 10 minutes. The reaction solution was heated under a nitrogen atmosphere and stirred under reflux for 2 hours. After the solution was cooled to room temperature, 30 mL was added and filtered. The resultant solid was washed with a small amount of toluene and ethanol and dried, to thereby produce 1.8 g of compound R-1.

NMR measurement data of compound R-1 were as follows.

$^1$H-NMR (400 MHz, $CDCl_3$, $\delta$, ppm) 8.35 (d, Ar, 4H), 8.15 (d, Ar, 4H), 7.96 (d, Ar, 4H), 7.87 (d, Ar, 4H), 7.68 (dd, Ar, 4H), 7.53-7.37 (dd, Ar, 12H), 5.43 (s, OH, 2H), 1.88 (s, $CH_3$, 6H)

Under a nitrogen atmosphere, compound R-1 (1.7 g), 2-isocyanatoethyl acrylate (055 g), and 30 mL of dichloromethane were mixed. Diazabicycloundecene (3.0 mg) was added thereto, and the mixture was stirred at room temperature. After completion of the reaction, 2 g of neutral silica gel was added to the reaction liquid, and the mixture was stirred at room temperature for 30 minutes. Thereafter, the silica gel and solid components were separated through filtration, and the solid was washed with dichloromethane. The resultant organic layer was concentrated, then dissolved in 10 mL of dichloromethane, and added dropwise to 50 mL of methanol cooled to 0°C. The resultant solid was filtered, washed with a small amount of cold methanol, and then dried with a vacuum dryer to produce 1.7 g of compound M-11.

NMR measurement data of compound M-11 were as follows.

$^1$H-NMR (400 MHz, $CDCl_3$, $\delta$, ppm) 8.32-8.19 (Ar, 4H), 8.17-8.03 (Ar, 4H), 7.94-7.81 (Ar, 8H), 7.63-7.36 (Ar, 16H), 6.15-6.05 ($CH=CH_2$, 2H), 5.72-5.51 ($CH=CH_2$, 4H), 4.98-4.36 (NH, 2H), 3.82-3.61 ($CH_2$, 4H), 3.27-2.99 ($CH_2$, 4H), 2.12-1.74 ($CH_3$, 6H)

A hologram recording medium was produced in the same manner as in Example 1 except for using compound M-11 (0.3804 g) as the polymerizable monomer, and the hologram recording medium was evaluated. The results are shown in Table 2 below.

Comparative Example 1

Compound R-1 was used as the compound of Comparative Example 1.

Comparative Example 2

Production of Compound R-2

Compound R-2 was produced by the following synthesis method.

[Chem. 33]

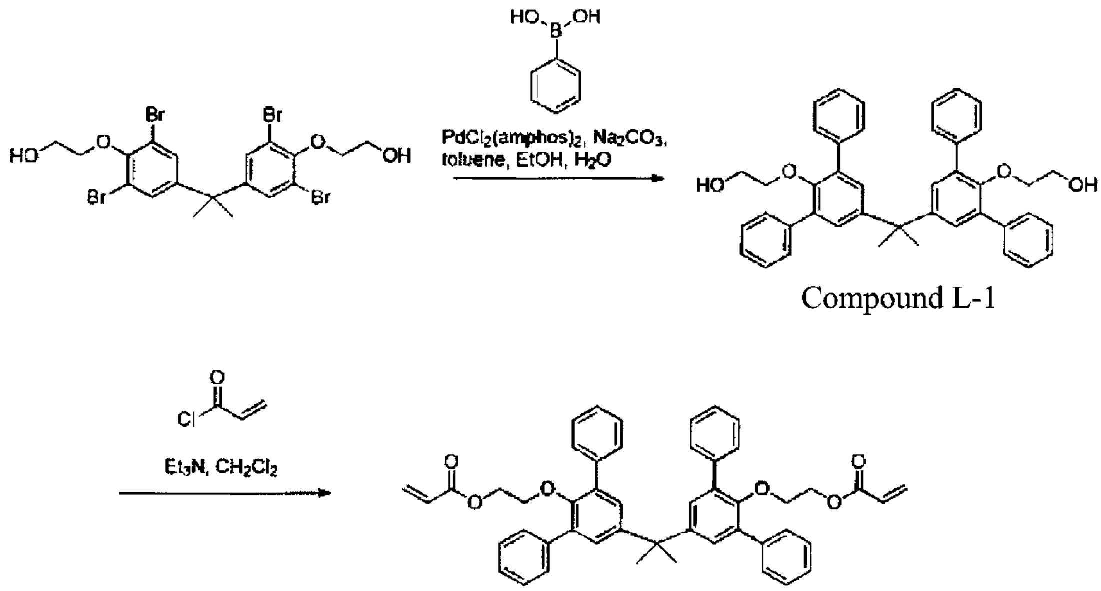


Compound L-1

Compound R-2

Tetrabromobisphenol A bis(2-hydroxyethyl) ether (5.0 g), phenylboronic acid (4.1 g), and sodium carbonate (3.8 g) were suspended in 40 mL of toluene, 40 mL of ethanol, and 20 mL of water, and degassed by passing nitrogen gas. To the reaction solution was added 0.6 mg of dichlorobis[di-t-butyl(p-dimethylaminophenyl)phosphino]palladium(II), and nitrogen gas was caused to further pass for 10 minutes. The reaction solution was heated under a nitrogen atmosphere and stirred under reflux for 2 hours. After the solution was cooled to room temperature, 50 mL of water was added, and the mixture was subjected to extraction with 50 mL of toluene. The organic layer was dried over sodium sulfate, and then concentrated. The resultant crude product was diluted with 20 mL of toluene and added dropwise to 80 mL of hexane, and the resultant solid was dried, to thereby produce 3.6 g of compound L-1.

NMR measurement data of compound L-1 were as follows.

$^1$H-NMR (400 MHz, DMSO-$d_6$, $\delta$, ppm) 7.58-7.52 (Ar, 8H), 7.46-7.39 (Ar, 8H), 7.38-7.31 (Ar, 4H), 7.26 (s, Ar, 4H), 4.25 (t, OH, 2H), 3.15 (t, $CH_2$, 4H) 3.03 (q, $CH_2$, 4H), 1.79 (s, $CH_3$, 6H)

Under a nitrogen atmosphere, compound L-1 (3.5 g) and triethylamine (1.8 g) were added to 25 mL of dichloromethane, and acryloyl chloride (1.2 g) was slowly added dropwise thereto in an ice bath. After completion of the reaction, 40 mL of 5 mass% aqueous sodium hydrogen carbonate solution was added to the reaction liquid, and the mixture was stirred for 1 hour and then subjected to extraction with 50 mL of dichloromethane. Subsequently, the organic layer was dried over sodium sulfate, and then concentrated. The resultant crude product was purified with a silica gel column (hexane, ethyl acetate), to thereby produce 1.8 g of compound R-2.

NMR measurement data of compound R-2 were as follows.

$^1$H-NMR (400 MHz, $CD_3OD$, $\delta$, ppm) 7.57-7.52 (Ar, 8H), 7.43-7.36 (Ar, 8H), 7.35-7.28 (Ar, 4H), 7.26 (s, Ar, 4H), 6.15 (dd, $CH=CH_2$, 2H), 5.88 (dd, $CH=CH_2$, 2H), 5.76 (dd, $CH=CH_2$, 2H), 3.81-3.76 ($CH_2$, 4H), 3.44-3.40 ($CH_2$, 4H), 1.80 (s, $CH_3$, 6H)

A hologram recording medium was produced in the same manner as in Example 1 except for using compound R-2 (0.2252 g) as the polymerizable monomer, and the hologram recording medium was evaluated. The results are shown in Table 2.

Comparative Example 3

Production of Compound R-3

Compound R-3 was produced by the following synthesis method.

[Chem. 34]

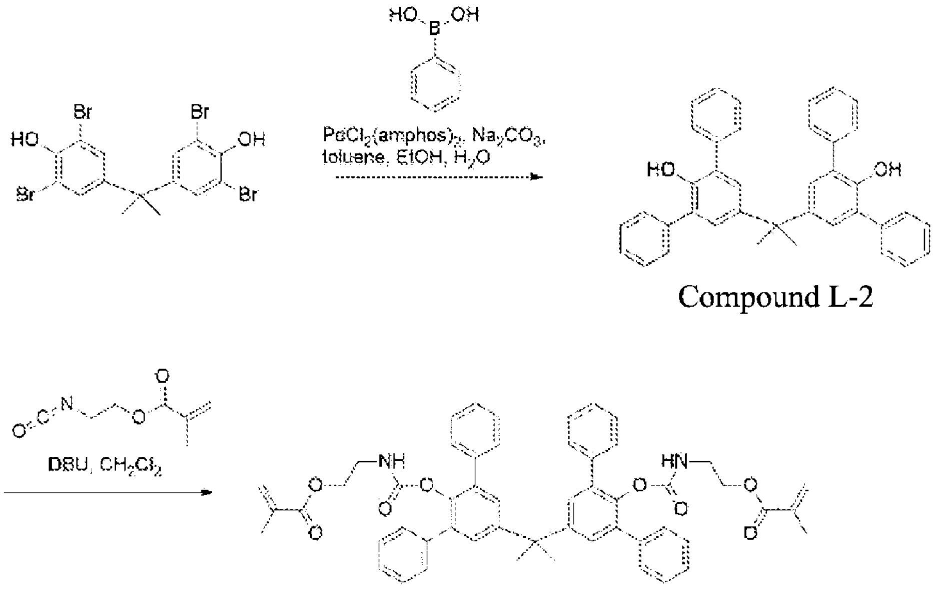


Compound L-2

Compound R-3

Tetrabromobisphenol A (15.1 g), phenylboronic acid (14.2 g), and sodium carbonate (14.6 g) were suspended in 120 mL of toluene, 120 mL of ethanol, and 60 mL of water, and degassed by passing nitrogen gas. To the reaction solution was added 2.0 mg of dichlorobis[di-t-butyl(p-dimethylaminophenyl)phosphino]palladium(II), and nitrogen gas was caused to further pass for 10 minutes. The reaction solution was heated under a nitrogen atmosphere and stirred under reflux for 2 hours. After the solution was cooled to room temperature, 100 mL of water was added, and the mixture was subjected to extraction with 100 mL of toluene. The organic layer was dried over sodium sulfate, and then concentrated. The resultant crude product was washed with hexane and then washed with a small amount of toluene, and the resultant solid was dried, to thereby produce 9.69 g of compound L-2.

NMR measurement data of compound L-2 were as follows.

$^1$H-NMR (400 MHz, $CDCl_3$, $\delta$, ppm) 7.60-7.31 (Ar, 20H), 7.20 (Ar, 4H), 5.28 (s, OH, 2H), 1.74 (s, $CH_3$, 6H)

Under a nitrogen atmosphere, compound L-2 (5.0 g), 2-isocyanatoethyl methacrylate (3.5 g), and 30 mL of dichloromethane were mixed. Diazabicycloundecene (14.3 mg) was added thereto, and the mixture was stirred for 1 hour. After the raw materials disappeared, 4 g of neutral silica gel was added to the reaction liquid, and the mixture was stirred at room temperature for 30 minutes. Thereafter, the silica gel and solid components were separated through filtration, and the solid was washed with dichloromethane. The resultant organic layer was concentrated, then dissolved in 20 mL of dichloromethane, and added dropwise to 125 mL of methanol cooled to 0°C. The resultant solid was filtered, washed with a small amount of cold methanol, and then dried with a vacuum dryer to produce 4.0 g of compound R-3.

NMR measurement data of compound R-3 were as follows.

$^1$H-NMR (400 MHz, $CD_3OD$, $\delta$, ppm) 7.51-7.26 (Ar, 24H), 6.06 (brs, C=$CH_2$, 2H), 5.58 (brs, C=$CH_2$, 2H), 4.99-4.43 (NH, 2H), 3.94-3.72 ($CH_2$, 4H), 3.32-3.12 ($CH_2$, 4H), 1.92 (s, $CH_3$, 6H), 1.78 (s, $CH_3$, 6H)

Compound R-3 as a polymerizable monomer had low solubility in liquid A, and a hologram recording medium failed to be produced and failed to be evaluated.

Comparative Example 4

In Comparative Example 4, NK ESTER A-BPEF: 9,9-bis[4-(2-hydroxyethoxy)phenyl]fluorene available from SHIN NAKAMURA CHEMICAL Co., Ltd. was used as Compound R-4 for evaluation.

Comparative Example 5

In Comparative Example 5, DNFE-A: 2,12-bis(hydroxyethoxyacryloyl)dinaphthofuran described in JP 2023-26379 A was synthesized by the method of Example 2 in JP 2023-26379 A and used as compound R-5 for evaluation.

Measurement of Refractive Index

The refractive indexes of the polymerizable monomers produced in Examples and Comparative Examples were measured by the following method. The results are shown in Table 1.
A test solution was prepared by dissolving a sample in a mixed solution of 3-phenoxybenzyl acrylate and trimethylolpropane trimethacrylate at a mass ratio of 4:1 so as to have a predetermined concentration.
Two types of test solutions having sample concentrations of 10 mass% and 20 mass% were prepared.
The refractive index of each test solution was measured with a Kalnew precision refractometer (available from Shimadzu Corporation, product name: KPR-2000). The temperature of the test solution was set to 23°C, and the measurement wavelength was a helium lamp d-line (587.6 nm). Based on the measurement results, a calibration curve showing the correlation between the sample concentration and the refractive index was prepared. From the prepared calibration curve, the refractive index when the sample concentration was 100 mass% was obtained and taken as the refractive index of the sample.
Compound R-1 of Comparative Example 1, compound R-3 of Comparative Example 3, and compound R-5 of Comparative Example 5 were not dissolved in a mixed solution of 3-phenoxybenzyl acrylate and trimethylolpropane trimethacrylate at a mass ratio of 4:1. Among these compounds, for the refractive index of compound R-5, the value described in JP 2023-26379 A (sample concentration: 100 mass%) is shown.

Solubility Test in Resin Raw Material

The state of a solution in which 100 mg of each of M-1 to M-11 and R-1 to R-5 as a polymerizable monomer was dissolved in 400 mg of DURANATE (registered trademark) TSS-100 was visually observed and evaluated with O (complete dissolution) and × (presence of undissolved solid). The results are shown in Table 1.

[Table 1]

| | Polymerizable monomer | Refractive Index | Solubility test |
|---|---|---|---|
| Example 1 | M-1 | 1.7048 | ○ |
| Example 2 | M-2 | 1.6802 | ○ |
| Example 3 | M-3 | 1.7058 | ○ |
| Example 4 | M-4 | 1.6876 | ○ |
| Example 5 | M-5 | 1.6923 | ○ |
| Example 6 | M-6 | 1.7048 | ○ |
| Example 7 | M-7 | 1.6788 | ○ |
| Example 8 | M-8 | 1.6673 | ○ |
| Example 9 | M-9 | 1.6804 | ○ |
| Example 10 | M-10 | 1.7184 | ○ |
| Example 11 | M-11 | 1.7016 | ○ |
| Comparative Example 1 | R-1 | - | × |
| Comparative Example 2 | R-2 | 1.6211 | ○ |
| Comparative Example 3 | R-3 | - | × |
| Comparative Example 4 | R-4(A-BPEF) | 1.6198 | × |
| Comparative Example 5 | R-5(DNFE-A) | 1.673* | × |
| * Value described in JP 2023-26379 A | | | |

As is clear from Table 1, the refractive indexes of the compounds of Examples are all 1.65 or more, and are equal to or higher than the refractive indexes of the compounds of Comparative Examples. As shown in the results of the solubility test, the compounds of Examples exhibit good solubility in the resin raw material as compared with the compounds of Comparative Examples. These results indicate that the compound of the present invention is an excellent high refractive index material.

[Table 2]

| | Polymerizable monomer | total Δn |
|---|---|---|
| Example 1 | M-1 | 0.0191 |
| Example 9 | M-9 | 0.0181 |
| Example 10 | M-10 | 0.0262 |
| Example 11 | M-11 | 0.0220 |
| Comparative Example 2 | R-2 | 0.0086 |
| Comparative Example 3 | R-3 | - |

As shown in Table 2, in compound R-2 of Comparative Example 2 in which the aromatic ring group to be substituted with a phenoxy ring is a monocyclic phenyl group, the total Δn of the hologram recording medium was 0.0086, and compound R-3 of Comparative Example 3 was not dissolved and failed to be evaluated. Since compound R-1 of Comparative Example 1 and compound R-5 of Comparative Example 5 having a hydroxyl group react with isocyanate under appropriate reaction conditions, a hologram recording medium cannot be produced, which is unsuitable.

In contrast, each of the compounds of the present invention in Example 1 and Examples 9 to 11 has a significantly high total Δn of 0.0150 or more.

In the application as an optical element of an AR glass light guide plate, when the total Δn of the hologram recording medium is higher, the projection image can be brighter, and the viewing angle can be wider. In the application as a memory, the recording capacity can be increased by improving total Δn. Meanwhile, a hologram recording medium is required to have high transparency, and turbidity due to mixing and generation of insolubles causes absorption and scattering of recording light, leading to deterioration of the performance of the hologram recording medium. In particular, light scattering due to insolubles in the application as an AR glass waveguide deteriorates light utilization efficiency and aesthetics. When an insoluble matter is precipitated on the waveguide over time, the absorption intensity of the guided light also changes over time, leading to deterioration of the performance stability of the waveguide. Thus, by using the compound of the present invention having both high total Δn and composition stability, particularly high solubility in resin raw materials, it is possible to make an AR glass light guide plate excellent in light use efficiency, aesthetics, and long-term performance stability.

From the above, it can be said that the compound of the present invention used in Examples are superior to the compounds of Comparative Examples.

Although the present invention has been described in detail with reference to specific embodiments, it will be apparent to those skilled in the art that various modifications can be made without departing from the spirit and scope of the present invention.

This application is based on Japanese Patent Application No. 2023-053749 filed on March 29, 2023, which is incorporated herein by reference in its entirety.

Reference Signs List

S: hologram recording medium
M1, M2, M3: mirror
L1: semiconductor laser light source for recording light
L2: laser light source for reproduction light
PD1, PD2, PD3: photodetector
PBS: polarizing beam splitter
1: LED unit

**Claims**

**1.** A compound represented by Formula (1) described below:

[Chem. 1]

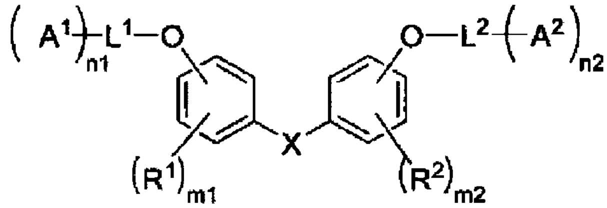

(1)

[in Formula (1), $A^1$ and $A^2$ each independently represent a polymerizable group;
n1 and n2 each independently represent an integer of 1 to 3; when n1 and n2 are 2 or 3, a plurality of $A^1$s or $A^2$s may be the same or different;
$L^1$ represents a single bond or an optionally branched (n1 + 1)-valent linking group, and $L^2$ represents a single bond or an optionally branched (n2 + 1)-valent linking group;
X represents a single bond, a divalent organic group having 1 to 20 carbons, a sulfonyl group, or a divalent oxygen atom or sulfur atom;
$R^1$ and $R^2$ each independently represent a fused aromatic ring group optionally having a substituent;
m1 and m2 are each independently an integer of 2 to 4, and a plurality of $R^1$s or $R^2{}_S$ may be the same or different;
and two benzene rings having $R^1$ and $R^2$ in the formula each independently optionally have an additional substituent other than $R^1$ or $R^2$].

**2.** The compound according to claim 1, wherein m1 and m2 are 2.

**3.** The compound according to claim 1, wherein X is a single bond or a divalent group selected from the group consisting of structures represented by Formulae (1a) to (1h) described below:

[Chem. 2]

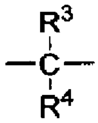

(1a)

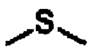

(1b)

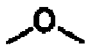

(1c)

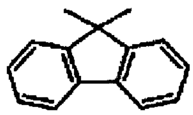

(1d)

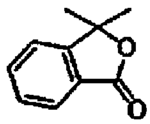

(1e)

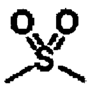

(1f)

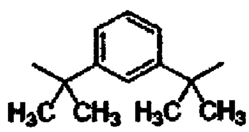

(1g)

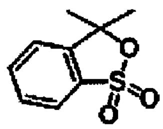

(1h)

[in Formula (1a), $R^3$ and $R^4$ each independently represent a hydrogen atom or an alkyl group having 1 to 4 carbons].

**4.** The compound according to claim 1, wherein $R^1$ and $R^2$ are each independently a phenanthryl group optionally having a substituent or a fused aromatic heterocyclic group optionally having a substituent.

**5.** A polymerizable composition comprising the compound described in any of claims 1 to 4 and a polymerization initiator.

**6.** A hologram recording medium comprising the polymerizable composition described in claim 5.

**7.** A polymer produced by polymerizing the polymerizable composition described in claim 5.

**8.** An optical material comprising the polymer described in claim 7.

**9.** An optical component comprising the polymer described in claim 7.

**10.** A large-capacity memory comprising the hologram recording medium described in claim 6.

**11.** An optical element produced by performing hologram recording on the hologram recording medium described in claim 6.

**12.** An AR light guide plate comprising the optical element described in claim 11.

**13.** An AR glass comprising the optical element described in claim 11.

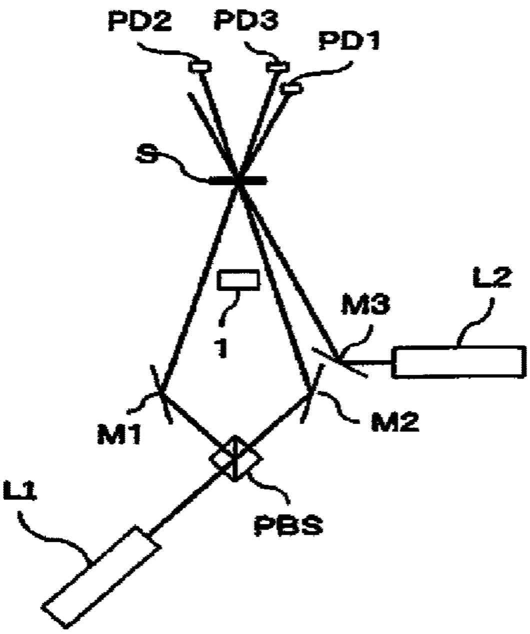


FIG. 1

**INTERNATIONAL SEARCH REPORT**

International application No. **PCT/JP2024/012674**

**A. CLASSIFICATION OF SUBJECT MATTER**

***C07C 69/54***(2006.01)i; ***C07C 271/48***(2006.01)i; ***C07D 307/885***(2006.01)i; ***C07D 333/76***(2006.01)i; ***C08F 2/46***(2006.01)i; ***C08F 20/30***(2006.01)i; ***G03H 1/02***(2006.01)i

FI: C07C69/54 Z; C07C271/48 CSP; C07D307/885; C07D333/76; C08F2/46; C08F20/30; G03H1/02

According to International Patent Classification (IPC) or to both national classification and IPC

**B. FIELDS SEARCHED**

Minimum documentation searched (classification system followed by classification symbols)

C07C69/54; C07C271/48; C07D307/885; C07D333/76; C08F2/46; C08F20/30; G03H1/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2023/038156 A1 (MITSUBISHI GAS CHEMICAL COMPANY, INC.) 16 March 2023 (2023-03-16)<br>claims, compounds 1-442 | 1-13 |
| X | WO 2023/036868 A1 (REUTER CHEMISCHE APPARATEBAU E.K.) 16 March 2023 (2023-03-16)<br>claims, compounds 1-442 | 1-13 |
| X | WO 2022/202538 A1 (MITSUBISHI CHEMICAL CORPORATION) 29 September 2022 (2022-09-29)<br>claims, compounds S-41, S-42, M-16, example 16 | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 May 2024** | **28 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/012674

C. DOCUMENTS CONSIDERED TO BE RELEVANT

| Category* | Citation of document, with indication, where appropriate, of the relevant passages | Relevant to claim No. |
|---|---|---|
| X | BELUSA, J. et al., Synthesis of 2,2-bis-/4-hydroxy-3,5-di-(2-benzotriazolyl) phenyl/propane, Tetrahedron Letters, 1968, vol. 9, no. 10, pages 1167-1170, DOI: 10.1016/S0040-4039(01)98913-0<br>pages 1168-1169 | 1-4 |
| A | | 5-13 |
| X | CAS REGISTRY No. 201416-15-1, DATABASE REGISTRY [online], 1998 (received date), [retrieved on 15 May 2024], retrieved from: STN<br>CAS REGISTRY No. 201416-15-1, particularly, chemical structural formula | 1-4 |
| A | | 5-13 |
| P, X | KR 10-2024-005381 A (LG CHEMICAL LTD.) 12 January 2024 (2024-01-12)<br>claims, paragraphs [0180], [0230]-[0302] | 1-13 |

Form PCT/ISA/210 (second sheet) (July 2022)

**INTERNATIONAL SEARCH REPORT**
**Information on patent family members**

International application No.
**PCT/JP2024/012674**

| Patent document cited in search report | Publication date (day/month/year) | Patent family member(s) | Publication date (day/month/year) |
|---|---|---|---|
| WO 2023/038156 A1 | 16 March 2023 | TW 202323232 A | |
| WO 2023/036868 A1 | 16 March 2023 | (Family: none) | |
| WO 2022/202538 A1 | 29 September 2022 | US 2024/0018137 A1<br>claims, compounds S-41, S-42, M-16, example 16<br>EP 4317155 A1<br>CN 117120486 A<br>TW 202302545 A | |
| KR 10-2024-005381 A | 12 January 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H06220131 A
- WO 2009151061 A
- JP 5664707 B
- JP 6089867 B
- JP 2009256342 A
- EP 2354845 B
- JP 2005133071 A
- JP 2008527413 A
- JP 6458645 B
- WO 2021100654 A
- WO 2021006011 A
- WO 2021006012 A
- JP H05230189 A
- JP 2000204284 A
- JP 2000119306 A
- JP 2001220526 A
- JP 2001310937 A
- JP 3737306 B
- JP 59152396 A
- JP 61151197 A
- JP H5241338 A
- JP H269 A
- JP H255446 B
- JP 2000010277 A
- JP 2004198446 A
- JP 6069294 B
- JP 2023026379 A
- JP 2023053749 A

### Non-patent literature cited in the description

- **H. KOGELNIK**. *The Bell System Technical Journal*, 1969, vol. 48, 2909-2947